# EUROPEAN PATENT SPECIFICATION

(11) **EP 3 206 717 B1**
(45) Date of publication and mention of the grant of the patent: **25.11.2020**
(21) Application number: 15784975.3
(22) Date of filing: 15.10.2015
(51) Int. Cl.: A61K 31/4709, A61K 31/506, A61K 31/517, A61K 31/5377, C07K 16/32, A61K 39/395, A61K 45/06, A61P 35/00, A61K 31/519, A61K 31/55, A61K 31/4706

(54) **COMBINATION OF CERITINIB WITH AN EGFR INHIBITOR**
KOMBINATION VON CERITINIB MIT EINEM EGFR-HEMMER
COMBINAISON DE CERITINIB ET D'UN INHIBITEUR D'EGFR

(30) Priority: 17.10.2014 US 201462065451 P; 24.10.2014 US 201462068175 P; 27.10.2014 US 201462068945 P; 20.02.2015 US 201562118710 P
(43) Date of publication of application: 23.08.2017
(73) Proprietor: Novartis AG, 4056 Basel (CH)
(72) Inventor: BORAL, Anthony, Cambridge, Massachusetts 02139 (US); LI, Fang, Cambridge, Massachusetts 02139 (US); LI, Nanxin, San Diego, California 92121 (US); PANTANO, Serafino, 4002 Basel (CH)
(74) Representative: Kristl, Jernej
(86) International application number: PCT/IB2015/057940
(87) International publication number: WO 2016/059600

(56) References cited:
- WO-A1-2015/024666
- WO-A1-2015/035410
- GRZEGORZ J. KORPANTY ET AL: "Detection of circulating tumor DNA in early- and late-stage human malignancies", FRONTIERS IN ONCOLOGY, vol. 6, no. 224, 11 August 2014 (2014-08-11), page 224ra24, XP055231461, DOI: 10.1126/scitranslmed.3007094
- KIM-SON H NGUYEN: "Review of the current targeted therapies for non-small-cell lung cancer", WORLD JOURNAL OF CLINICAL ONCOLOGY, vol. 5, no. 4, 1 January 2014 (2014-01-01) , page 576, XP055231426, US ISSN: 2218-4333, DOI: 10.5306/wjco.v5.i4.576
- KHASHAYAR ESFAHANI ET AL: "ALK signaling and target therapy in anaplastic large cell lymphoma", FRONTIERS IN ONCOLOGY, vol. 2, no. ix152, 21 July 2014 (2014-07-21), page 41, XP055231428, DOI: 10.3389/fonc.2012.00041
- SCAGLIOTTI G V ET AL: "Addressing the unmet need in lung cancer: The potential of immuno-oncology", CANCER TREATMENT REVIEWS, vol. 41, no. 6, 25 June 2015 (2015-06-25), pages 465-475, XP029169666, ISSN: 0305-7372, DOI: 10.1016/J.CTRV.2015.04.001 & WO 2015/073644 A1 (NOVARTIS AG [CH]; MANNICK JOAN [US]; GLASS DAVID JONATHAN [US]; MURPHY) 21 May 2015 (2015-05-21)

## Description

### Sequence Listing

The instant application contains a Sequence Listing which has been submitted electronically in ASCII format. Said ASCII copy, created on October 9, 2015, is named PAT056502-WO-PCT_SL.txt and is 230,153 bytes in size.

### Field of the Disclosure

The present disclosure relates to a pharmaceutical composition comprising two Tyrosine Kinase Inhibitors (TKIs). The present combination is administered independently or separately, in a quantity which is jointly therapeutically effective for the treatment of a TKI mediated disease. The disclosure further relates to the use of such combination as a medicine and to a kit of parts comprising such a combination.

### Background of the Disclosure

Targeted therapies, such as Tyrosine Kinase Inhibitors (TKIs), are widely used to treat several types of cancers and offer an alternative to standard platinum-based chemotherapy.

ALK is a member of the insulin receptor superfamily of receptor tyrosine kinases. Chromosomal rearrangements involving anaplastic lymphoma kinase (ALK) has been detected in a variety of human malignancies, leading to disturbances in the regulation pathway of the cells. Inhibition or suppression of the ALK pathways using an ALK tyrosine kinase inhibitor engenders the cell growth arrest and apoptosis of malignant cells. Targeted therapies involving ALK tyrosine kinase inhibitors have been developed.

Ceritinib (LDK378) is an Anaplastic Lymphoma Kinase (ALK) inhibitor. Its chemical formula is 5-chloro-*N*²-(2-isopropoxy-5-methyl-4-(piperidin-4-yl)phenyl)-*N*⁴-[2-(propane-2-sulfonyl)-phenyl]-pyrimidine-2,4-diamine. A process for preparing Ceritinib was disclosed in WO2008/073687. The compound has been approved by the US FDA as Zykadia® for the treatment of patients with Anaplastic Lymphoma Kinase (ALK)-positive metastatic non-small cell lung cancer (NSCLC), who have progressed on or are intolerant to crizotinib.

The Epidermal Growth Factor Receptor is a receptor for ligands of the epidermal growth factor family with several family members. Several types of cancers are known to be dependent on EGFR over-activity or over-expression.

### Brief Description of the Disclosure

It was observed that EGFR signaling can bypass the ALK inhibition. This can happen in EGFR mutant cancer types as well as cancers with no EGFR mutation. Based on clinical experience with ALK inhibitors it was observed that patients with ALK mutations developed resistance to the targeted therapies ALK tyrosine kinase inhibitors, 1 to 2 years following the start of the treatment. Surprisingly, it was observed that the acquired resistance was not only due to the development of secondary ALK mutations but also the emergence of other oncogenic drivers such as EGFR.

EGFR inhibitor alone is not sufficient when the cells acquired resistance to the treatment with an ALK inhibitor. The combination of an ALK inhibitor and EGFR inhibitor is required. Therefore, the present disclosure deals with a combination of ceritinib and an EGFR inhibitor that can provide an advantageous effect from the start of the treatment. It became clear that the combination would be valuable in the treatment of an ALK-naïve patient, for example where the patient has previously not been treated with an ALK inhibitor. In addition, the combination overcomes possible acquired resistance in ALK-positive cancers. The combination would thus be useful in post ALK inhibitor setting, such as post crizotinib or post ceritinib setting.

The first aspect of the present disclosure is a pharmaceutical combination comprising (i) ceritinib, or a pharmaceutically acceptable salt thereof, and (ii) an EGFR inhibitor, or a pharmaceutically acceptable salt thereof, wherein the EGFR inhibitor is selected from the group consisting of erlotinib, gefitinib, lapatinib, canertinib, pelitinib, neratinib, (*R,E*)-*N*-(7-chloro-1-(1-(4-(dimethylamino)but-2-enoyl)azepan-3-yl)-1H-benzo[d]imidazol-2-yl)-2-methylisonicotinamide, panitumumab, matuzumab, pertuzumab, nimotuzumab, zalutumumab, icotinib, afatinib and cetuximab, and pharmaceutically acceptable salt thereof, or the EGFR inhibitor is an isolated antibody or fragment thereof comprising a heavy chain CDR3 selected from the group consisting of SEQ ID NO: 4, SEQ ID NO: 10, SEQ ID NO: 22, SEQ ID NO: 28, SEQ ID NO: 40, SEQ ID NO: 46, SEQ ID NO: 58, SEQ ID NO: 64, SEQ ID NO: 76, SEQ ID NO: 82, SEQ ID NO: 94, SEQ ID NO: 100, SEQ ID NO: 112, SEQ ID NO: 118, SEQ ID NO: 130, SEQ ID NO: 136, SEQ ID NO: 148, SEQ ID NO: 166, SEQ ID NO: 184, SEQ ID NO: 202, SEQ ID NO: 220, SEQ ID NO: 238, SEQ ID NO: 256, SEQ ID NO: 274, SEQ ID NO: 292, SEQ ID NO: 310, SEQ ID NO: 328, SEQ ID NO: 346, and SEQ ID NO: 364.

Another aspect of this disclosure is a use of the combination of the first aspect of the disclosure as a medicine.

A yet another aspect of the disclosure is ceritinib for use as a medicine, wherein ceritinib, or a pharmaceutically acceptable salt thereof, is administered in combination with an EGFR inhibitor, or a pharmaceutically acceptable salt thereof, wherein the EGFR inhibitor is erlotinib, gefitinib, lapatinib, canertinib, pelitinib, neratinib, (*R,E*)-*N*-(7-chloro-1-(1-(4-(dimethylamino)but-2-enoyl)azepan-3-yl)-1H-benzo[d]imidazol-2-yl)-2-methylisonicotinamide, panitumumab, matuzumab, pertuzumab, nimotuzumab, zalutumumab, icotinib, afatinib, cetuximab, or a pharmaceutically acceptable salt thereof, or an isolated antibody or fragment thereof comprising a heavy chain CDR3 selected from the group consisting of SEQ ID NO: 4, SEQ ID NO: 10, SEQ ID NO: 22, SEQ ID NO: 28, SEQ ID NO: 40, SEQ ID NO: 46, SEQ ID NO: 58, SEQ ID NO: 64, SEQ ID NO: 76, SEQ ID NO: 82, SEQ ID NO: 94, SEQ ID NO: 100, SEQ ID NO: 112, SEQ ID NO: 118, SEQ ID NO: 130, SEQ ID NO: 136, SEQ ID NO: 148, SEQ ID NO: 166, SEQ ID NO: 184, SEQ ID NO: 202, SEQ ID NO: 220, SEQ ID NO: 238, SEQ ID NO: 256, SEQ ID NO: 274, SEQ ID NO: 292, SEQ ID NO: 310, SEQ ID NO: 328, SEQ ID NO: 346, and SEQ ID NO: 364.

### Brief Description of Figures

**Fig. 1** depicts a scatter plot obtained by the Cell Titer Glo assay on H2228 cell line showing which ligands could reverse the growth inhibitory effect of ceritinib.
**Fig. 2** depicts cell proliferation of MGH049 and MGH051 cells treated with the indicated doses of ceritinib for 6 days.
**Fig. 3** depicts effects on MGH049 cell growth by the EGFR inhibitors alone (top line of the graphs) or EGFR inhibitors in combination with 0.5 µM ceritinib (bottom line on the graphs).
**Fig. 4** depicts effects on MGH051 cell growth by the EGFR inhibitors alone (top line of the graphs) or EGFR inhibitors in combination with 0.5 µM ceritinib (bottom line on the graphs).
**Fig. 5****.** Inhibition of ALK induces activation of HER3 in ALK positive NSCLC cell lines. Cells were harvested after short-term and long-term ceritinib (LDK378) treatment, and whole cell lysates were analyzed by Western blotting. Long-term ALK inhibition led to up-regulation of HER3 phosphorylation.
**Fig. 6****.** Addition of HER3 and EGFR inhibitors represses the rebound in AKT phosphorylation. MGH051 cells were treated with ceritinib (LDK378) for 4 hours or 8 days. Antibody A, erlotinib and afatinib were added for the last day of the long-term LDK378 treatment.
**Fig. 7****.** Erlotinib enhances the anti-proliferation activity of ceritinib (LDK378) in ALK positive NSCLC cells. Cells were exposed to ceritinib (0.5 µM), erlotinib (1 µM), or the combination for 7 days. For each cell line, all dishes were fixed, stained and photographed at the same time.
**Fig. 8A** **and** **8B****.** Efficacy of ceritinib (LDK378) alone and in combination with cetuximab and/or Antibody A (Ab A; MOR10703 from table 1) in H2228 NSCLC xenograft in female SCID-beige mice.

### Specific Description of the Disclosure

It was surprisingly found that EGFR signaling can bypass the ALK inhibition. Results from *in vitro* and *in vivo* studies in ALK-positive NSCLC cell lines showed that inhibition of EGFR and ALK worked synergic in settings where an ALK inhibitor has not been used and where the model already initially showed resistance to an ALK inhibitor. Surprisingly, those studies also led to the discovery of a synergistic anti-cancer drug combinations able to overcome possible resistance pathway induced while treating the ALK mutated cell with ceritinib. It was found that the resistance could be overcome by combining ceritinib with an EGFR inhibitor. And it was also found that acquired resistance to ceritinib could be overcome by combining ceritinib with an EGFR inhibitor to prevent EGFR pathway from bypassing the ALK signaling in ALK positive NSCLC. The synergistic combination of ceritinib and an EGFR inhibitor according to the disclosure can be administered independently at the same time or separately within time intervals. Therefore, the present disclosure provides a pharmaceutical combination (e.g. a combination product) comprising (i) ceritinib, or a pharmaceutically acceptable salt thereof, and (ii) an EGFR inhibitor, or a pharmaceutically acceptable salt thereof, wherein the EGFR inhibitor is selected from the group consisting of erlotinib, gefitinib, lapatinib, canertinib, pelitinib, neratinib, (*R,E*)-*N*-(7-chloro-1-(1-(4-(dimethylamino)but-2-enoyl)azepan-3-yl)-1H-benzo[d]imidazol-2-yl)-2-methylisonicotinamide, panitumumab, matuzumab, pertuzumab, nimotuzumab, zalutumumab, icotinib, afatinib and cetuximab, and pharmaceutically acceptable salt thereof, or the EGFR inhibitor is an isolated antibody or fragment thereof comprising a heavy chain CDR3 selected from the group consisting of SEQ ID NO: 4, SEQ ID NO: 10, SEQ ID NO: 22, SEQ ID NO: 28, SEQ ID NO: 40, SEQ ID NO: 46, SEQ ID NO: 58, SEQ ID NO: 64, SEQ ID NO: 76, SEQ ID NO: 82, SEQ ID NO: 94, SEQ ID NO: 100, SEQ ID NO: 112, SEQ ID NO: 118, SEQ ID NO: 130, SEQ ID NO: 136, SEQ ID NO: 148, SEQ ID NO: 166, SEQ ID NO: 184, SEQ ID NO: 202, SEQ ID NO: 220, SEQ ID NO: 238, SEQ ID NO: 256, SEQ ID NO: 274, SEQ ID NO: 292, SEQ ID NO: 310, SEQ ID NO: 328, SEQ ID NO: 346, and SEQ ID NO: 364.

Pharmaceutically acceptable salts can be formed, for example, as acid addition salts, preferably with organic or inorganic acids. Suitable inorganic acids are, for example, halogen acids, such as hydrochloric acid. Suitable organic acids are, *e.g*., carboxylic acids or sulfonic acids, such as fumaric acid or methanesulfonic acid. For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred. In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient. The salts of compounds of formula (I) are preferably pharmaceutically acceptable salts; suitable counter-ions forming pharmaceutically acceptable salts are known in the field.

The present disclosure, according to a first embodiment mentioned above, relates to a pharmaceutical combination, especially a pharmaceutical combination product, comprising the mentioned combination partners.

EGFR inhibitor targets, decreases or inhibits the activity of the epidermal growth factor family of receptor tyrosine kinases (EGFR (ErbB1), ErbB2, ErbB3, ErbB4 as homo- or heterodimers) and their mutants. EGFR (ErbB1, HER1), ErbB2 (HER2), ErbB3 (HER3) and ErbB4 (HER4) are structurally related single chain transmembrane glycoprotein receptors consisting of an extracellular ligand-binding ectodomain, a transmembrane domain, a short juxtamembrane section, a tyrosine kinase domain and a tyrosine-containing C-terminal tail. Compounds which target, decrease or inhibit the activity of the epidermal growth factor receptor family are especially compounds, proteins or antibodies which inhibit members of the EGF receptor tyrosine kinase family, *e.g*. EGF receptor (ErbB1), ErbB2, ErbB3 and ErbB4 or bind to EGF or EGF related ligands. In a particular embodiment, EGFR inhibitor targets, decreases or inhibits the activity of EGFR, aka ErbB-1 and/or ErbB3 (aka HER3). In a particular embodiment, the EGFR inhibitor is EGFR specific (ErbB1, HER1). In another embodiment the EGFR inhibitor is ErbB3 specific (HER3). In yet another embodiment, the EGFR inhibitor targets EGFR with somatic mutations of the EGFR gene. These mutations can be small deletions that affect amino acids 747 through 750 or point mutations (most commonly a replacement of leucine by arginine at codon 858 [L858R]). In addition, or alternative, EGFR inhibitor can inhibit EGFR T790M. In a specific embodiment, EGFR inhibitor can inhibit a wild type EGFR.

For example, EGFR inhibitor can be selected from the group consisting of erlotinib, gefitinib, lapatinib, canertinib, pelitinib, neratinib, (*R,E*)-*N*-(7-chloro-1-(1-(4-(dimethylamino)but-2-enoyl)azepan-3-yl)-1H-benzo[d]imidazol-2-yl)-2-methylisonicotinamide, panitumumab, matuzumab, pertuzumab, nimotuzumab, zalutumumab, icotinib, afatinib and cetuximab, and pharmaceutically acceptable salt thereof.

According to the present disclosure, gefitinib, cetuximab and erlotinib are particularly preferred EGFR inhibitors that can be combined with ceritinib.

Erlotinib (marketed in Tarceva®, Roche, Basel, Switzerland) is an EGFR inhibitor disclosed in WO 96/30347 as example 20 with formula 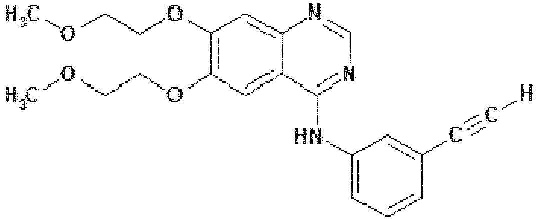 = N-(3-ethynylphenyl)-6,7-bis- (2-methoxyethoxy)quinazolin-4-amine, or a pharmaceutically acceptable salt thereof.

Gefitinib (marketed in Iressa®, AstraZeneca) was for example disclosed in WO96/33980, Example 1 with the structure 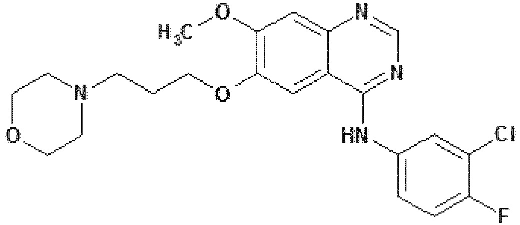 = *N*-(3-chloro-4-fluorophenyl)-7-methoxy-6-[3-(morpholin-4-yl)propoxy]quinazolin-4-amine, or a pharmaceutically acceptable salt thereof. This compound or its pharmaceutically acceptable salts are especially preferred in the embodiments of the present disclosure.

Another EGFR inhibitor, lapatinib (marketed in Tykerb® (USA), Tyverb® (EP), GlaxoSmithKline) was disclosed in US 6,391,874, US 7,157,466, US 6,828,320, 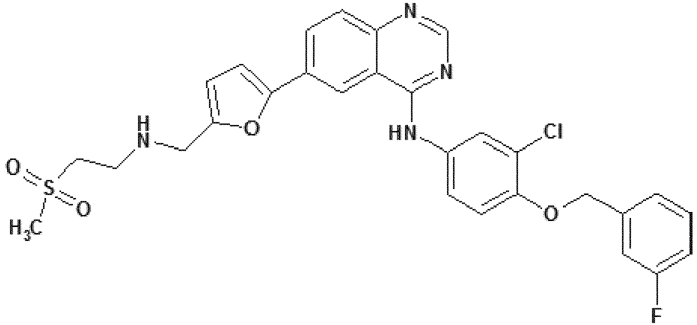 = *N*-[3-chloro-4-(3-fluorobenzyloxy)phenyl]- 6-{5-[4-(methylsulfonyl)- 2-azabutyl]-2-furyl} quinazolin-4-amine, or a pharmaceutically acceptable salt or prodrug thereof, see e.g. WO9935146 (Example 29).

Canertinib (Pfizer) (e.g. used as dihydrochloride)), or *N*-[4-[(3-Chloro-4-fluorophenyl)amino]-7-(3-morpholin-4-ylpropoxy)quinazolin-6-yl]prop-2-enamide, or a pharmaceutically acceptable salt or prodrug thereof, was disclosed in WO2000031048 with the formula.

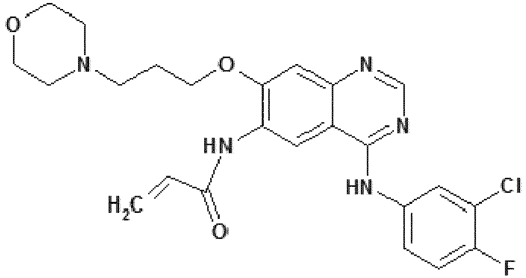

Pelitinib (Wyeth, owned by Pfizer) with the name (2*E*)-*N*-[4-[(3-Chloro-4-fluorophenyl)amino]-3-cyano-7-ethoxy-6-quinolinyl]-4-(dimethylamino)-2-butenamide, and formula 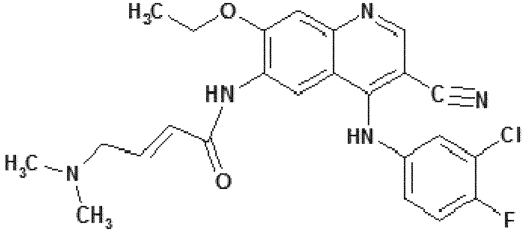 see WO2005028443 (Example 20)

Neratinib (Pfizer Inc.), (2*E*)-*N*-[4-[[3-chloro-4-[(pyridin-2-yl)methoxy]phenyl]amino]-3-cyano-7-ethoxyquinolin-6-yl]-4-(dimethylamino)but-2-enamide, or a pharmaceutically acceptable salt or prodrug thereof with formula 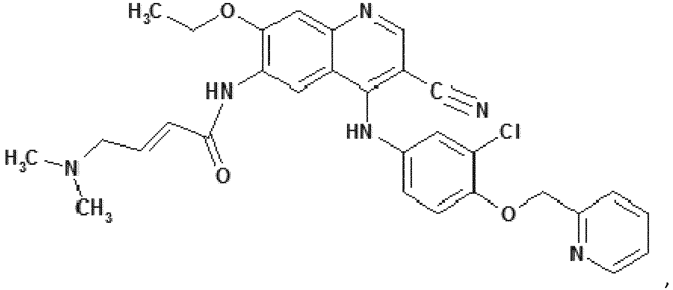 see e.g. WO2005028443 Example 2.

Another EGFR inhibitor used in combination with ceritinib can be (*R,E*)-*N*-(7-chloro-1-(1-(4-(dimethylamino)but-2-enoyl)azepan-3-yl)-1*H*-benzo[d]imidazol-2-yl)-2-methylisonicotinamide (compound A) 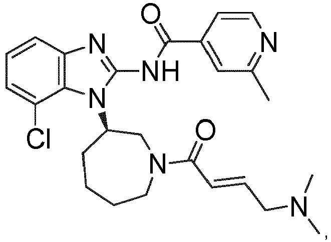 disclosed in WO2013184757.

Another EGFR inhibitor, panitumumab (marketed as VECTIBIX®(US), Amgen) was disclosed in US 6,235,883 and US 7,807,798.

Another EGFR inhibitor, pertuzumab (marketed as PERJETA® (US), Genentech Inc.) was disclosed in WO200100244.

Another EGFR inhibitor, matuzumab (EMD 72000, Merk Serono), was disclosed in clinical trials studies (NCT00111839 and NCT00215644).

Another EGFR inhibitor, zalutumumab (developed by Genmab), was disclosed in clinical trials studies (NCT00093041) and Bastholt et al. Radiother. Oncol. 2007, 85(1), 24-28.

Another EGFR inhibitor, icotinib (approved in China under the name of Conmana, Zhejiang Bata Pharma Ltd.) was disclosed in WO2003082830 (example 15), with formula 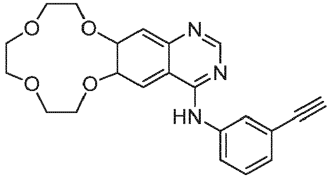 =3-Ethylnyl-phenyl-(7,8,10,11,13,14-hexahydro-6,9,12,15-tetraoxa-1,3-diazacyclododeca[b]naphthalene-4-yl)-amine or a pharmaceutically acceptable salt thereof.

Another EGFR inhibitor, nimotuzumab (THERALOC® (EU), Oncoscience AG) was disclosed Grosse et al. J. Cell. Biochem. 1992, 49(2), 157-165 and Bartels et al. Future Oncol. 2009, 5(9), 1349-1361.

Another EGFR inhibitor, afatinib (marketed as GILOTRIF® (US), GIOTRIF® (EU), Boehringer Ingelheim Pharmaceuticals) was disclosed in US 8,735,409B2 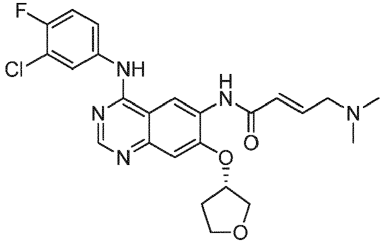 = (*S,E*)-*N*-(4-((3-chloro-4-fluorophenyl)amino)-7-((tetrahydrofuran-3-yl)oxy)quinazolin-6-yl)-4-(dimethylamino)but-2-enamide or a pharmaceutically acceptable salt thereof.

Among the possible EGFR inhibitors, also antibodies may be mentioned, e.g. Cetuximab (Erbitux®) (ImClone Systems, Bristol-Myers Squibb and Merck KgaA) which is a chimeric (mouse/human) monoclonal antibody, active as an epidermal growth factor receptor (EGFR) inhibitor, which can be administered e.g. intravenously.

In one embodiment, the EGFR inhibitor is an isolated antibody or fragment thereof to a HER3 receptor comprising 1, 2, 3, 4, 5, or 6 CDRs calculated by Kabat or Chothia of any of the antibodies shown in Table 1.

In one example, the EGFR inhibitor is an isolated antibody or fragment thereof comprising a heavy chain CDR3 selected from the group consisting of SEQ ID NO: 4, SEQ ID NO: 10, SEQ ID NO: 22, SEQ ID NO: 28, SEQ ID NO: 40, SEQ ID NO: 46, SEQ ID NO: 58, SEQ ID NO: 64, SEQ ID NO: 76, SEQ ID NO: 82, SEQ ID NO: 94, SEQ ID NO: 100, SEQ ID NO: 112, SEQ ID NO: 118, SEQ ID NO: 130, SEQ ID NO: 136, SEQ ID NO: 148, SEQ ID NO: 166, SEQ ID NO: 184, SEQ ID NO: 202, SEQ ID NO: 220, SEQ ID NO: 238, SEQ ID NO: 256, SEQ ID NO: 274, SEQ ID NO: 292, SEQ ID NO: 310, SEQ ID NO: 328, SEQ ID NO: 346, and SEQ ID NO: 364. Examples of isolated HER3 antibody or fragment include:
a VH comprising SEQ ID NO: 15 and a VL comprising SEQ ID NO: 14, or an amino acid sequence with 97-99 percent identity thereof;
a VH comprising SEQ ID NO: 33 and a VL comprising SEQ ID NO: 32, or an amino acid sequence with 97-99 percent identity thereof;
a VH comprising SEQ ID NO: 51 and a VL comprising SEQ ID NO: 50, or an amino acid sequence with 97-99 percent identity thereof;
a VH comprising SEQ ID NO: 69 and a VL comprising SEQ ID NO: 68, or an amino acid sequence with 97-99 percent identity thereof;
a VH comprising SEQ ID NO: 87 and a VL comprising SEQ ID NO: 86, or an amino acid sequence with 97-99 percent identity thereof;
a VH comprising SEQ ID NO: 105 and a VL comprising SEQ ID NO: 104, or an amino acid sequence with 97-99 percent identity thereof;
a VH comprising SEQ ID NO: 123 and a VL comprising SEQ ID NO: 122, or an amino acid sequence with 97-99 percent identity thereof
a VH comprising SEQ ID NO: 141 and a VL comprising SEQ ID NO: 140, or an amino acid sequence with 97-99 percent identity thereof;
a VH comprising SEQ ID NO: 159 and a VL comprising SEQ ID NO: 158, or an amino acid sequence with 97-99 percent identity thereof;
a VH comprising SEQ ID NO: 177 and a VL comprising SEQ ID NO: 176, or an amino acid sequence with 97-99 percent identity thereof;
a VH comprising SEQ ID NO: 195 and a VL comprising SEQ ID NO: 194, or an amino acid sequence with 97-99 percent identity thereof;
a VH comprising SEQ ID NO: 213 and a VL comprising SEQ ID NO: 212, or an amino acid sequence with 97-99 percent identity thereof;
a VH comprising SEQ ID NO: 231 and a VL comprising SEQ ID NO: 230, or an amino acid sequence with 97-99 percent identity thereof;
a VH comprising SEQ ID NO: 249 and a VL comprising SEQ ID NO: 248, or an amino acid sequence with 97-99 percent identity thereof;
a VH comprising SEQ ID NO: 267 and a VL comprising SEQ ID NO: 266, or an amino acid sequence with 97-99 percent identity thereof;
a VH comprising SEQ ID NO: 285 and a VL comprising SEQ ID NO: 284, or an amino acid sequence with 97-99 percent identity thereof
a VH comprising SEQ ID NO: 303 and a VL comprising SEQ ID NO: 302, or an amino acid sequence with 97-99 percent identity thereof;
a VH comprising SEQ ID NO: 321 and a VL comprising SEQ ID NO: 320, or an amino acid sequence with 97-99 percent identity thereof;
a VH comprising SEQ ID NO: 339 and a VL comprising SEQ ID NO: 338, or an amino acid sequence with 97-99 percent identity thereof;
a VH comprising SEQ ID NO: 357 and a VL comprising SEQ ID NO: 356, or an amino acid sequence with 97-99 percent identity thereof; and
a VH comprising SEQ ID NO: 375 and a VL comprising SEQ ID NO: 374, or an amino acid sequence with 97-99 percent identity thereof.

In one example, the EGFR inhibitor is an antibody or fragment thereof that recognizes a conformational epitope of a HER3 receptor comprising amino acid residues 265-277, and 315 within domain 2 and amino acid residues 571, 582-584, 596-597, 600-602, and 609-615 within domain 4 of the HER3 receptor of SEQ ID NO: 1, the sequence SEQ ID NO: 1 being as defined in WO2013/084148, and wherein the antibody or fragment thereof blocks both ligand-dependent and ligand-independent signal transduction. In alternative, the EGFR inhibitor is an antibody or fragment thereof that comprises a heavy chain variable region CDR1 of SEQ ID NO: 128; CDR2 of SEQ ID NO: 129; CDR3 of SEQ ID NO: 130; and a light chain variable region CDR1 of SEQ ID NO: 131; CDR2 of SEQ ID NO: 132; and CDR3 of SEQ ID NO: 133. Possible variant of the antibody or the fragment thereof are those that recognize a conformational epitope of a HER3 receptor as defined above and comprises at least one variable region CDR1 of SEQ ID NO: 128; CDR2 of SEQ ID NO: 129; or CDR3 of SEQ ID NO: 130; or at least one light chain variable region CDR1 of SEQ ID NO: 131; CDR2 of SEQ ID NO: 132; or CDR3 of SEQ ID NO: 133. A preferred EGFR inhibitor is an antibody or fragment thereof comprising the sequences of MOR10703 as defined in Table 1 (Antibody A). A process of preparing the antibody was described in WO2013/084148. Ceritinib or a pharmaceutically acceptable salt thereof can also be combined with an antibody comprising the sequences of MOR10703 as defined in Table 1 (Antibody A), or fragment thereof, and cetuximab. The Antibody A and cetuximab combined can significantly improve the efficacy of ceritinib. The triple combination is especially efficacious in the treatment of NSCLC.

An EGFR inhibitor (HER3 inhibitor), which can be combined with ceritinib, can be an antibody that comprises a heavy chain variable region CDR1 of SEQ ID NO: 128. An EGFR inhibitor can also be an antibody that comprises a heavy chain variable region CDR2 of SEQ ID NO: 129. The antibody can comprise a heavy chain variable region CDR3 of SEQ ID NO: 130.

An EGFR inhibitor (HER3 inhibitor), which can be combined with ceritinib, can be an antibody that comprises a light chain variable region CDR1 of SEQ ID NO: 131. In the same or alternative embodiment, an EGFR inhibitor can be an antibody that comprises a light chain variable region CDR2 of SEQ ID NO: 132. An EGFR inhibiting antibody can also comprise a light chain variable region CDR3 of SEQ ID NO: 133. Further combinations of said CDRs are contemplated herein.

**Table 1**

| **SEQ ID NUMBER** | **Ab region** | |
|---|---|---|
| **MOR09823** | | |
| SEQ ID NO: 2 (Kabat) | HCDR1 | SYAMS |
| SEQ ID NO: 3 (Kabat) | HCDR2 | VTGAVGRTYYPDSVKG |
| SEQ ID NO: 4 (Kabat) | HCDR3 | WGDEGFDI |
| SEQ ID NO: 5 (Kabat) | LCDR1 | RASQGISNWLA |
| SEQ ID NO: 6 (Kabat) | LCDR2 | GASSLQS |
| SEQ ID NO: 7 (Kabat) | LCDR3 | QQYSSFPTT |
| SEQ ID NO: 8 (Chothia) | HCDR1 | GFTFSSY |
| SEQ ID NO: 9 (Chothia) | HCDR2 | GAVGR |
| SEQ ID NO: 10 (Chothia) | HCDR3 | WGDEGFDI |
| SEQ ID NO: 11 (Chothia) | LCDR1 | SQGISNW |
| SEQ ID NO: 12 (Chothia) | LCDR2 | GAS |
| SEQ ID NO: (Chothia) 13 | LCDR3 | YSSFPT |
| SEQ ID NO: 14 | VL | |
| SEQ ID NO: 15 | VH | |
| SEQ ID NO: 16 | DNA VL | |
| SEQ ID NO: 17 | DNA VH | |
| SEQ ID NO: 18 | Light Kappa | |
| SEQ ID NO: 19 | Heavy IgG1 | |

| **MOR09824** | | |
|---|---|---|
| SEQ ID NO: 20 (Kabat) | HCDR1 | SYAMS |
| SEQ ID NO: 21 (Kabat) | HCDR2 | VISAWGHVKYYADSVKG |
| SEQ ID NO: 22 (Kabat) | HCDR3 | WGDEGFDI |
| SEQ ID NO: 23 (Kabat) | LCDR1 | RASQGISNWLA |
| SEQ ID NO: 24 (Kabat) | LCDR2 | GASSLQS |
| SEQ ID NO: 25 (Kabat) | LCDR3 | QQYSSFPTT |
| SEQ ID NO: 26 (Chothia) | HCDR1 | GFTFSSY |
| SEQ ID NO: 27 (Chothia) | HCDR2 | SAWGHV |
| SEQ ID NO: 28 (Chothia) | HCDR3 | WGDEGFDI |
| SEQ ID NO: 29 (Chothia) | LCDR1 | SQGISNW |
| SEQ ID NO: 30 (Chothia) | LCDR2 | GAS |
| SEQ ID NO: 31 (Chothia) | LCDR3 | YSSFPT |
| SEQ ID NO: 32 | VL | |
| SEQ ID NO: 33 | VH | |
| SEQ ID NO: 34 | DNA VL | |
| SEQ ID NO: 35 | DNA VH | |
| SEQ ID NO: 36 | Light Kappa | |
| SEQ ID NO: 37 | Heavy IgG1 | |

| **MOR09825** | | |
|---|---|---|
| SEQ ID NO: 38 (Kabat) | HCDR1 | SYAMS |
| SEQ ID NO: 39 (Kabat) | HCDR2 | AINSQGKSTYYADSVKG |
| SEQ ID NO: 40 (Kabat) | HCDR3 | WGDEGFDI |
| SEQ ID NO: 41 (Kabat) | LCDR1 | RASQGISNWLA |
| SEQ ID NO: 42 (Kabat) | LCDR2 | GASSLQS |
| SEQ ID NO: 43 (Kabat) | LCDR3 | QQYSSFPTT |
| SEQ ID NO: 44 (Chothia) | HCDR1 | GFTFSSY |
| SEQ ID NO: 45 (Chothia) | HCDR2 | NSQGKS |
| SEQ ID NO: 46 (Chothia) | HCDR3 | WGDEGFDI |
| SEQ ID NO: 47 (Chothia) | LCDR1 | SQGISNW |
| SEQ ID NO: 48 (Chothia) | LCDR2 | GAS |
| SEQ ID NO: 49 (Chothia) | LCDR3 | YSSFPT |
| SEQ ID NO: 50 | VL | |
| SEQ ID NO: 51 | VH | |
| SEQ ID NO: 52 | DNA VL | |
| SEQ ID NO: 53 | DNA VH | |
| SEQ ID NO: 54 | Light Kappa | |
| SEQ ID NO: 55 | Heavy IgG1 | |

| **MOR09974** | | |
|---|---|---|
| SEQ ID NO: 56 (Kabat) | HCDR1 | SYAMS |
| SEQ ID NO: 57 (Kabat) | HCDR2 | VINPSGNFTNYADSVKG |
| SEQ ID NO: 58 (Kabat) | HCDR3 | WGDEGFDI |
| SEQ ID NO: 59 (Kabat) | LCDR1 | RASQGISNWLA |
| SEQ ID NO: 60 (Kabat) | LCDR2 | GASSLQS |
| SEQ ID NO: 61 (Kabat) | LCDR3 | QQYSSFPTT |
| SEQ ID NO: 62 (Chothia) | HCDR1 | GFTFSSY |
| SEQ ID NO: 63 (Chothia) | HCDR2 | NPSGNF |
| SEQ ID NO: 64 (Chothia) | HCDR3 | WGDEGFDI |
| SEQ ID NO: 65 (Chothia) | LCDR1 | SQGISNW |
| SEQ ID NO: 66 (Chothia) | LCDR2 | GAS |
| SEQ ID NO: 67 (Chothia) | LCDR3 | YSSFPT |
| SEQ ID NO: 68 | VL | |
| SEQ ID NO: 69 | VH | |
| SEQ ID NO: 70 | DNA VL | |
| SEQ ID NO: 71 | DNA VH | |
| SEQ ID NO: 72 | Light Kappa | |
| SEQ ID NO: 73 | Heavy IgG1 | |

| **MOR10452** | | |
|---|---|---|
| SEQ ID NO: 74 (Kabat) | HCDR1 | SYAMS |
| SEQ ID NO: 75 (Kabat) | HCDR2 | NTSPIGYTYYAGSVKG |
| SEQ ID NO: 76 (Kabat) | HCDR3 | WGDEGFDI |
| SEQ ID NO: 77 (Kabat) | LCDR1 | RASQGISNWLA |
| SEQ ID NO: 78 (Kabat) | LCDR2 | GASSLQS |
| SEQ ID NO: 79 (Kabat) | LCDR3 | QQYSSFPTT |
| SEQ ID NO: 80 (Chothia) | HCDR1 | GFTFSSY |
| SEQ ID NO: 81 (Chothia) | HCDR2 | SPIGY |
| SEQ ID NO: 82 (Chothia) | HCDR3 | WGDEGFDI |
| SEQ ID NO: 83 (Chothia) | LCDR1 | SQGISNW |
| SEQ ID NO: 84 (Chothia) | LCDR2 | GAS |
| SEQ ID NO: 85 (Chothia) | LCDR3 | YSSFPT |
| SEQ ID NO: 86 | VL | |
| SEQ ID NO: 87 | VH | |
| SEQ ID NO: 88 | DNA VL | |
| SEQ ID NO: 89 | DNA VH | |
| SEQ ID NO: 90 | Light Kappa | |
| SEQ ID NO: 91 | Heavy Chain (only VH and CH1 domains) | |

| **MOR10701** | | |
|---|---|---|
| SEQ ID NO: 92 (Kabat) | HCDR1 | SYAMS |
| SEQ ID NO: 93 (Kabat) | HCDR2 | VTGAVGRSTYYPDSVKG |
| SEQ ID NO: 94 (Kabat) | HCDR3 | WGDEGFDI |
| SEQ ID NO: 95 (Kabat) | LCDR1 | RASQGISNWLA |
| SEQ ID NO: 96 (Kabat) | LCDR2 | GASSLQS |
| SEQ ID NO: 97 (Kabat) | LCDR3 | QQYSSFPTT |
| SEQ ID NO: 98 (Chothia) | HCDR1 | GFTFSSY |
| SEQ ID NO: 99 (Chothia) | HCDR2 | GAVGRS |
| SEQ ID NO: 100 (Chothia) | HCDR3 | WGDEGFDI |
| SEQ ID NO: 101 (Chothia) | LCDR1 | SQGISNW |
| SEQ ID NO: 102 (Chothia) | LCDR2 | GAS |
| SEQ ID NO: 103 (Chothia) | LCDR3 | YSSFPT |
| SEQ ID NO: 104 | VL | |
| SEQ ID NO: 105 | VH | |
| SEQ ID NO: 106 | DNA VL | |
| SEQ ID NO: 107 | DNA VH | |
| SEQ ID NO: 108 | Light Kappa | |
| SEQ ID NO: 109 | Heavy IgG1 | |

| **MOR10702** | | |
|---|---|---|
| SEQ ID NO: 110 (Kabat) | HCDR1 | SYAMS |
| SEQ ID NO: 111 (Kabat) | HCDR2 | VISAWGHVKYYADSVKG |
| SEQ ID NO: 112 (Kabat) | HCDR3 | WGDEGFDI |
| SEQ ID NO: 113 (Kabat) | LCDR1 | RASQGISNWLA |
| SEQ ID NO: 114 (Kabat) | LCDR2 | GASSLQS |
| SEQ ID NO: 115 (Kabat) | LCDR3 | QQYSSFPTT |
| SEQ ID NO: 116 (Chothia) | HCDR1 | GFTFSSY |
| SEQ ID NO: 117 (Chothia) | HCDR2 | SAWGHV |
| SEQ ID NO: 118 (Chothia) | HCDR3 | WGDEGFDI |
| SEQ ID NO: 119 (Chothia) | LCDR1 | SQGISNW |
| SEQ ID NO: 120 (Chothia) | LCDR2 | GAS |
| SEQ ID NO: 121 (Chothia) | LCDR3 | YSSFPT |
| SEQ ID NO: 122 | VL | |
| SEQ ID NO: 123 | VH | |
| SEQ ID NO: 124 | DNA VL | |
| SEQ ID NO: 125 | DNA VH | |
| SEQ ID NO: 126 | Light Kappa | |
| SEQ ID NO: 127 | Heavy IgG1 | |
| | | |

| **MOR10703** | | |
|---|---|---|
| SEQ ID NO: 128 (Kabat) | HCDR1 | SYAMS |
| SEQ ID NO: 129 (Kabat) | HCDR2 | AINSQGKSTYYADSVKG |
| SEQ ID NO: 130 (Kabat) | HCDR3 | WGDEGFDI |
| SEQ ID NO: 131 (Kabat) | LCDR1 | RASQGISNWLA |
| SEQ ID NO: 132 (Kabat) | LCDR2 | GASSLQS |
| SEQ ID NO: 133 (Kabat) | LCDR3 | QQYSSFPTT |
| SEQ ID NO: 134 (Chothia) | HCDR1 | GFTFSSY |
| SEQ ID NO: 135 (Chothia) | HCDR2 | NSQGKS |
| SEQ ID NO: 136 (Chothia) | HCDR3 | WGDEGFDI |
| SEQ ID NO: 137 (Chothia) | LCDR1 | SQGISNW |
| SEQ ID NO: 138 (Chothia) | LCDR2 | GAS |
| SEQ ID NO: 139 (Chothia) | LCDR3 | YSSFPT |
| SEQ ID NO: 140 | VL | |
| SEQ ID NO: 141 | VH | |
| SEQ ID NO: 142 | DNA VL | |
| SEQ ID NO: 143 | DNA VH | |
| SEQ ID NO: 144 | Light Kappa | |
| SEQ ID NO: 145 | Heavy IgG1 | |
| | | |

| **MOR10703 N52S** | | |
|---|---|---|
| SEQ ID NO: 146 (Kabat) | HCDR1 | SYAMS |
| SEQ ID NO: 147 (Kabat) | HCDR2 | AI**S**SQGKSTYYADSVKG |
| SEQ ID NO: 148 (Kabat) | HCDR3 | WGDEGFDI |
| SEQ ID NO: 149 (Kabat) | LCDR1 | RASQGISNWLA |
| SEQ ID NO: 150 (Kabat) | LCDR2 | GASSLQS |
| SEQ ID NO: 151 (Kabat) | LCDR3 | QQYSSFPTT |
| SEQ ID NO: 152 (Chothia) | HCDR1 | GFTFSSY |
| SEQ ID NO: 153 (Chothia) | HCDR2 | **S**SQGKS |
| SEQ ID NO: 154 (Chothia) | HCDR3 | WGDEGFDI |
| SEQ ID NO: 155 (Chothia) | LCDR1 | SQGISNW |
| SEQ ID NO: 156 (Chothia) | LCDR2 | GAS |
| SEQ ID NO: 157 (Chothia) | LCDR3 | YSSFPT |
| SEQ ID NO: 158 | VL | |
| SEQ ID NO: 159 | VH | |
| SEQ ID NO: 160 | DNA VL | |
| SEQ ID NO: 161 | DNA VH | |
| SEQ ID NO: 162 | Light Kappa | |
| SEQ ID NO: 163 | Heavy IgG1 | |
| | | |

| **MOR10703 N52G** | | |
|---|---|---|
| SEQ ID NO: 164 (Kabat) | HCDR1 | SYAMS |
| SEQ ID NO: 165 (Kabat) | HCDR2 | AI**G**SQGKSTYYADSVKG |
| SEQ ID NO: 166 (Kabat) | HCDR3 | WGDEGFDI |
| SEQ ID NO: 167 (Kabat) | LCDR1 | RASQGISNWLA |
| SEQ ID NO: 168 (Kabat) | LCDR2 | GASSLQS |
| SEQ ID NO: 169 (Kabat) | LCDR3 | QQYSSFPTT |
| SEQ ID NO: 170 (Chothia) | HCDR1 | GFTFSSY |
| SEQ ID NO: 171 (Chothia) | HCDR2 | **G**SQGKS |
| SEQ ID NO: 172 (Chothia) | HCDR3 | WGDEGFDI |
| SEQ ID NO: 173 (Chothia) | LCDR1 | SQGISNW |
| SEQ ID NO: 174 (Chothia) | LCDR2 | GAS |
| SEQ ID NO: 175 (Chothia) | LCDR3 | YSSFPT |
| SEQ ID NO: 176 | VL | |
| SEQ ID NO: 177 | VH | |
| SEQ ID NO: 178 | DNA VL | |
| SEQ ID NO: 179 | DNA VH | |
| | | |
| SEQ ID NO: 180 | Light Kappa | |
| SEQ ID NO: 181 | Heavy IgG1 | |

| **MOR10703 N52S_S52aN** | | |
|---|---|---|
| SEQ ID NO: 182 (Kabat) | HCDR1 | SYAMS |
| SEQ ID NO: 183 (Kabat) | HCDR2 | AI**SN**QGKSTYYADSVKG |
| SEQ ID NO: 184 (Kabat) | HCDR3 | WGDEGFDI |
| SEQ ID NO: 185 (Kabat) | LCDR1 | RASQGISNWLA |
| SEQ ID NO: 186 (Kabat) | LCDR2 | GASSLQS |
| SEQ ID NO: 187 (Kabat) | LCDR3 | QQYSSFPTT |
| SEQ ID NO: 188 (Chothia) | HCDR1 | GFTFSSY |
| SEQ ID NO: 189 (Chothia) | HCDR2 | **SN**QGKS |
| SEQ ID NO: 190 (Chothia) | HCDR3 | WGDEGFDI |
| SEQ ID NO: 191 (Chothia) | LCDR1 | SQGISNW |
| SEQ ID NO: 192 (Chothia) | LCDR2 | GAS |
| SEQ ID NO: 193 (Chothia) | LCDR3 | YSSFPT |
| SEQ ID NO: 194 | VL | |
| SEQ ID NO: 195 | VH | |
| SEQ ID NO: 196 | DNA VL | |
| SEQ ID NO: 197 | DNA VH | |
| SEQ ID NO: 198 | Light Kappa | |
| SEQ ID NO: 199 | Heavy IgG1 | |

| **MOR10703 A50V_N52S** | | |
|---|---|---|
| SEQ ID NO: 200 (Kabat) | HCDR1 | SYAMS |
| SEQ ID NO: 201 (Kabat) | HCDR2 | **V**I**S**SQGKSTYYADSVKG |
| SEQ ID NO: 202 (Kabat) | HCDR3 | WGDEGFDI |
| SEQ ID NO: 203 (Kabat) | LCDR1 | RASQGISNWLA |
| SEQ ID NO: 204 (Kabat) | LCDR2 | GASSLQS |
| SEQ ID NO: 205 (Kabat) | LCDR3 | QQYSSFPTT |
| SEQ ID NO: 206 (Chothia) | HCDR1 | GFTFSSY |
| SEQ ID NO: 207 (Chothia) | HCDR2 | **S**SQGKS |
| SEQ ID NO: 208 (Chothia) | HCDR3 | WGDEGFDI |
| SEQ ID NO: 209 (Chothia) | LCDR1 | SQGISNW |
| SEQ ID NO: 210 (Chothia) | LCDR2 | GAS |
| SEQ ID NO: 211 (Chothia) | LCDR3 | YSSFPT |
| SEQ ID NO: 212 | VL | |
| SEQ ID NO: 213 | VH | |
| SEQ ID NO: 214 | DNA VL | |
| SEQ ID NO: 215 | DNA VH | |
| SEQ ID NO: 216 | Light Kappa | |
| SEQ ID NO: 217 | Heavy IgG1 | |

| **MOR10703 A50V_N52G** | | |
|---|---|---|
| SEQ ID NO: 218 (Kabat) | HCDR1 | SYAMS |
| SEQ ID NO: 219 (Kabat) | HCDR2 | **V**I**G**SQGKSTYYADSVKG |
| SEQ ID NO: 220 (Kabat) | HCDR3 | WGDEGFDI |
| SEQ ID NO: 221 (Kabat) | LCDR1 | RASQGISNWLA |
| SEQ ID NO: 222 (Kabat) | LCDR2 | GASSLQS |
| SEQ ID NO: 223 (Kabat) | LCDR3 | QQYSSFPTT |
| SEQ ID NO: 224 (Chothia) | HCDR1 | GFTFSSY |
| SEQ ID NO: 225 (Chothia) | HCDR2 | **G**SQGKS |
| SEQ ID NO: 226 (Chothia) | HCDR3 | WGDEGFDI |
| SEQ ID NO: 227 (Chothia) | LCDR1 | SQGISNW |
| SEQ ID NO: 228 (Chothia) | LCDR2 | GAS |
| SEQ ID NO: 229 (Chothia) | LCDR3 | YSSFPT |
| SEQ ID NO: 230 | VL | |
| SEQ ID NO: 231 | VH | |
| SEQ ID NO: 232 | DNA VL | |
| SEQ ID NO: 233 | DNA VH | |
| SEQ ID NO: 234 | Light Kappa | |
| SEQ ID NO: 235 | Heavy IgG1 | |
| | | |

| **MOR10703 S52aA** | | |
|---|---|---|
| SEQ ID NO: 236 (Kabat) | HCDR1 | SYAMS |
| SEQ ID NO: 237 (Kabat) | HCDR2 | AIN**A**QGKSTYYADSVKG |
| SEQ ID NO: 238 (Kabat) | HCDR3 | WGDEGFDI |
| SEQ ID NO: 239 (Kabat) | LCDR1 | RASQGISNWLA |
| SEQ ID NO: 240 (Kabat) | LCDR2 | GASSLQS |
| SEQ ID NO: 241 (Kabat) | LCDR3 | QQYSSFPTT |
| SEQ ID NO: 242 (Chothia) | HCDR1 | GFTFSSY |
| SEQ ID NO: 243 (Chothia) | HCDR2 | N**A**QGKS |
| SEQ ID NO: 244 (Chothia) | HCDR3 | WGDEGFDI |
| SEQ ID NO: 245 (Chothia) | LCDR1 | SQGISNW |
| SEQ ID NO: 246 (Chothia) | LCDR2 | GAS |
| SEQ ID NO: 247 (Chothia) | LCDR3 | YSSFPT |
| SEQ ID NO: 248 | VL | |
| SEQ ID NO: 249 | VH | |
| SEQ ID NO: 250 | DNA VL | |
| SEQ ID NO: 251 | DNA VH | |
| | | |
| SEQ ID NO: 252 | Light Kappa | |
| SEQ ID NO: 253 | Heavy IgG1 | |

| **MOR10703 S52aT** | | |
|---|---|---|
| SEQ ID NO: 254 (Kabat) | HCDR1 | SYAMS |
| SEQ ID NO: 255 (Kabat) | HCDR2 | AIN**T**QGKSTYYADSVKG |
| SEQ ID NO: 256 (Kabat) | HCDR3 | WGDEGFDI |
| SEQ ID NO: 257 (Kabat) | LCDR1 | RASQGISNWLA |
| SEQ ID NO: 258 (Kabat) | LCDR2 | GASSLQS |
| SEQ ID NO: 259 (Kabat) | LCDR3 | QQYSSFPTT |
| SEQ ID NO: 260 (Chothia) | HCDR1 | GFTFSSY |
| SEQ ID NO: 261 (Chothia) | HCDR2 | N**T**QGKS |
| SEQ ID NO: 262 (Chothia) | HCDR3 | WGDEGFDI |
| SEQ ID NO: 263 (Chothia) | LCDR1 | SQGISNW |
| SEQ ID NO: 264 (Chothia) | LCDR2 | GAS |
| SEQ ID NO: 265 (Chothia) | LCDR3 | YSSFPT |
| SEQ ID NO: 266 | VL | |
| SEQ ID NO: 267 | VH | |
| SEQ ID NO: 268 | DNA VL | |
| SEQ ID NO: 269 | DNA VH | |
| SEQ ID NO: 270 | Light Kappa | |
| SEQ ID NO: 271 | Heavy IgG1 | |

| **MOR10701 R55S** | | |
|---|---|---|
| SEQ ID NO: 272 (Kabat) | HCDR1 | SYAMS |
| SEQ ID NO: 273 (Kabat) | HCDR2 | VTGAVGS**S**TYYPDSVKG |
| SEQ ID NO: 274 (Kabat) | HCDR3 | WGDEGFDI |
| SEQ ID NO: 275 (Kabat) | LCDR1 | RASQGISNWLA |
| SEQ ID NO: 276 (Kabat) | LCDR2 | GASSLQS |
| SEQ ID NO: 277 (Kabat) | LCDR3 | QQYSSFPTT |
| SEQ ID NO: 278 (Chothia) | HCDR1 | GFTFSSY |
| SEQ ID NO: 279 (Chothia) | HCDR2 | GAVG**S**S |
| SEQ ID NO: 280 (Chothia) | HCDR3 | WGDEGFDI |
| SEQ ID NO: 281 (Chothia) | LCDR1 | SQGISNW |
| SEQ ID NO: 282 (Chothia) | LCDR2 | GAS |
| SEQ ID NO: 283 (Chothia) | LCDR3 | YSSFPT |
| SEQ ID NO: 284 | VL | |
| SEQ ID NO: 285 | VH | |
| SEQ ID NO: 286 | DNA VL | |
| SEQ ID NO: 287 | DNA VH | |
| SEQ ID NO: 288 | Light Kappa | |
| SEQ ID NO: 289 | Heavy IgG1 | |

| **MOR10701 R55G** | | |
|---|---|---|
| SEQ ID NO: 290 (Kabat) | HCDR1 | SYAMS |
| SEQ ID NO: 291 (Kabat) | HCDR2 | VTGAVG**G**STYYPDSVKG |
| SEQ ID NO: 292 (Kabat) | HCDR3 | WGDEGFDI |
| SEQ ID NO: 293 (Kabat) | LCDR1 | RASQGISNWLA |
| SEQ ID NO: 294 (Kabat) | LCDR2 | GASSLQS |
| SEQ ID NO: 295 (Kabat) | LCDR3 | QQYSSFPTT |
| SEQ ID NO: 296 (Chothia) | HCDR1 | GFTFSSY |
| SEQ ID NO: 297 (Chothia) | HCDR2 | GAVG**G**S |
| SEQ ID NO: 298 (Chothia) | HCDR3 | WGDEGFDI |
| SEQ ID NO: 299 (Chothia) | LCDR1 | SQGISNW |
| SEQ ID NO: 300 (Chothia) | LCDR2 | GAS |
| SEQ ID NO: 301 (Chothia) | LCDR3 | YSSFPT |
| SEQ ID NO: 302 | VL | |
| SEQ ID NO: 303 | VH | |
| SEQ ID NO: 304 | DNA VL | |
| SEQ ID NO: 305 | DNA VH | |
| SEQ ID NO: 306 | Light Kappa | |
| SEQ ID NO: 307 | Heavy IgG1 | |
| | | |

| **MOR10701 R55K** | | |
|---|---|---|
| SEQ ID NO: 308 (Kabat) | HCDR1 | SYAMS |
| SEQ ID NO: 309 (Kabat) | HCDR2 | VTGAVG**K**STYYPDSVKG |
| SEQ ID NO: 310 (Kabat) | HCDR3 | WGDEGFDI |
| SEQ ID NO: 311 (Kabat) | LCDR1 | RASQGISNWLA |
| SEQ ID NO: 312 (Kabat) | LCDR2 | GASSLQS |
| SEQ ID NO: 313 (Kabat) | LCDR3 | QQYSSFPTT |
| SEQ ID NO: 314 (Chothia) | HCDR1 | GFTFSSY |
| SEQ ID NO: 315 (Chothia) | HCDR2 | GAVG**K**S |
| SEQ ID NO: 316 (Chothia) | HCDR3 | WGDEGFDI |
| SEQ ID NO: 317 (Chothia) | LCDR1 | SQGISNW |
| SEQ ID NO: 318 (Chothia) | LCDR2 | GAS |
| SEQ ID NO: 319 (Chothia) | LCDR3 | YSSFPT |
| SEQ ID NO: 320 | VL | |
| SEQ ID NO: 321 | VH | |
| SEQ ID NO: 322 | DNA VL | |
| SEQ ID NO: 323 | DNA VH | |
| SEQ ID NO: 324 | Light Kappa | |
| SEQ ID NO: 325 | Heavy IgG1 | |

| **MOR10701 deletion S56** | | |
|---|---|---|
| SEQ ID NO: 326 (Kabat) | HCDR1 | SYAMS |
| SEQ ID NO: 327 (Kabat) | HCDR2 | VTGAVGRTYYPDSVKG |
| SEQ ID NO: 328 (Kabat) | HCDR3 | WGDEGFDI |
| SEQ ID NO: 329 (Kabat) | LCDR1 | RASQGISNWLA |
| SEQ ID NO: 330 (Kabat) | LCDR2 | GASSLQS |
| SEQ ID NO: 331 (Kabat) | LCDR3 | QQYSSFPTT |
| SEQ ID NO: 332 (Chothia) | HCDR1 | GFTFSSY |
| SEQ ID NO: 333 (Chothia) | HCDR2 | GAVGRT |
| SEQ ID NO: 334 (Chothia) | HCDR3 | WGDEGFDI |
| SEQ ID NO: 335 (Chothia) | LCDR1 | SQGISNW |
| SEQ ID NO: 336 (Chothia) | LCDR2 | GAS |
| SEQ ID NO: 337 (Chothia) | LCDR3 | YSSFPT |
| SEQ ID NO: 338 | VL | |
| SEQ ID NO: 339 | VH | |
| SEQ ID NO: 340 | DNA VL | |
| | | |
| SEQ ID NO: 341 | DNA VH | |
| SEQ ID NO: 342 | Light Kappa | |
| SEQ ID NO: 343 | Heavy IgG1 | |

| **MOR12609** | | |
|---|---|---|
| SEQ ID NO: 344 (Kabat) | HCDR1 | SYAMS |
| SEQ ID NO: 345 (Kabat) | HCDR2 | VINGLGYTTFYADSVKG |
| SEQ ID NO: 346 (Kabat) | HCDR3 | WGDEGFDI |
| SEQ ID NO: 347 (Kabat) | LCDR1 | RASQGISNWLA |
| SEQ ID NO: 348 (Kabat) | LCDR2 | GASSLQS |
| SEQ ID NO: 349 (Kabat) | LCDR3 | QQYSSFPTT |
| SEQ ID NO: 350 (Chothia) | HCDR1 | GFTFSSY |
| SEQ ID NO: 351 (Chothia) | HCDR2 | NGLGYT |
| SEQ ID NO: 352 (Chothia) | HCDR3 | WGDEGFDI |
| SEQ ID NO: 353 (Chothia) | LCDR1 | SQGISNW |
| SEQ ID NO: 354 (Chothia) | LCDR2 | GAS |
| SEQ ID NO: 355 (Chothia) | LCDR3 | YSSFPT |
| SEQ ID NO: 356 | VL | |
| SEQ ID NO: 357 | VH | |
| SEQ ID NO: 358 | DNA VL | |
| SEQ ID NO: 359 | DNA VH | |
| SEQ ID NO: 360 | Light Kappa | |
| SEQ ID NO: 361 | Heavy IgG1 | |

| **MOR12610** | | |
|---|---|---|
| SEQ ID NO: 362 (Kabat) | HCDR1 | SYAMS |
| SEQ ID NO: 363 (Kabat) | HCDR2 | GTGPYGGTYYPDSVKG |
| SEQ ID NO: 364 (Kabat) | HCDR3 | WGDEGFDI |
| SEQ ID NO: 365 (Kabat) | LCDR1 | RASQGISNWLA |
| SEQ ID NO: 366 (Kabat) | LCDR2 | GASSLQS |
| SEQ ID NO: 367 (Kabat) | LCDR3 | QQYSSFPTT |
| SEQ ID NO: 368 (Chothia) | HCDR1 | GFTFSSY |
| SEQ ID NO: 369 (Chothia) | HCDR2 | GPYGG |
| SEQ ID NO: 370 (Chothia) | HCDR3 | WGDEGFDI |
| SEQ ID NO: 371 (Chothia) | LCDR1 | SQGISNW |
| SEQ ID NO: 372 (Chothia) | LCDR2 | GAS |
| SEQ ID NO: 373 (Chothia) | LCDR3 | YSSFPT |
| SEQ ID NO: 374 | VL | |
| SEQ ID NO: 375 | VH | |
| SEQ ID NO: 376 | DNA VL | |
| SEQ ID NO: 377 | DNA VH | |
| SEQ ID NO: 378 | Light Kappa | |
| SEQ ID NO: 379 | Heavy IgG1 | |
| | | |

In accordance with the present disclosure the compounds in the pharmaceutical combination, components (i) ceritinib, or a pharmaceutically acceptable salt thereof, and (ii) an EGFR inhibitor, or a pharmaceutically acceptable salt thereof can be administered separately or together.

The pharmaceutical combination, according to the present disclosure, for use as a medicine, wherein ceritinib and the EGFR inhibitor are administered independently at the same time or separately within time intervals, wherein time intervals allow that the combination partners are jointly active.

The term "pharmaceutical combination" as used herein refers to a product obtained from mixing or combining in a non-fixed combination the active ingredients, e.g. (i) ceritinib, or a pharmaceutically acceptable salt thereof, and (ii) an EGFR inhibitor or a pharmaceutically acceptable salt thereof separately or together.

The term "combination" refers to formulations of the separate partners with or without instructions for combined use or to combination products. The combined compounds can be manufactured and/or formulated by the same or different manufacturers. The combination partners may thus be entirely separate pharmaceutical dosage forms or pharmaceutical compositions that are also sold independently of each other and where just instructions for their combined use are provided: (i) prior to release to physicians (*e.g*. in the case of a "kit of part" comprising the compound of the disclosure and the other therapeutic agent); (ii) by the physician themselves (or under the guidance of a physician) shortly before administration; (iii) the patient themselves by a physician or medical staff.

The term "non-fixed combination" means that the active ingredients, *e.g*. ceritinib and an EGFR tyrosine kinase inhibitor, are both administered separately or together, independently at the same time or separately within time intervals, wherein such administration provides therapeutically effective levels of the active ingredient in the subject in need. The latter also applies to cocktail therapy, *e.g*. the administration of three or more active ingredients. This term defines especially a "kit of parts" in the sense that the combination partners (i) ceritinib and (ii) EGFR inhibitor (and if present further one or more coagents) as defined herein can be dosed independently of each other. Nevertheless, it is contemplated herein that ceritinib and an EGFR inhibitor could be administered in a reduced dose compared to the respective doses used when the drugs are used alone. Particularly this can be advantageous in case tolerability and drug related adverse events are problematic when using the compound. Drug dose reduction is such instances could help to keep the subject, *e.g*. patient, on the drug, while adding the combination partner. Overall, such approach bestows the clinical team with better flexibility as to the treatment options for the subject. One example of such combination where the reduced doses of either one, or both, of the drugs is administered is the combination of ceritinib and afatinib. It would be expected that gastrointestinal side effects, for example, diarrhea, could be adequately mitigated or at least reduced.

The present disclosure also includes the pharmaceutical combination according to the disclosure, wherein the EGFR inhibitor is erlotinib, gefitinib or cetuximab.

In another embodiment, the pharmaceutical combination according to the disclosure comprises as an EGFR inhibitor an isolated antibody or fragment thereof to a HER3 receptor comprising 1, 2, 3, 4, 5, or 6 CDRs calculated by Kabat or Chothia of any of the antibodies shown in Table 1.

A further embodiment of the disclosure provides a combination (*e.g*. combination product) comprising a quantity which is jointly therapeutically effective for the treatment of an ALK and/or EGFR mediated disease. The term "jointly therapeutically effective" means that the compounds show synergistic interaction when administered separately or together, independently at the same time or separately within time intervals, to treat a subject in need, such as a warm-blooded animal in particular a human.

The term "mediated disease" means that the condition or the disease lacks a definitive etiology, but is characterized by a common pathway leading to a medical disorder such as dysregulation, inflammation, immune response, cell growth, cell apoptosis, allergy.

It was shown that the combination of the present disclosure possesses beneficial therapeutic properties, *e.g.* synergistic interaction, strong *in-vivo* and *in-vitro* antitumor response, which can be used as a medicine. Its characteristics render it particularly useful for the treatment of cancer.

Suitable cancers that can be treated with the combination of the present disclosure include but are not limited to anaplastic large cell lymphoma (ALCL), neuroblastoma, lung cancer, non-small cell lung cancer (NSCLC). In a preferred embodiment, the cancer is NSCLC. In one embodiment, the cancer comprises wt EGFR. In another embodiment, the cancer comprises T790M EGFR.

A further embodiment of the disclosure relates to the pharmaceutical combination according to the present disclosure, wherein the ALK and/or EGFR mediated disease is NSCLC.

The combination according to the present disclosure can besides or in addition be administered especially for cancer therapy in combination with chemotherapy, radiotherapy, immunotherapy, surgical intervention, or in combination of these. Long-term therapy is equally possible as is adjuvant therapy in the context of other treatment strategies, as described above. Other possible treatments are therapy to maintain the patient's status after tumor regression, or even chemo-preventive therapy, for example in patients at risk.

The terms "treat", "treating" or "treatment" of any disease or disorder refers to ameliorating the disease or disorder (*e.g*. slowing or arresting or reducing the development of the disease or at least one of the clinical symptoms thereof), to preventing or delaying the onset or development or progression of the disease or disorder. In addition those terms refers to alleviating or ameliorating at least one physical parameter including those which may not be discernible by the patient and also to modulating the disease or disorder, either physically (*e.g.* stabilization of a discernible symptom), physiologically (*e.g.* stabilization of a physical parameter), or both.

The term "treatment" comprises, for example, the therapeutic administration of the combination partners to a warm-blooded animal, in particular a human being, in need of such treatment with the aim to cure the disease or to have an effect on disease regression or on the delay of progression of a disease.

The term "subject in need" refers to a warm-blooded animal, in particular a human being that would benefit biologically, medically or in quality of life from the treatment.

The combination of ceritinib and EGFR inhibitor can be used to manufacture a medicament for an ALK and/or EGFR mediated disease as described above. Likewise the combination can be used in a method for the treatment of an ALK and/or an EGFR mediated disease, as described above, said method comprising administering an effective amount of a combination of (i) ceritinib, or a pharmaceutically acceptable salt thereof, and (ii) an EGFR inhibitor or a pharmaceutically acceptable salt thereof separately or together, to a subject in need thereof, according to the present disclosure.

For example, the term "jointly (therapeutically) active" may mean that the compounds may be given separately or sequentially (in a chronically staggered manner, especially a sequence specific manner) in such time intervals that they preferably, in the warm-blooded animal, especially human, to be treated, and still show a (preferably synergistic) interaction (joint therapeutic effect). A joint therapeutic effect can, *inter alia,* be determined by following the blood levels, showing that both compounds are present in the blood of the human to be treated at least during certain time intervals, but this is not to exclude the case where the compounds are jointly active although they are not present in blood simultaneously.

Subject or patient that can get the combination administered encompasses mammals and non-mammals. Examples of mammals include, but are not limited to, humans, chimpanzees, apes, monkeys, cattle, horses, sheep, goats, swine; rabbits, dogs, cats, rats, mice, guinea pigs, and the like. Examples of non-mammals include, but are not limited to, birds, fish and the like. In a most preferred embodiment, the subject or patient is human. It may be a human who has been diagnosed as in need of treatment for a disease or disorder disclosed herein.

The present disclosure also describes the method for the treatment of an ALK and/or an EGFR mediated disease, wherein the combination of (i) ceritinib, or a pharmaceutically acceptable salt thereof, and (ii) an EGFR inhibitor or a pharmaceutically acceptable salt thereof separately or together.

The present disclosure relates to a pharmaceutical composition comprising effective amounts of (i) ceritinib, or a pharmaceutically acceptable salt thereof, and (ii) the EGFR inhibitor, or a pharmaceutically acceptable salt thereof. The pharmaceutical composition can be prepared with a pharmaceutically acceptable carrier, which can be for example any suitable pharmaceutical excipient. The carrier includes any and all binders, fillers, solvents, dispersion media, coatings, surfactants, antioxidants, preservatives (e.g., antibacterial agents, antifungal agents), isotonic agents, absorption delaying agents, salts, drug stabilizers, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329; Remington: The Science and Practice of Pharmacy, 21st Ed. Pharmaceutical Press 2011; and subsequent versions thereof). Except insofar as any conventional carrier is incompatible with the active ingredient, its use in the therapeutic or pharmaceutical compositions is contemplated.

In accordance with the present disclosure the combination partners can be administered independently at the same time or separately within time intervals in separate unit dosage forms. The two therapeutic partners may be prepared in a manner known per se and are suitable for enteral, such as oral or rectal, topical and parenteral administration to subject in need thereof, including warm-blooded animal, in particular a human being. Suitable pharmaceutical compositions contain, e.g. from about 0.1% to about 99.9% of active ingredient.

The pharmaceutical composition can be processed to prepare a final dosage form - a tablet or a capsule. This can be achieved by compressing the final blend of the combination, optionally together with one or more excipients. The compression can be achieved for example with a rotary tablet press. Tablet of different shapes can be prepared (round, ovaloid, or other suitable shape). The tablet can be coated or uncoated by known techniques to delay disintegration and absorption in the gastrointestinal tract and thereby provide a sustained action over a longer period. If not indicated otherwise, these are prepared in a manner known per se, e.g. by means of mixing, granulating, sugar-coating processes. Formulation for oral use can be presented as hard gelatin capsules wherein the active ingredient is mixed with an inert solid diluent, for example, calcium carbonate, calcium phosphate or cellulose-based excipient, or as soft gelatin capsules wherein the active ingredient is mixed with water or an oil medium, for example, olive oil, liquid paraffin or peanut oil.

The term "effective amount" means the amount of the subject compound that will engender a biological or medical response in a cell, tissue, organ, system, animal or human that is being sought by the researcher, veterinarian, medical doctor or other clinician. The effective dosage of each combination partner agents employed in the combination of the invention may vary depending on the particular compound or pharmaceutical composition employed, the mode of administration, the condition being treated, the severity the condition being treated. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the combination's drugs availability to target sites. This involves a consideration of the distribution, equilibrium and elimination of a drug.

The present disclosure also describes the pharmaceutical combination according to the present disclosure in the form of a "kit of parts" for the combined administration. The combination can refer to either a fixed combination in one dosage unit form, or a kit of parts for the combined administration where (i) ceritinib, or a pharmaceutically acceptable salt thereof, and (ii) EGFR inhibitor, or a pharmaceutically acceptable salt thereof, may be administered independently at the same time or separately within time intervals, especially where these time intervals allow that the combination partners show a cooperative (= joint) effect. The independent formulations or the parts of the formulation, product, or composition, can then, *e.g.* be administered simultaneously or chronologically staggered, that is at different time points and with equal or different time intervals for any part of the kit of parts. In the combination therapies of the disclosure, the compounds useful according to the disclosure may be manufactured and/or formulated by the same or different manufacturers. Moreover, the combination partners may be brought together into a combination therapy: (i) prior to release of the combination product to physicians (e.g. in the case of a kit comprising ceritinib and the EGFR inhibitor); (ii) by the physician themselves (or under the guidance of a physician) shortly before administration; (iii) in the patient themselves, *e.g*. during sequential administration of the compound of the disclosure and the other therapeutic agent. In one embodiment the effect of the combination is synergistic.

The therapeutically effective dosage of the combination of the disclosure, or pharmaceutical composition, is dependent on the species of the subject, the body weight, age and individual condition, the disorder or disease or the severity thereof being treated, and can be determined by standard clinical techniques. In addition, *in vitro* or *in vivo* assays can optionally be employed to help identify optimal dosage ranges. The precise dose to be employed can also depend on the route of administration, and the seriousness of the condition being treated and can be decided according to the judgment of the practitioner and each subject's circumstances in view of, e.g., published clinical studies. In general, satisfactory results are indicated to be obtained systemically at daily dosages of from 150 mg to 750 mg of ceritinib orally. In most cases, the daily dose for ceritinib can be between 300 mg and 750 mg. For example gefitinib daily dose can be for example 250 mg orally. Erlotinib can be combined in the usual dose of 150 mg each day, particularly in case when the patient has non-small cell lung cancer. In some cases, 100 mg erlotinib can be given each day. The erlotinib dose may also be adjusted in 50 mg steps.

Cetuximab, for example, is administered once a week with the initial dose of 400 mg cetuximab per m2 body surface area. All subsequent weekly doses are 250 mg cetuximab per m2 each.

For example, Afatinib can administered once daily in dosages from 20 mg to 40 mg.

For example, nimotuzumab can be administered daily in 150mg/m2.

The term "pharmaceutically acceptable" refers to those compounds, materials, compositions, and/or dosage forms which are suitable for use in contact with the tissues of human beings and animals without excessive toxicity, irritation, allergic response, or other problem or complication, commensurate with a reasonable benefit/risk ratio.

Another aspect of the disclosure is ceritinib for use as a medicine, wherein ceritinib, or a pharmaceutically acceptable salt thereof, is to be administered in combination with an EGFR inhibitor, or a pharmaceutically acceptable salt thereof, for the treatment of an ALK and/or EGFR mediated disease, e.g. cancer.

The term "ALK and/or EGFR mediated disease" refers to a disease in which activity of one or both kinases leads to abnormal activity of the regulatory pathways including overexpression, mutation or relative lack of activity of other regulatory pathways in the cell, e.g. cancer.

### Abbreviations

ALCL: Anaplastic Large Cell Lymphoma
ALK: Anaplastic Lymphoma Kinase.
NSCLC: Non-Small Cell Lung Cancer.
EGFR: Epidermal Growth Factor Receptor.
TKI: Tyrosine Kinase Inhibitor.

The following Examples illustrate the disclosure and provide specific embodiments, however without limiting the scope of the disclosure.

### Example 1. EGFR ligands desensitize H2228 cell line to ceritinib treatment

NCI-H2228 was obtained from ATCC. The cell lines harbored EML4-ALK rearrangements. NCI-H2228 cells were cultured in RPMI-1640 (ATCC Catalog # 30-2001) supplemented with 15% FBS.

Secretome screening was performed as described previously (Lito P, Pratilas CA, et al. Cancer Cell 2012; 22: 668-82). In brief, 317 cDNA constructs that represent 220 unique secreted proteins were reverse transfected into HEK293T cells individually and incubated 4 days to allow accumulation of secreted proteins in supernatant. The supernatant was transferred to the assay cells, namely NCI-H2228 cells, followed by addition of ceritinib to a final concentration of 0.5 µM. Cell proliferation was measured using CellTiter-Glo after 96 hours. Cells treated with DMSO in the absence of conditioned media and with ceritinib alone were used as controls.

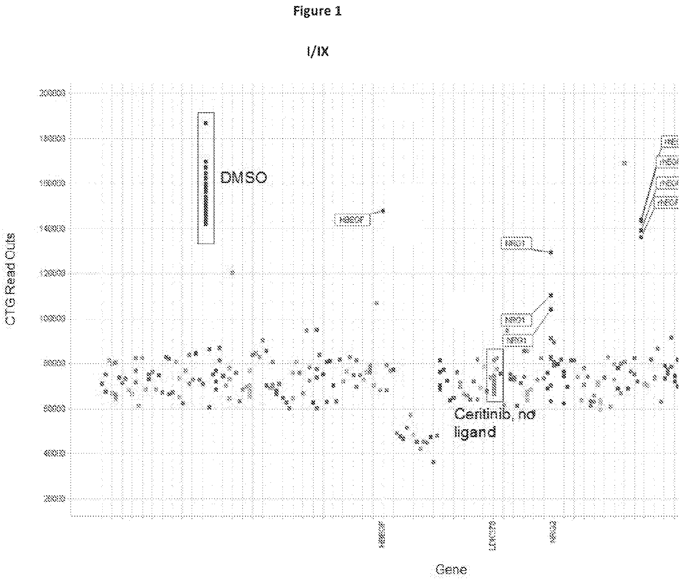

The Cell Titer Glo assay was done with H2228 cell line. The cell line stems from adenocarcinoma; non-small cell lung cancer. Cell Titer Glo assay is a Cell Viability Assay that determined the number of viable cells in culture based on quantitation of the ATP present, which signaled the presence of metabolically active cells. **Figure 1** shows the results.

"Ceritinib no ligand" represents cells that were treated with ceritinib only (absence of any ligand) and represents the impact of ceritinib on cell viability.

"DMSO" represents the basic cell viability (control level) in the presence of a vehicle, but without any compound and all other dots represent ceritinib in the presence of a specific ligand. When cell viability level shifted from the low level of "Ceritinib no ligand" toward DMSO cell viability levels, it means that the particular ligand was able to activate pathway(s) that compensated for the loss of viability due to inhibition of ALK signaling. Ligands for EGFR and ERBB3 clearly overcame the inhibitory effect of ceritinib and could push the cell viability toward the levels observed when cells were treated with DMSO only. EGFR signaling can bypass the ALK inhibition.

The H2228 cell line represents well patient naïve settings - settings where the subject (e.g. patient) has not been pretreated with an ALK inhibitor. Therefore, the experiment indicated that the pharmaceutical combination comprising ceritinib and an EGFR inhibitor would be effective already in naïve patient.

### Example 2. Cell proliferation assay of MGH049 and MGH051 cells

MGH049 and MGH051 were obtained from Massachusetts General Hospital (Friboulet L, Li N, et al. Cancer Discovery 2014; 4: 662-73). These cell lines harbor EML4-ALK rearrangements. MGH049 and MGH051 were cultured in DMEM (ATCC Catalog # 30-2002) supplemented with 10% FBS.

To determine the dose-response relationship between the dose of ceritinib and the magnitude of its effect on cell proliferation, 1K MGH049 and 4K MGH051 cells were plated in each well of 384 well-plates, and grown for 24 hours prior to treatment. Cells were then treated with DMSO or ceritinib at concentrations ranging from 4 nM to 1 µM (3-fold dilutions). After 6 days, cell proliferation was measured using the CellTiter-Glo luminescent cell viability assay. Percent inhibition was calculated relative to median DMSO signal.

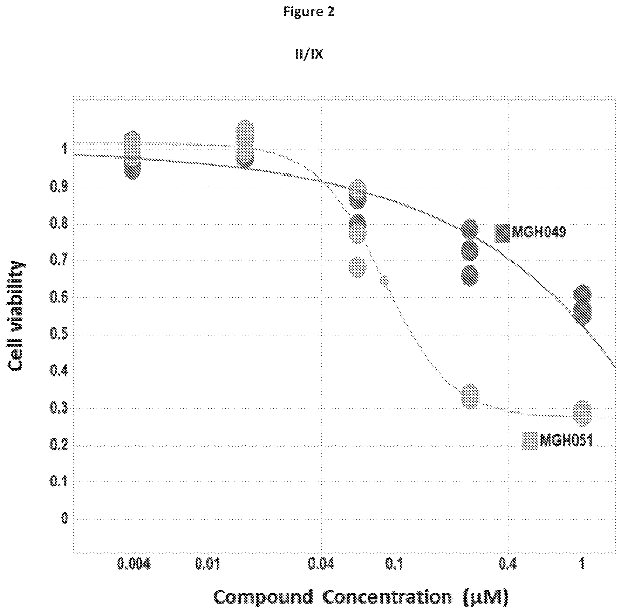

**Figure 2** shows the results of the cell proliferation assay after MGH049 and MGH051 cells were treated with the indicated doses of ceritinib for 6 days. MGH051 is more sensitive to ceritinib treatment than MGH049. The percent inhibition in MGH051 cells is 70% at 0.3 µM ceritinib, while MGH049 is 20%.

### Example 3. Ceritinib in combination with EGFR inhibitors reduce cell growth

1K MGH049 and 4K MGH051 cells were plated in each well of 384 well-plates, and treated with escalating doses of lapatinib, erlotinib, gefitinib and Compound A in the following day, in the absence or presence of 0.5 µM ceritinib. At the end of 6 days, inhibition of cell proliferation was assessed using CellTiter-Glo. Three replicate plates were set up for each cell line and drug compound, with or without ceritinib. A representative dose response curve was then calculated by taking the mean across the 3 replicates with and without ceritinib. Proliferation inhibition values were normalized to the measured inhibition value at zero dose of the compound, with and without ceritinib.

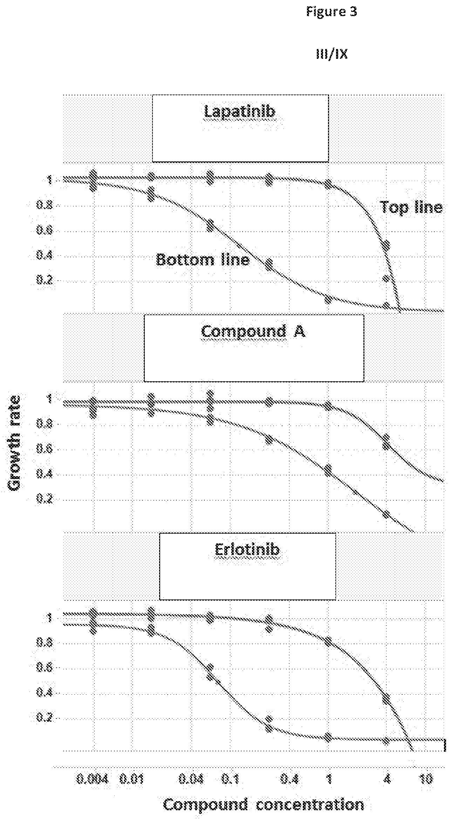

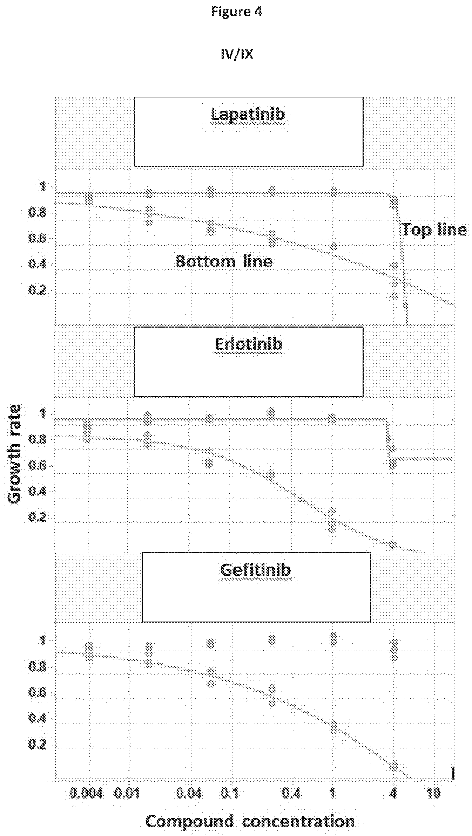

Experiments performed with cell lines MGH049 and MGH051 are depicted on **Figure 3** and **Figure 4****,** respectively. Cell growth was observed while adding different concentration of each of the EGFR inhibitors (gefitinib, erlotinib, lapatinib or compound A) either singly (top line of the graphs) or each of the EGFR inhibitors in combination with 0.5 µM ceritinib (bottom line on the graphs). From the figures it is clear that the combination synergistically reduced cell growth. Cell line MGH049 was resistant to ceritinib but did not bear any ALK resistance mutations.

The experiment showed that the combination of ceritinib and an EGFR inhibitor was effective even in ALK resistant settings, i.e. where the cells were resistant to the treatment of ceritinib alone. Therefore, the combination provided herein would be useful in cases where patients have been previously treated with an ALK inhibitor (for example post crizotinib, or post ceritinib treatment).

Meaningful data obtained with the combination of ceritinib and gefitinib, erlotinib, lapatinib or compound A, respectively, indicated, that the combination of ceritinib with other EGFR inhibitors is most likely, if not certain, also very advantageous and valuable.

### Example 4. Treatment of ALK Positive NSCLC Cell Lines Is Associated with Activation of HER3 (ErbB3)

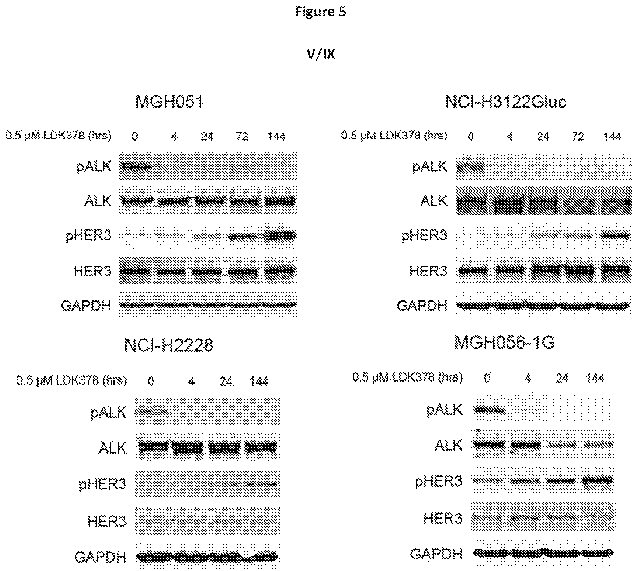

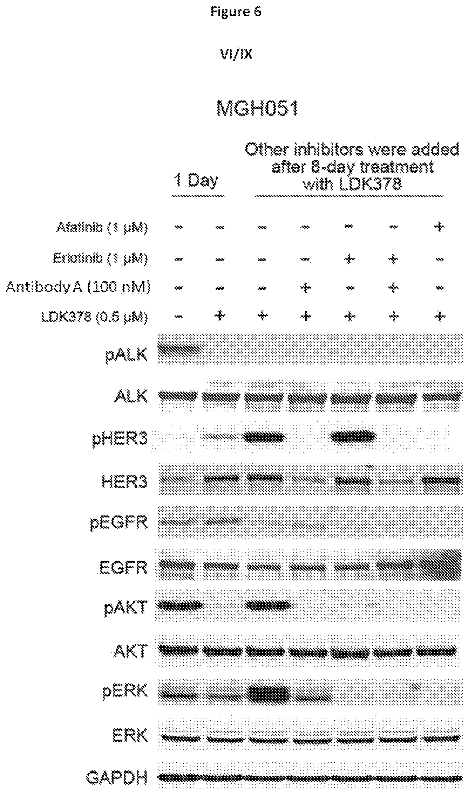

The activated states of ALK and HER3 of multiple NSCLC cell lines that harbor EML4-ALK translocations were assessed in both short-term and long-term cultures. We observed that long-term treatments of 4 ALK positive cell lines with ceritinib (LDK378) led to an induction of HER3 phosphorylation, indicating the activation of HER3 receptors (Fig.5). In addition, the levels of pHER3 correlated with the duration of the treatments (Fig. 5). Next, we assessed the activation of the downstream signaling of HER3 and the repression of ceritinib-induced HER3 activation using various inhibitors. After initial inhibition of AKT phosphorylation in MGH051, the 8-day treatment of ceritinib resulted in a profound rebound in AKT phosphorylation, accompanying the increased phosphorylation of HER3 (Fig. 6). Addition of Antibody A (MOR10703 from table 1), an anti-HER3 antibody, for the last day of the long-term treatment markedly suppressed the activation of HER3 and the rebound in AKT phosphorylation (Fig. 6). The EGFR inhibitor erlotinib and the pan-HER inhibitor afatinib were also able to suppress the rebound in AKT phosphorylation (Fig. 6), suggesting that the re-activation of the PI3K pathway by HER3 is EGFR-dependent. Furthermore, addition of erlotinib and afatinib for the last day of the treatment led to enhanced inhibition of ERK phosphorylation (Fig. 6). These results suggested that a feedback activation of HER3 is elicited by ALK inhibition, and provided a rational for a therapeutic strategy of combining ALK and HER3 inhibitors or combining ALK and EGFR inhibitors.

### Example 5. EGFR Inhibition Enhances the Inhibition of ALK Positive NSCLC Cell Growth by CERITINIB

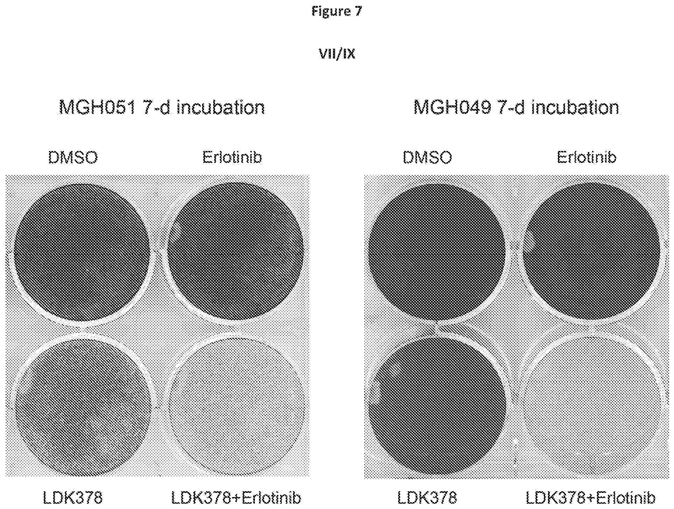

To assess the anti-proliferative effect of ceritinib (LDK378) in combination with the EGFR inhibitor erlotinib, we applied cell colony formation assays using MGH049 and MGH051 cells. Cells were exposed to ceritinib, erlotinib or the combination for 7 days, and stained with crystal violet. As depicted in Figure 7, ceritinib was partially effective in inhibiting the cell growth of MGH051, but less effective in MGH049 cells. Ceritinib and erlotinib combination had greater anti-proliferative effect and was more efficacious in killing MGH049 and MGH051 cells than ceritinib alone (Fig. 7). Erlotinib had no effect on cell growth as a single agent, suggesting that these cell lines are ALK-dependent and EGFR signaling functions as a survival pathway or confers resistance to ceritinib (Fig. 7). These results suggested that combination of ALK and EGFR inhibition can either enhance the anti-tumor activity of ceritinib or overcome ceritinib resistance in ALK positive NSCLC.

### Example 6. Efficacy of ceritinib (LDK378) alone and in combination with cetuximab and/or Antibody A (MOR10703 from table 1) in H2228 NSCLC xenograft in female SCID-beige mice

In this experiment female SCID Beige mice bearing H2228 tumors were used. At start, the mice were 8 to 12 weeks of age. First, the mice were inoculated with 4 mm³ H2228 tumor fragments by subcutaneous injection in flank. The fragments were dipped in Matrigel before implanting. When tumors reached an average size of 100 - 150 mm³ the treatment according to the scheme below commenced. The body weight was measured daily for the first 5 days and then biweekly until end. The tumor was measured biweekly by caliper until end. Ceritinib (LDK378) was administered *per os* and cetuximab and the Antibody A (Ab A; MOR10703 from table 1) were administered intraperitoneally (*ip*)*.* Dosing with the compounds ended on study day 32. After this the tumor size was monitored during an observation phase for regrowth.

| **#** | **N of mice** | **Regimen 1** | | | **Regimen 2** | | | **Regimen 3** | | |
|---|---|---|---|---|---|---|---|---|---|---|
| | | **Agent** | **mg/kg** | **Schedule** | **Agent** | **mg/kg** | **Schedule** | **Agent** | **mg/kg** | **Schedule** |
| 1^{#} | 8 | vehicle | - | qd x 32 | - | - | - | - | - | - |
| 2 | 7 | LDK378 | 25 | qd x 32 | - | - | - | - | - | - |
| 3 | 7 | LDK378 | 50 | qd x 32 | - | - | - | - | - | - |
| 4 | 7 | LDK378 | 100 | qd x 32 | - | - | - | - | - | - |
| 5 | 7 | cetuximab | 20 | biwk x 5 | - | - | - | - | - | - |
| 6 | 7 | Ab A | 25 | qod x 17 | - | - | - | - | - | - |
| 7 | 8 | Ab A | 25 | qod x 17 | cetuximab | 20 | biwk x 5 | - | - | - |
| 8 | 8 | LDK378 | 25 | qd x 32 | cetuximab | 20 | biwk x 5 | - | - | - |
| 9 | 8 | LDK378 | 50 | qd x 32 | cetuximab | 20 | biwk x 5 | - | - | - |
| 10 | 8 | LDK378 | 25 | qd x 32 | Ab A | 25 | qod x 17 | - | - | - |
| 11 | 8 | LDK378 | 50 | qd x 32 | Ab A | 25 | qod x 17 | - | - | - |
| 12 | 8 | LDK378 | 25 | qd x 32 | cetuximab | 20 | biwk x 5 | Ab A | 25 | qod x 17 |
| 13 | 8 | LDK378 | 50 | qd x 32 | cetuximab | 20 | biwk x 5 | Ab A | 25 | qod x 17 |

| | | | | | | | | | | |
|---|---|---|---|---|---|---|---|---|---|---|
| - Control group | | | | | | | | | | |

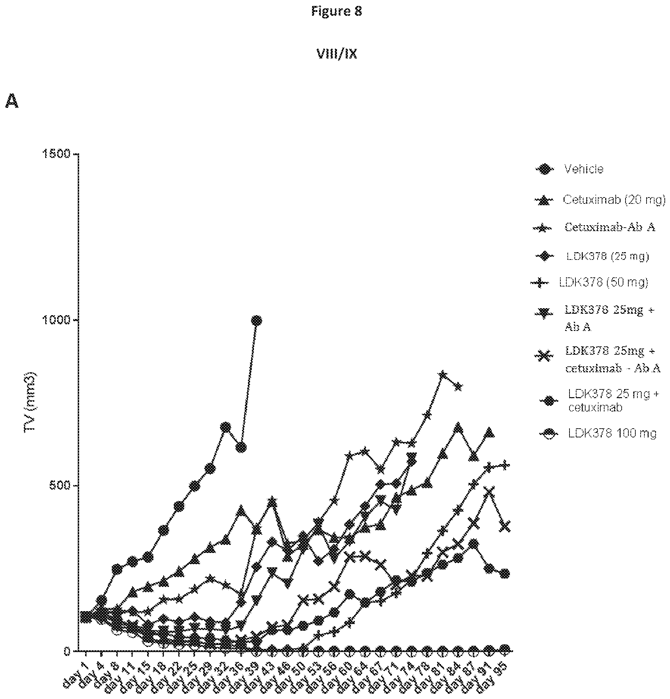

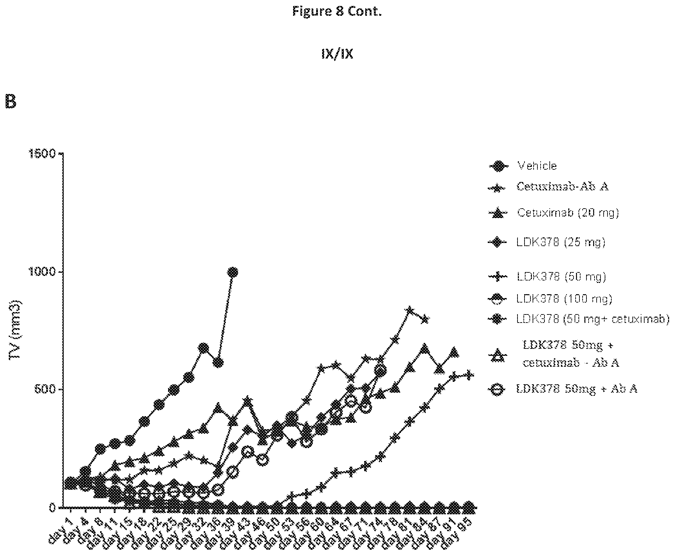

Results are depicted in Figure 8A and 8B. The average tumor volume data in the graphs is not complete through the end of the study for all groups. Once a group lost more than 50% of the animals due to death or ethical sacrifice, the average tumor volume for the remaining animals in these groups was no longer graphed. The data showed that the combination of either of the Antibody A or cetuximab significantly improved efficacy of LDK378.

## Claims

1. A pharmaceutical combination comprising (i) ceritinib, or a pharmaceutically acceptable salt thereof, and (ii) an EGFR inhibitor, or a pharmaceutically acceptable salt thereof, wherein the EGFR inhibitor is selected from the group consisting of erlotinib, gefitinib, lapatinib, canertinib, pelitinib, neratinib, (*R,E*)-*N*-(7-chloro-1-(1-(4-(dimethylamino)but-2-enoyl)azepan-3-yl)-1H-benzo[d]imidazol-2-yl)-2-methylisonicotinamide, panitumumab, matuzumab, pertuzumab, nimotuzumab, zalutumumab, icotinib, afatinib and cetuximab, and pharmaceutically acceptable salt thereof, or the EGFR inhibitor is an isolated antibody or fragment thereof comprising a heavy chain CDR3 selected from the group consisting of SEQ ID NO: 4, SEQ ID NO: 10, SEQ ID NO: 22, SEQ ID NO: 28, SEQ ID NO: 40, SEQ ID NO: 46, SEQ ID NO: 58, SEQ ID NO: 64, SEQ ID NO: 76, SEQ ID NO: 82, SEQ ID NO: 94, SEQ ID NO: 100, SEQ ID NO: 112, SEQ ID NO: 118, SEQ ID NO: 130, SEQ ID NO: 136, SEQ ID NO: 148, SEQ ID NO: 166, SEQ ID NO: 184, SEQ ID NO: 202, SEQ ID NO: 220, SEQ ID NO: 238, SEQ ID NO: 256, SEQ ID NO: 274, SEQ ID NO: 292, SEQ ID NO: 310, SEQ ID NO: 328, SEQ ID NO: 346, and SEQ ID NO: 364.

2. The pharmaceutical combination according to claim 1, wherein the EGFR inhibitor is an antibody or fragment thereof comprising the sequences of MOR10703 as defined in Table 1 (Antibody A) or cetuximab.

3. The pharmaceutical combination according to any of the claims 1 or 2, for use as a medicine.

4. Ceritinib for use as a medicine, wherein ceritinib, or a pharmaceutically acceptable salt thereof, is administered in combination with an EGFR inhibitor, or a pharmaceutically acceptable salt thereof, wherein the EGFR inhibitor is erlotinib, gefitinib, lapatinib, canertinib, pelitinib, neratinib, (*R*,*E*)-*N*-(7-chloro-1-(1-(4-(dimethylamino)but-2-enoyl)azepan-3-yl)-1H-benzo[d]imidazol-2-yl)-2-methylisonicotinamide, panitumumab, matuzumab, pertuzumab, nimotuzumab, zalutumumab, icotinib, afatinib, cetuximab, or a pharmaceutically acceptable salt thereof, or an isolated antibody or fragment thereof comprising a heavy chain CDR3 selected from the group consisting of SEQ ID NO: 4, SEQ ID NO: 10, SEQ ID NO: 22, SEQ ID NO: 28, SEQ ID NO: 40, SEQ ID NO: 46, SEQ ID NO: 58, SEQ ID NO: 64, SEQ ID NO: 76, SEQ ID NO: 82, SEQ ID NO: 94, SEQ ID NO: 100, SEQ ID NO: 112, SEQ ID NO: 118, SEQ ID NO: 130, SEQ ID NO: 136, SEQ ID NO: 148, SEQ ID NO: 166, SEQ ID NO: 184, SEQ ID NO: 202, SEQ ID NO: 220, SEQ ID NO: 238, SEQ ID NO: 256, SEQ ID NO: 274, SEQ ID NO: 292, SEQ ID NO: 310, SEQ ID NO: 328, SEQ ID NO: 346, and SEQ ID NO: 364.

5. Ceritinib for use as a medicine according to claim 4, for the treatment of cancer.

6. Ceritinib for use as a medicine according to claim 5, wherein the cancer is a non-small cell lung cancer.

7. Ceritinib for use as a medicine in combination, according to any one of claims 4 to 6, wherein the EGFR inhibitor is an antibody or fragment thereof comprising the sequences of MOR10703 as defined in Table 1 (Antibody A), erlotinib, gefitinib or cetuximab.

8. The pharmaceutical combination according to any one of claims 1 to 3, or ceritinib for use as a medicine according to any one of claims 4 to 7, wherein the EGFR inhibitor is gefitinib.

9. The pharmaceutical combination according to any one of claims 1 to 3, or ceritinib for use as a medicine according to any one of claims 4 to 7, wherein the EGFR inhibitor is erlotinib.

10. The pharmaceutical combination according to any one of claims 1 to 3, or ceritinib for use as a medicine according to any one of claims 4 to 7, wherein the EGFR inhibitor is cetuximab.

11. The pharmaceutical combination according to any one of claims 1 to 3, or ceritinib for use as a medicine according to any one of claims 4 to 7, wherein the EGFR inhibitor is an isolated antibody or fragment thereof that comprises a heavy chain variable region CDR1 of SEQ ID NO: 128; CDR2 of SEQ ID NO: 129; CDR3 of SEQ ID NO: 130; and a light chain variable region CDR1 of SEQ ID NO: 131; CDR2 of SEQ ID NO: 132; and CDR3 of SEQ ID NO: 133.

12. The pharmaceutical combination for use as a medicine according to any one of claims 3 or 8 to 11, or ceritinib for use as a medicine according to any one of claims 4 to 11, for the treatment of cancer, wherein the cancer comprises wt EGFR.

13. The pharmaceutical combination for use as a medicine according to any one of claims 3 or 8 to 11, or ceritinib for use as a medicine according to any one of claims 4 to 11, for the treatment of cancer, wherein the cancer comprises T790M EGFR.

14. The pharmaceutical combination for use as a medicine according to any one of claims 3 or 8 to 13, or ceritinib for use as a medicine according to any one of claims 4 to 13, wherein ceritinib and EGFR inhibitor are administered to an ALK-naïve patient.

15. The pharmaceutical combination for use as a medicine according to any one of claims 3 or 8 to 13, or ceritinib for use as a medicine according to any one of claims 4 to 13, wherein ceritinib and EGFR inhibitor are administered to a patient that has been pretreated with an ALK inhibitor.

## Patentansprüche

1. Pharmazeutische Kombination, umfassend (i) Ceritinib oder ein pharmazeutisch unbedenkliches Salz davon und (ii) einen EGFR-Inhibitor oder ein pharmazeutisch unbedenkliches Salz davon, wobei der EGFR-Inhibitor aus der Gruppe bestehend aus Erlotinib, Gefitinib, Lapatinib, Canertinib, Pelitinib, Neratinib, (*R,E*)-N-(7-Chlor-1-(1-(4-(dimethylamino)but-2-enoyl)azepan-3-yl)-1H-benzo-[d]imidazol-2-yl)-2-methylisonicotinamid, Panitumumab, Matuzumab, Pertuzumab, Nimotuzumab, Zalutumumab, Icotinib, Afatinib und Cetuximab und pharmazeutisch unbedenklichen Salzen davon ausgewählt ist oder es sich bei dem EGFR-Inhibitor um einen isolierten Antikörper oder ein Fragment davon mit einer schwere-Kette-CDR3 aus der Gruppe bestehend aus SEQ ID NO: 4, SEQ ID NO: 10, SEQ ID NO: 22, SEQ ID NO: 28, SEQ ID NO: 40, SEQ ID NO: 46, SEQ ID NO: 58, SEQ ID NO: 64, SEQ ID NO: 76, SEQ ID NO: 82, SEQ ID NO: 94, SEQ ID NO: 100, SEQ ID NO: 112, SEQ ID NO: 118, SEQ ID NO: 130, SEQ ID NO: 136, SEQ ID NO: 148, SEQ ID NO: 166, SEQ ID NO: 184, SEQ ID NO: 202, SEQ ID NO: 220, SEQ ID NO: 238, SEQ ID NO: 256, SEQ ID NO: 274, SEQ ID NO: 292, SEQ ID NO: 310, SEQ ID NO: 328, SEQ ID NO: 346 und SEQ ID NO: 364 handelt.

2. Pharmazeutische Kombination nach Anspruch 1, wobei es sich bei dem EGFR-Inhibitor um einen Antikörper oder ein Fragment davon mit den Sequenzen von MOR10703 gemäß Tabelle 1 (Antikörper A) oder Cetuximab handelt.

3. Pharmazeutische Kombination nach Anspruch 1 oder 2 zur Verwendung als Medizin.

4. Ceritinib zur Verwendung als Medizin, wobei Ceritinib oder ein pharmazeutisch unbedenkliches Salz davon in Kombination mit einen EGFR-Inhibitor oder einem pharmazeutisch unbedenklichen Salz davon verabreicht wird, wobei es sich bei dem EGFR-Inhibitor um Erlotinib, Gefitinib, Lapatinib, Canertinib, Pelitinib, Neratinib, (*R,E*)-*N-(7-*Chlor-1-(1-(4-(dimethylamino)but-2-enoyl)azepan-3-yl)-1H-benzo[d]imidazol-2-yl)-2-methylisonicotin-amid, Panitumumab, Matuzumab, Pertuzumab, Nimotuzumab, Zalutumumab, Icotinib, Afatinib, Cetuximab oder ein pharmazeutisch unbedenkliches Salz davon oder einen isolierten Antikörper oder ein Fragment davon mit einer schwere-Kette-CDR3 aus der Gruppe bestehend aus SEQ ID NO: 4, SEQ ID NO: 10, SEQ ID NO: 22, SEQ ID NO: 28, SEQ ID NO: 40, SEQ ID NO: 46, SEQ ID NO: 58, SEQ ID NO: 64, SEQ ID NO: 76, SEQ ID NO: 82, SEQ ID NO: 94, SEQ ID NO: 100, SEQ ID NO: 112, SEQ ID NO: 118, SEQ ID NO: 130, SEQ ID NO: 136, SEQ ID NO: 148, SEQ ID NO: 166, SEQ ID NO: 184, SEQ ID NO: 202, SEQ ID NO: 220, SEQ ID NO: 238, SEQ ID NO: 256, SEQ ID NO: 274, SEQ ID NO: 292, SEQ ID NO: 310, SEQ ID NO: 328, SEQ ID NO: 346 und SEQ ID NO: 364 handelt.

5. Ceritinib zur Verwendung als Medizin nach Anspruch 4 zur Behandlung von Krebs.

6. Ceritinib zur Verwendung als Medizin nach Anspruch 5, wobei es sich bei dem Krebs um einen nichtkleinzelligen Lungenkrebs handelt.

7. Ceritinib zur Verwendung als Medizin in Kombination nach einem der Ansprüche 4 bis 6, wobei es sich bei dem EGFR-Inhibitor um einen Antikörper oder ein Fragment davon mit den Sequenzen von MOR10703 gemäß Tabelle 1 (Antikörper A), Erlotinib, Gefitinib oder Cetuximab handelt.

8. Pharmazeutische Kombination nach einem der Ansprüche 1 bis 3 oder Ceritinib zur Verwendung als Medizin nach einem der Ansprüche 4 bis 7, wobei es sich bei dem EGFR-Inhibitor um Gefitinib handelt.

9. Pharmazeutische Kombination nach einem der Ansprüche 1 bis 3 oder Ceritinib zur Verwendung als Medizin nach einem der Ansprüche 4 bis 7, wobei es sich bei dem EGFR-Inhibitor um Erlotinib handelt.

10. Pharmazeutische Kombination nach einem der Ansprüche 1 bis 3 oder Ceritinib zur Verwendung als Medizin nach einem der Ansprüche 4 bis 7, wobei es sich bei dem EGFR-Inhibitor um Cetuximab handelt.

11. Pharmazeutische Kombination nach einem der Ansprüche 1 bis 3 oder Ceritinib zur Verwendung als Medizin nach einem der Ansprüche 4 bis 7, wobei es sich bei dem EGFR-Inhibitor um einen isolierten Antikörper oder ein Fragment davon mit einer Schwere-Kette-variable-Region CDR1 gemäß SEQ ID NO: 128; CDR2 gemäß SEQ ID NO: 129; CDR3 gemäß SEQ ID NO: 130 und einer Leichte-Kette-variable-Region CDR1 gemäß SEQ ID NO: 131; CDR2 gemäß SEQ ID NO: 132 und CDR3 gemäß SEQ ID NO: 133 handelt.

12. Pharmazeutische Kombination zur Verwendung als Medizin nach einem der Ansprüche 3 oder 8 bis 11 oder Ceritinib zur Verwendung als Medizin nach einem der Ansprüche 4 bis 11 zur Behandlung von Krebs, wobei der Krebs WT-EGFR umfasst.

13. Pharmazeutische Kombination zur Verwendung als Medizin nach einem der Ansprüche 3 oder 8 bis 11 oder Ceritinib zur Verwendung als Medizin nach einem der Ansprüche 4 bis 11 zur Behandlung von Krebs, wobei der Krebs T790M-EGFR umfasst.

14. Pharmazeutische Kombination zur Verwendung als Medizin nach einem der Ansprüche 3 oder 8 bis 13 oder Ceritinib zur Verwendung als Medizin nach einem der Ansprüche 4 bis 13, wobei Ceritinib und EGFR-Inhibitor an einen ALK-naiven Patienten verabreicht werden.

15. Pharmazeutische Kombination zur Verwendung als Medizin nach einem der Ansprüche 3 oder 8 bis 13 oder Ceritinib zur Verwendung als Medizin nach einem der Ansprüche 4 bis 13, wobei Ceritinib und EGFR-Inhibitor an einen mit einem ALK-Inhibitor vorbehandelten Patienten verabreicht werden.

## Revendications

1. Combinaison pharmaceutique comprenant (i) le céritinib, ou un sel pharmaceutiquement acceptable de celui-ci, et (ii) un inhibiteur d'EGFR, ou un sel pharmaceutiquement acceptable de celui-ci, dans laquelle l'inhibiteur d'EGFR est choisi dans le groupe constitué des erlotinib, géfitinib, lapatinib, canertinib, pélitinib, nératinib, (R,E)-N-(7-chloro-1-(1-(4-(diméthylamino)but-2-énoyl)azépan-3-yl)-1H-benzo[d]imidazol-2-yl)-2-méthylisonicotinamide, panitumumab, matuzumab, pertuzumab, nimotuzumab, zalutumumab, icotinib, afatinib et cétuximab, et un sel pharmaceutiquement acceptable de ceux-ci, ou l'inhibiteur d'EGFR est un anticorps isolé ou un fragment de celui-ci comprenant une CDR3 de chaîne lourde choisie dans le groupe constitué de SEQ ID NO : 4, SEQ ID NO : 10, SEQ ID NO : 22, SEQ ID NO : 28, SEQ ID NO : 40, SEQ ID NO : 46, SEQ ID NO : 58, SEQ ID NO : 64, SEQ ID NO : 76, SEQ ID NO : 82, SEQ ID NO : 94, SEQ ID NO : 100, SEQ ID NO : 112, SEQ ID NO : 118, SEQ ID NO : 130, SEQ ID NO : 136, SEQ ID NO : 148, SEQ ID NO : 166, SEQ ID NO : 184, SEQ ID NO : 202, SEQ ID NO : 220, SEQ ID NO : 238, SEQ ID NO : 256, SEQ ID NO : 274, SEQ ID NO : 292, SEQ ID NO : 310, SEQ ID NO : 328, SEQ ID NO : 346, et SEQ ID NO : 364.

2. Combinaison pharmaceutique selon la revendication 1, dans laquelle l'inhibiteur d'EGFR est un anticorps ou un fragment de celui-ci comprenant les séquences de MOR10703 telles que définies dans le tableau 1 (anticorps A) ou le cétuximab.

3. Combinaison pharmaceutique selon l'une quelconque des revendications 1 et 2, pour utilisation en tant que médicament.

4. Céritinib pour utilisation en tant que médicament, le céritinib, ou un sel pharmaceutiquement acceptable de celui-ci, étant administré en combinaison avec un inhibiteur d'EGFR, ou un sel pharmaceutiquement acceptable de celui-ci, l'inhibiteur d'EGFR étant l'erlotinib, le géfitinib, le lapatinib, le canertinib, le pélitinib, le nératinib, le (R,E)-N-(7-chloro-1-(1-(4-(diméthylamino)but-2-énoyl)azépan-3-yl)-1H-benzo[d]imidazol-2-yl)-2-méthylisonicotinamide, le panitumumab, le matuzumab, le pertuzumab, le nimotuzumab, le zalutumumab, l'icotinib, l'afatinib, le cétuximab, ou un sel pharmaceutiquement acceptable de ceux-ci, ou un anticorps isolé ou un fragment de celui-ci comprenant une CDR3 de chaîne lourde choisie dans le groupe constitué de SEQ ID NO : 4, SEQ ID NO : 10, SEQ ID NO : 22, SEQ ID NO : 28, SEQ ID NO : 40, SEQ ID NO : 46, SEQ ID NO : 58, SEQ ID NO : 64, SEQ ID NO : 76, SEQ ID NO : 82, SEQ ID NO : 94, SEQ ID NO : 100, SEQ ID NO : 112, SEQ ID NO : 118, SEQ ID NO : 130, SEQ ID NO : 136, SEQ ID NO : 148, SEQ ID NO : 166, SEQ ID NO : 184, SEQ ID NO : 202, SEQ ID NO : 220, SEQ ID NO : 238, SEQ ID NO : 256, SEQ ID NO : 274, SEQ ID NO : 292, SEQ ID NO : 310, SEQ ID NO : 328, SEQ ID NO : 346 et SEQ ID NO : 364.

5. Céritinib pour utilisation en tant que médicament selon la revendication 4, pour le traitement du cancer.

6. Céritinib pour utilisation en tant que médicament selon la revendication 5, le cancer étant un cancer du poumon non à petites cellules.

7. Céritinib pour utilisation en tant que médicament en combinaison, selon l'une quelconque des revendications 4 à 6, l'inhibiteur d'EGFR étant un anticorps ou un fragment de celui-ci comprenant les séquences de MOR10703 telles que définies dans le tableau 1 (anticorps A), l'erlotinib, le géfitinib ou le cétuximab.

8. Combinaison pharmaceutique selon l'une quelconque des revendications 1 à 3, ou céritinib pour utilisation en tant que médicament selon l'une quelconque des revendications 4 à 7, l'inhibiteur d'EGFR étant le géfitinib.

9. Combinaison pharmaceutique selon l'une quelconque des revendications 1 à 3, ou céritinib pour utilisation en tant que médicament selon l'une quelconque des revendications 4 à 7, l'inhibiteur d'EGFR étant l'erlotinib.

10. Combinaison pharmaceutique selon l'une quelconque des revendications 1 à 3, ou céritinib pour utilisation en tant que médicament selon l'une quelconque des revendications 4 à 7, l'inhibiteur d'EGFR étant le cétuximab.

11. Combinaison pharmaceutique selon l'une quelconque des revendications 1 à 3, ou céritinib pour utilisation en tant que médicament selon l'une quelconque des revendications 4 à 7, dans lesquels l'inhibiteur d'EGFR est un anticorps isolé ou un fragment de celui-ci qui comprend une région variable de chaîne lourde CDR1 de SEQ ID NO : 128 ; CDR2 de SEQ ID NO : 129 ; CDR3 de SEQ ID NO : 130 ; et une région variable de chaîne légère CDR1 de SEQ ID NO : 131 ; CDR2 de SEQ ID NO : 132 ; et CDR3 de SEQ ID NO : 133.

12. Combinaison pharmaceutique pour utilisation en tant que médicament selon l'une quelconque des revendications 3 ou 8 à 11, ou céritinib pour utilisation en tant que médicament selon l'une quelconque des revendications 4 à 11, pour le traitement du cancer, le cancer comprenant EGFR de type sauvage.

13. Combinaison pharmaceutique pour utilisation en tant que médicament selon l'une quelconque des revendications 3 ou 8 à 11, ou céritinib pour utilisation en tant que médicament selon l'une quelconque des revendications 4 à 11, pour le traitement du cancer, le cancer comprenant EGFR T790M.

14. Combinaison pharmaceutique pour utilisation en tant que médicament selon l'une quelconque des revendications 3 ou 8 à 13, ou céritinib pour utilisation en tant que médicament selon l'une quelconque des revendications 4 à 13, le céritinib et l'inhibiteur d'EGFR étant administrés à un patient naïf pour ALK.

15. Combinaison pharmaceutique pour utilisation en tant que médicament selon l'une quelconque des revendications 3 ou 8 à 13, ou céritinib pour utilisation en tant que médicament selon l'une quelconque des revendications 4 à 13, le céritinib et l'inhibiteur d'EGFR étant administrés à un patient qui a été prétraité avec un inhibiteur d'ALK.


### SEQUENCE LISTING

<110> NOVARTIS AG
<120> COMBINATION OF CERITINIB WITH AN EGFR INHIBITOR
<130> PAT056502-WO-PCT
<140>
   <141>
<150> 62/118,710
   <151> 2015-02-20
<150> 62/068,945
   <151> 2014-10-27
<150> 62/068,175
   <151> 2014-10-24
<150> 62/065,451
   <151> 2014-10-17
<160> 379
<170> PatentIn version 3.5
<210> 1
   <211> 1342
   <212> PRT
   <213> Homo sapiens
<400> 1
<210> 2
   <211> 5
   <212> PRT
   <213> Homo sapiens
<400> 2
   Ser Tyr Ala Met Ser
   1 5
<210> 3
   <211> 16
   <212> PRT
   <213> Homo sapiens
<400> 3
   Val Thr Gly Ala Val Gly Arg Thr Tyr Tyr Pro Asp Ser Val Lys Gly
   1 5 10 15
<210> 4
   <211> 8
   <212> PRT
   <213> Homo sapiens
<400> 4
   Trp Gly Asp Glu Gly Phe Asp Ile
   1 5
<210> 5
   <211> 11
   <212> PRT
   <213> Homo sapiens
<400> 5
   Arg Ala Ser Gln Gly Ile Ser Asn Trp Leu Ala
   1 5 10
<210> 6
   <211> 7
   <212> PRT
   <213> Homo sapiens
<400> 6
   Gly Ala Ser Ser Leu Gln Ser
   1 5
<210> 7
   <211> 9
   <212> PRT
   <213> Homo sapiens
<400> 7
   Gln Gln Tyr Ser Ser Phe Pro Thr Thr
   1 5
<210> 8
   <211> 7
   <212> PRT
   <213> Homo sapiens
<400> 8
   Gly Phe Thr Phe Ser Ser Tyr
   1 5
<210> 9
   <211> 5
   <212> PRT
   <213> Homo sapiens
<400> 9
   Gly Ala Val Gly Arg
   1 5
<210> 10
   <211> 8
   <212> PRT
   <213> Homo sapiens
<400> 10
   Trp Gly Asp Glu Gly Phe Asp Ile
   1 5
<210> 11
   <211> 7
   <212> PRT
   <213> Homo sapiens
<400> 11
   Ser Gln Gly Ile Ser Asn Trp
   1 5
<210> 12
   <211> 3
   <212> PRT
   <213> Homo sapiens
<400> 12
   Gly Ala Ser
   1
<210> 13
   <211> 6
   <212> PRT
   <213> Homo sapiens
<400> 13
   Tyr Ser Ser Phe Pro Thr
   1 5
<210> 14
   <211> 107
   <212> PRT
   <213> Homo sapiens
<400> 14
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Ser Asn Trp
   20 25 30
   Leu Ala Trp Tyr Gln Gln Lys Pro Gly Lys Ala Pro Lys Leu Leu Ile
   35 40 45
   Tyr Gly Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Val Tyr Tyr Cys Gln Gln Tyr Ser Ser Phe Pro Thr
   85 90 95
   Thr Phe Gly Gln Gly Thr Lys Val Glu Ile Lys
   100 105
<210> 15
   <211> 116
   <212> PRT
   <213> Homo sapiens
<400> 15
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Thr Gly Ala Val Gly Arg Thr Tyr Tyr Pro Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Trp Gly Asp Glu Gly Phe Asp Ile Trp Gly Gln Gly Thr Leu Val
               100                 105                 110
   Thr Val Ser Ser
           115
<210> 16
   <211> 321
   <212> DNA
   <213> Homo sapiens
<400> 16
   gatatccaga tgacccagag cccgtctagc ctgagcgcga gcgtgggtga tcgtgtgacc      60
   attacctgca gagcgagcca gggtatttct aattggctgg cttggtacca gcagaaacca     120
   ggtaaagcac cgaaactatt aatttatggt gcttcttctt tgcaaagcgg ggtcccgtcc     180
   cgttttagcg gctctggatc cggcactgat tttaccctga ccattagcag cctgcaacct     240
   gaagactttg cggtttatta ttgccagcag tattcttctt ttcctactac ctttggccag     300
   ggtacgaaag ttgaaattaa a                                               321
<210> 17
   <211> 348
   <212> DNA
   <213> Homo sapiens
<400> 17
   caggtgcaat tggtggaaag cggcggcggc ctggtgcaac cgggcggcag cctgcgtctg        60
   agctgcgcgg cctccggatt tacctttagc agctatgcga tgagctgggt gcgccaagcc       120
   cctgggaagg gtctcgagtg ggtgagcgtt actggtgctg ttggtcgtac ttattatcct       180
   gattctgtta agggtcgttt taccatttca cgtgataatt cgaaaaacac cctgtatctg       240
   caaatgaaca gcctgcgtgc ggaagatacg gccgtgtatt attgcgcgcg ttggggtgat       300
   gagggttttg atatttgggg ccaaggcacc ctggtgacgg ttagctca                    348
<210> 18
   <211> 214
   <212> PRT
   <213> Homo sapiens
<400> 18
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 19
   <211> 446
   <212> PRT
   <213> Homo sapiens
<400> 19
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Thr Gly Ala Val Gly Arg Thr Tyr Tyr Pro Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
   85 90 95
   Arg Trp Gly Asp Glu Gly Phe Asp Ile Trp Gly Gln Gly Thr Leu Val
   100 105 110
   Thr Val Ser Ser Ala Ser Thr Lys Gly Pro Ser Val Phe Pro Leu Ala
   115 120 125
   Pro Ser Ser Lys Ser Thr Ser Gly Gly Thr Ala Ala Leu Gly Cys Leu
   130 135 140
   Val Lys Asp Tyr Phe Pro Glu Pro Val Thr Val Ser Trp Asn Ser Gly
   145 150 155 160
   Ala Leu Thr Ser Gly Val His Thr Phe Pro Ala Val Leu Gln Ser Ser
   165 170 175
   Gly Leu Tyr Ser Leu Ser Ser Val Val Thr Val Pro Ser Ser Ser Leu
   180 185 190
   Gly Thr Gln Thr Tyr Ile Cys Asn Val Asn His Lys Pro Ser Asn Thr
   195 200 205
   Lys Val Asp Lys Arg Val Glu Pro Lys Ser Cys Asp Lys Thr His Thr
   210 215 220
   Cys Pro Pro Cys Pro Ala Pro Glu Leu Leu Gly Gly Pro Ser Val Phe
   225 230 235 240
   Leu Phe Pro Pro Lys Pro Lys Asp Thr Leu Met Ile Ser Arg Thr Pro
   245 250 255
   Glu Val Thr Cys Val Val Val Asp Val Ser His Glu Asp Pro Glu Val
   260 265 270
   Lys Phe Asn Trp Tyr Val Asp Gly Val Glu Val His Asn Ala Lys Thr
   275 280 285
   Lys Pro Arg Glu Glu Gln Tyr Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Ala Leu Pro Ala Pro Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Arg Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Gln Gly Asn Val Phe Ser Cys Ser Val Met His Glu Ala Leu His
               420                 425                 430
   Asn His Tyr Thr Gln Lys Ser Leu Ser Leu Ser Pro Gly Lys
           435                 440                 445
<210> 20
   <211> 5
   <212> PRT
   <213> Homo sapiens
<400> 20
   Ser Tyr Ala Met Ser
   1               5
<210> 21
   <211> 17
   <212> PRT
   <213> Homo sapiens
<400> 21
   Val Ile Ser Ala Trp Gly His Val Lys Tyr Tyr Ala Asp Ser Val Lys
   1               5                   10                  15
   Gly
<210> 22
   <211> 8
   <212> PRT
   <213> Homo sapiens
<400> 22
   Trp Gly Asp Glu Gly Phe Asp Ile
   1 5
<210> 23
   <211> 11
   <212> PRT
   <213> Homo sapiens
<400> 23
   Arg Ala Ser Gln Gly Ile Ser Asn Trp Leu Ala
   1 5 10
<210> 24
   <211> 7
   <212> PRT
   <213> Homo sapiens
<400> 24
   Gly Ala Ser Ser Leu Gln Ser
   1 5
<210> 25
   <211> 9
   <212> PRT
   <213> Homo sapiens
<400> 25
   Gln Gln Tyr Ser Ser Phe Pro Thr Thr
   1 5
<210> 26
   <211> 7
   <212> PRT
   <213> Homo sapiens
<400> 26
   Gly Phe Thr Phe Ser Ser Tyr
   1 5
<210> 27
   <211> 6
   <212> PRT
   <213> Homo sapiens
<400> 27
   Ser Ala Trp Gly His Val
   1 5
<210> 28
   <211> 8
   <212> PRT
   <213> Homo sapiens
<400> 28
   Trp Gly Asp Glu Gly Phe Asp Ile
   1 5
<210> 29
   <211> 7
   <212> PRT
   <213> Homo sapiens
<400> 29
   Ser Gln Gly Ile Ser Asn Trp
   1 5
<210> 30
   <211> 3
   <212> PRT
   <213> Homo sapiens
<400> 30
   Gly Ala Ser
   1
<210> 31
   <211> 6
   <212> PRT
   <213> Homo sapiens
<400> 31
   Tyr Ser Ser Phe Pro Thr
   1 5
<210> 32
   <211> 107
   <212> PRT
   <213> Homo sapiens
<400> 32
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 33
   <211> 117
   <212> PRT
   <213> Homo sapiens
<400> 33
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Ile Ser Ala Trp Gly His Val Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 34
   <211> 321
   <212> DNA
   <213> Homo sapiens
<400> 34
   gatatccaga tgacccagag cccgtctagc ctgagcgcga gcgtgggtga tcgtgtgacc       60
   attacctgca gagcgagcca gggtatttct aattggctgg cttggtacca gcagaaacca      120
   ggtaaagcac cgaaactatt aatttatggt gcttcttctt tgcaaagcgg ggtcccgtcc      180
   cgttttagcg gctctggatc cggcactgat tttaccctga ccattagcag cctgcaacct      240
   gaagactttg cggtttatta ttgccagcag tattcttctt ttcctactac ctttggccag      300
   ggtacgaaag ttgaaattaa a                                                321
<210> 35
   <211> 351
   <212> DNA
   <213> Homo sapiens
<400> 35
   caggtgcaat tggtggaaag cggcggcggc ctggtgcaac cgggcggcag cctgcgtctg        60
   agctgcgcgg cctccggatt tacctttagc agctatgcga tgagctgggt gcgccaagcc       120
   cctgggaagg gtctcgagtg ggtgagcgtt atttctgctt ggggtcatgt taagtattat       180
   gctgattctg ttaagggtcg ttttaccatt tcacgtgata attcgaaaaa caccctgtat       240
   ctgcaaatga acagcctgcg tgcggaagat acggccgtgt attattgcgc gcgttggggt       300
   gatgagggtt ttgatatttg gggccaaggc accctggtga cggttagctc a               351
<210> 36
   <211> 214
   <212> PRT
   <213> Homo sapiens
<400> 36
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 37
   <211> 447
   <212> PRT
   <213> Homo sapiens
<400> 37
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Ile Ser Ala Trp Gly His Val Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Arg Val Glu Pro Lys Ser Cys Asp Lys Thr His
   210 215 220
   Thr Cys Pro Pro Cys Pro Ala Pro Glu Leu Leu Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 38
   <211> 5
   <212> PRT
   <213> Homo sapiens
<400> 38
   Ser Tyr Ala Met Ser
   1 5
<210> 39
   <211> 17
   <212> PRT
   <213> Homo sapiens
<400> 39
   Ala Ile Asn Ser Gln Gly Lys Ser Thr Tyr Tyr Ala Asp Ser Val Lys
   1 5 10 15
   Gly
<210> 40
   <211> 8
   <212> PRT
   <213> Homo sapiens
<400> 40
   Trp Gly Asp Glu Gly Phe Asp Ile
   1 5
<210> 41
   <211> 11
   <212> PRT
   <213> Homo sapiens
<400> 41
   Arg Ala Ser Gln Gly Ile Ser Asn Trp Leu Ala
   1 5 10
<210> 42
   <211> 7
   <212> PRT
   <213> Homo sapiens
<400> 42
   Gly Ala Ser Ser Leu Gln Ser
   1 5
<210> 43
   <211> 9
   <212> PRT
   <213> Homo sapiens
<400> 43
   Gln Gln Tyr Ser Ser Phe Pro Thr Thr
   1               5
<210> 44
   <211> 7
   <212> PRT
   <213> Homo sapiens
<400> 44
   Gly Phe Thr Phe Ser Ser Tyr
   1               5
<210> 45
   <211> 6
   <212> PRT
   <213> Homo sapiens
<400> 45
   Asn Ser Gln Gly Lys Ser
   1               5
<210> 46
   <211> 8
   <212> PRT
   <213> Homo sapiens
<400> 46
   Trp Gly Asp Glu Gly Phe Asp Ile
   1               5
<210> 47
   <211> 7
   <212> PRT
   <213> Homo sapiens
<400> 47
   Ser Gln Gly Ile Ser Asn Trp
   1               5
<210> 48
   <211> 3
   <212> PRT
   <213> Homo sapiens
<400> 48
   Gly Ala Ser
   1
<210> 49
   <211> 6
   <212> PRT
   <213> Homo sapiens
<400> 49
   Tyr Ser Ser Phe Pro Thr
   1               5
<210> 50
   <211> 107
   <212> PRT
   <213> Homo sapiens
<400> 50
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 51
   <211> 117
   <212> PRT
   <213> Homo sapiens
<400> 51
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Asn Ser Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 52
   <211> 321
   <212> DNA
   <213> Homo sapiens
<400> 52
   gatatccaga tgacccagag cccgtctagc ctgagcgcga gcgtgggtga tcgtgtgacc      60
   attacctgca gagcgagcca gggtatttct aattggctgg cttggtacca gcagaaacca     120
   ggtaaagcac cgaaactatt aatttatggt gcttcttctt tgcaaagcgg ggtcccgtcc     180
   cgttttagcg gctctggatc cggcactgat tttaccctga ccattagcag cctgcaacct     240
   gaagactttg cggtttatta ttgccagcag tattcttctt ttcctactac ctttggccag     300
   ggtacgaaag ttgaaattaa a                                               321
<210> 53
   <211> 351
   <212> DNA
   <213> Homo sapiens
<400> 53
   caggtgcaat tggtggaaag cggcggcggc ctggtgcaac cgggcggcag cctgcgtctg 60
   agctgcgcgg cctccggatt tacctttagc agctatgcga tgagctgggt gcgccaagcc 120
   cctgggaagg gtctcgagtg ggtgagcgct attaattctc agggtaagtc tacttattat 180
   gctgattctg ttaagggtcg ttttaccatt tcacgtgata attcgaaaaa caccctgtat 240
   ctgcaaatga acagcctgcg tgcggaagat acggccgtgt attattgcgc gcgttggggt 300
   gatgagggtt ttgatatttg gggccaaggc accctggtga cggttagctc a 351
<210> 54
   <211> 214
   <212> PRT
   <213> Homo sapiens
<400> 54
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 55
   <211> 447
   <212> PRT
   <213> Homo sapiens
<400> 55
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
   35 40 45
   Ser Ala Ile Asn Ser Gln Gly Lys Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Trp Gly Asp Glu Gly Phe Asp Ile Trp Gly Gln Gly Thr Leu
   100 105 110
   Val Thr Val Ser Ser Ala Ser Thr Lys Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145 150 155 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Arg Val Glu Pro Lys Ser Cys Asp Lys Thr His
   210 215 220
   Thr Cys Pro Pro Cys Pro Ala Pro Glu Leu Leu Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 56
   <211> 5
   <212> PRT
   <213> Homo sapiens
<400> 56
   Ser Tyr Ala Met Ser
   1 5
<210> 57
   <211> 17
   <212> PRT
   <213> Homo sapiens
<400> 57
   Val Ile Asn Pro Ser Gly Asn Phe Thr Asn Tyr Ala Asp Ser Val Lys
   1 5 10 15
   Gly
<210> 58
   <211> 8
   <212> PRT
   <213> Homo sapiens
<400> 58
   Trp Gly Asp Glu Gly Phe Asp Ile
   1 5
<210> 59
   <211> 11
   <212> PRT
   <213> Homo sapiens
<400> 59
   Arg Ala Ser Gln Gly Ile Ser Asn Trp Leu Ala
   1 5 10
<210> 60
   <211> 7
   <212> PRT
   <213> Homo sapiens
<400> 60
   Gly Ala Ser Ser Leu Gln Ser
   1 5
<210> 61
   <211> 9
   <212> PRT
   <213> Homo sapiens
<400> 61
   Gln Gln Tyr Ser Ser Phe Pro Thr Thr
   1 5
<210> 62
   <211> 7
   <212> PRT
   <213> Homo sapiens
<400> 62
   Gly Phe Thr Phe Ser Ser Tyr
   1 5
<210> 63
   <211> 6
   <212> PRT
   <213> Homo sapiens
<400> 63
   Asn Pro Ser Gly Asn Phe
   1 5
<210> 64
   <211> 8
   <212> PRT
   <213> Homo sapiens
<400> 64
   Trp Gly Asp Glu Gly Phe Asp Ile
   1 5
<210> 65
   <211> 7
   <212> PRT
   <213> Homo sapiens
<400> 65
   Ser Gln Gly Ile Ser Asn Trp
   1 5
<210> 66
   <211> 3
   <212> PRT
   <213> Homo sapiens
<400> 66
   Gly Ala Ser
   1
<210> 67
   <211> 6
   <212> PRT
   <213> Homo sapiens
<400> 67
   Tyr Ser Ser Phe Pro Thr
   1 5
<210> 68
   <211> 107
   <212> PRT
   <213> Homo sapiens
<400> 68
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 69
   <211> 117
   <212> PRT
   <213> Homo sapiens
<400> 69
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Ile Asn Pro Ser Gly Asn Phe Thr Asn Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 70
   <211> 321
   <212> DNA
   <213> Homo sapiens
<400> 70
   gatatccaga tgacccagag cccgtctagc ctgagcgcga gcgtgggtga tcgtgtgacc       60
   attacctgca gagcgagcca gggtatttct aattggctgg cttggtacca gcagaaacca      120
   ggtaaagcac cgaaactatt aatttatggt gcttcttctt tgcaaagcgg ggtcccgtcc      180
   cgttttagcg gctctggatc cggcactgat tttaccctga ccattagcag cctgcaacct      240
   gaagactttg cggtttatta ttgccagcag tattcttctt ttcctactac ctttggccag      300
   ggtacgaaag ttgaaattaa a                                                321
<210> 71
   <211> 351
   <212> DNA
   <213> Homo sapiens
<400> 71
   caggtgcaat tggtggaaag cggcggcggc ctggtgcaac cgggcggcag cctgcgtctg       60
   agctgcgcgg cctccggatt tacctttagc agctatgcga tgagctgggt gcgccaagcc      120
   cctgggaagg gtctcgagtg ggtgagcgtt attaatcctt ctggtaattt tactaattat      180
   gctgattctg ttaagggtcg ttttaccatt tcacgtgata attcgaaaaa caccctgtat      240
   ctgcaaatga acagcctgcg tgcggaagat acggccgtgt attattgcgc gcgttggggt      300
   gatgagggtt ttgatatttg gggccaaggc accctggtga cggttagctc a               351
<210> 72
   <211> 214
   <212> PRT
   <213> Homo sapiens
<400> 72
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 73
   <211> 447
   <212> PRT
   <213> Homo sapiens
<400> 73
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Val Ile Asn Pro Ser Gly Asn Phe Thr Asn Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Trp Gly Asp Glu Gly Phe Asp Ile Trp Gly Gln Gly Thr Leu
   100 105 110
   Val Thr Val Ser Ser Ala Ser Thr Lys Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 74
   <211> 5
   <212> PRT
   <213> Homo sapiens
<400> 74
   Ser Tyr Ala Met Ser
   1 5
<210> 75
   <211> 16
   <212> PRT
   <213> Homo sapiens
<400> 75
   Asn Thr Ser Pro Ile Gly Tyr Thr Tyr Tyr Ala Gly Ser Val Lys Gly
   1 5 10 15
<210> 76
   <211> 8
   <212> PRT
   <213> Homo sapiens
<400> 76
   Trp Gly Asp Glu Gly Phe Asp Ile
   1 5
<210> 77
   <211> 11
   <212> PRT
   <213> Homo sapiens
<400> 77
   Arg Ala Ser Gln Gly Ile Ser Asn Trp Leu Ala
   1 5 10
<210> 78
   <211> 7
   <212> PRT
   <213> Homo sapiens
<400> 78
   Gly Ala Ser Ser Leu Gln Ser
   1 5
<210> 79
   <211> 9
   <212> PRT
   <213> Homo sapiens
<400> 79
   Gln Gln Tyr Ser Ser Phe Pro Thr Thr
   1 5
<210> 80
   <211> 7
   <212> PRT
   <213> Homo sapiens
<400> 80
   Gly Phe Thr Phe Ser Ser Tyr
   1 5
<210> 81
   <211> 5
   <212> PRT
   <213> Homo sapiens
<400> 81
   Ser Pro Ile Gly Tyr
   1 5
<210> 82
   <211> 8
   <212> PRT
   <213> Homo sapiens
<400> 82
   Trp Gly Asp Glu Gly Phe Asp Ile
   1 5
<210> 83
   <211> 7
   <212> PRT
   <213> Homo sapiens
<400> 83
   Ser Gln Gly Ile Ser Asn Trp
   1 5
<210> 84
   <211> 3
   <212> PRT
   <213> Homo sapiens
<400> 84
   Gly Ala Ser
   1
<210> 85
   <211> 6
   <212> PRT
   <213> Homo sapiens
<400> 85
   Tyr Ser Ser Phe Pro Thr
   1 5
<210> 86
   <211> 107
   <212> PRT
   <213> Homo sapiens
<400> 86
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Ser Asn Trp
   20 25 30
   Leu Ala Trp Tyr Gln Gln Lys Pro Gly Lys Ala Pro Lys Leu Leu Ile
   35 40 45
   Tyr Gly Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Val Tyr Tyr Cys Gln Gln Tyr Ser Ser Phe Pro Thr
   85 90 95
   Thr Phe Gly Gln Gly Thr Lys Val Glu Ile Lys
   100 105
<210> 87
   <211> 116
   <212> PRT
   <213> Homo sapiens
<400> 87
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Asn Thr Ser Pro Ile Gly Tyr Thr Tyr Tyr Ala Gly Ser Val Lys
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Trp Gly Asp Glu Gly Phe Asp Ile Trp Gly Gln Gly Thr Leu Val
   100 105 110
   Thr Val Ser Ser
   115
<210> 88
   <211> 321
   <212> DNA
   <213> Homo sapiens
<400> 88
   gatatccaga tgacccagag cccgtctagc ctgagcgcga gcgtgggtga tcgtgtgacc 60
   attacctgca gagcgagcca gggtatttct aattggctgg cttggtacca gcagaaacca 120
   ggtaaagcac cgaaactatt aatttatggt gcttcttctt tgcaaagcgg ggtcccgtcc 180
   cgttttagcg gctctggatc cggcactgat tttaccctga ccattagcag cctgcaacct 240
   gaagactttg cggtttatta ttgccagcag tattcttctt ttcctactac ctttggccag 300
   ggtacgaaag ttgaaattaa a 321
<210> 89
   <211> 348
   <212> DNA
   <213> Homo sapiens
<400> 89
   caggtgcaat tggtggaaag cggcggcggc ctggtgcaac cgggcggcag cctgcgtctg 60
   agctgcgcgg cctccggatt tacctttagc agctatgcga tgagctgggt gcgccaagcc 120
   cctgggaagg gtctcgagtg ggtgagcaat acttctccta ttggttatac ttattatgct 180
   ggttctgtta agggtcgttt taccatttca cgtgataatt cgaaaaacac cctgtatctg 240
   caaatgaaca gcctgcgtgc ggaagatacg gccgtgtatt attgcgcgcg ttggggtgat 300
   gagggttttg atatttgggg ccaaggcacc ctggtgacgg ttagctca 348
<210> 90
   <211> 214
   <212> PRT
   <213> Homo sapiens
<400> 90
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Ser Asn Trp
   20 25 30
   Leu Ala Trp Tyr Gln Gln Lys Pro Gly Lys Ala Pro Lys Leu Leu Ile
   35 40 45
   Tyr Gly Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Val Tyr Tyr Cys Gln Gln Tyr Ser Ser Phe Pro Thr
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Ala
   210
<210> 91
   <211> 218
   <212> PRT
   <213> Homo sapiens
<400> 91
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Asn Thr Ser Pro Ile Gly Tyr Thr Tyr Tyr Ala Gly Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Trp Gly Asp Glu Gly Phe Asp Ile Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser
       210                 215
<210> 92
   <211> 5
   <212> PRT
   <213> Homo sapiens
<400> 92
   Ser Tyr Ala Met Ser
   1               5
<210> 93
   <211> 17
   <212> PRT
   <213> Homo sapiens
<400> 93
   Val Thr Gly Ala Val Gly Arg Ser Thr Tyr Tyr Pro Asp Ser Val Lys
   1               5                   10                  15
   Gly
<210> 94
   <211> 8
   <212> PRT
   <213> Homo sapiens
<400> 94
   Trp Gly Asp Glu Gly Phe Asp Ile
   1               5
<210> 95
   <211> 11
   <212> PRT
   <213> Homo sapiens
<400> 95
   Arg Ala Ser Gln Gly Ile Ser Asn Trp Leu Ala
   1               5                   10
<210> 96
   <211> 7
   <212> PRT
   <213> Homo sapiens
<400> 96
   Gly Ala Ser Ser Leu Gln Ser
   1               5
<210> 97
   <211> 9
   <212> PRT
   <213> Homo sapiens
<400> 97
   Gln Gln Tyr Ser Ser Phe Pro Thr Thr
   1               5
<210> 98
   <211> 7
   <212> PRT
   <213> Homo sapiens
<400> 98
   Gly Phe Thr Phe Ser Ser Tyr
   1 5
<210> 99
   <211> 6
   <212> PRT
   <213> Homo sapiens
<400> 99
   Gly Ala Val Gly Arg Ser
   1 5
<210> 100
   <211> 8
   <212> PRT
   <213> Homo sapiens
<400> 100
   Trp Gly Asp Glu Gly Phe Asp Ile
   1 5
<210> 101
   <211> 7
   <212> PRT
   <213> Homo sapiens
<400> 101
   Ser Gln Gly Ile Ser Asn Trp
   1 5
<210> 102
   <211> 3
   <212> PRT
   <213> Homo sapiens
<400> 102
   Gly Ala Ser
   1
<210> 103
   <211> 6
   <212> PRT
   <213> Homo sapiens
<400> 103
   Tyr Ser Ser Phe Pro Thr
   1 5
<210> 104
   <211> 107
   <212> PRT
   <213> Homo sapiens
<400> 104
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 105
   <211> 117
   <212> PRT
   <213> Homo sapiens
<400> 105
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Thr Gly Ala Val Gly Arg Ser Thr Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 106
   <211> 321
   <212> DNA
   <213> Homo sapiens
<400> 106
   gatatccaga tgacccagag ccccagcagc ctgagcgcca gcgtgggcga cagagtgacc       60
   atcacctgtc gggccagcca gggcatcagc aactggctgg cctggtatca gcagaagccc      120
   ggcaaggccc ccaagctgct gatctacggc gccagctccc tgcagagcgg cgtgccaagc      180
   agattcagcg gcagcggctc cggcaccgac ttcaccctga ccatcagcag cctgcagccc      240
   gaggacttcg ccacctacta ctgccagcag tacagcagct tccccaccac cttcggccag      300
   ggcaccaagg tggaaatcaa g                                                321
<210> 107
   <211> 351
   <212> DNA
   <213> Homo sapiens
<400> 107
   gaggtgcaat tgctggaaag cggcggaggc ctggtgcagc ctggcggcag cctgagactg        60
   tcttgcgccg ccagcggctt caccttcagc agctacgcca tgagctgggt ccgccaggcc       120
   cctggcaagg gactggaatg ggtgtccgtg acaggcgccg tgggcagaag cacctactac       180
   cccgacagcg tgaagggccg gttcaccatc agccgggaca acagcaagaa caccctgtac       240
   ctgcagatga acagcctgcg ggccgaggac accgccgtgt actactgtgc cagatggggc       300
   gacgagggct tcgacatctg gggccagggc accctggtca ccgtcagctc a                351
<210> 108
   <211> 214
   <212> PRT
   <213> Homo sapiens
<400> 108
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 109
   <211> 447
   <212> PRT
   <213> Homo sapiens
<400> 109
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Thr Gly Ala Val Gly Arg Ser Thr Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 110
   <211> 5
   <212> PRT
   <213> Homo sapiens
<400> 110
   Ser Tyr Ala Met Ser
   1 5
<210> 111
   <211> 17
   <212> PRT
   <213> Homo sapiens
<400> 111
   Val Ile Ser Ala Trp Gly His Val Lys Tyr Tyr Ala Asp Ser Val Lys
   1 5 10 15
   Gly
<210> 112
   <211> 8
   <212> PRT
   <213> Homo sapiens
<400> 112
   Trp Gly Asp Glu Gly Phe Asp Ile
   1 5
<210> 113
   <211> 11
   <212> PRT
   <213> Homo sapiens
<400> 113
   Arg Ala Ser Gln Gly Ile Ser Asn Trp Leu Ala
   1 5 10
<210> 114
   <211> 7
   <212> PRT
   <213> Homo sapiens
<400> 114
   Gly Ala Ser Ser Leu Gln Ser
   1 5
<210> 115
   <211> 9
   <212> PRT
   <213> Homo sapiens
<400> 115
   Gln Gln Tyr Ser Ser Phe Pro Thr Thr
   1 5
<210> 116
   <211> 7
   <212> PRT
   <213> Homo sapiens
<400> 116
   Gly Phe Thr Phe Ser Ser Tyr
   1 5
<210> 117
   <211> 6
   <212> PRT
   <213> Homo sapiens
<400> 117
   Ser Ala Trp Gly His Val
   1 5
<210> 118
   <211> 8
   <212> PRT
   <213> Homo sapiens
<400> 118
   Trp Gly Asp Glu Gly Phe Asp Ile
   1 5
<210> 119
   <211> 7
   <212> PRT
   <213> Homo sapiens
<400> 119
   Ser Gln Gly Ile Ser Asn Trp
   1 5
<210> 120
   <211> 3
   <212> PRT
   <213> Homo sapiens
<400> 120
   Gly Ala Ser
   1
<210> 121
   <211> 6
   <212> PRT
   <213> Homo sapiens
<400> 121
   Tyr Ser Ser Phe Pro Thr
   1 5
<210> 122
   <211> 107
   <212> PRT
   <213> Homo sapiens
<400> 122
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 123
   <211> 117
   <212> PRT
   <213> Homo sapiens
<400> 123
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Ile Ser Ala Trp Gly His Val Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 124
   <211> 321
   <212> DNA
   <213> Homo sapiens
<400> 124
   gatatccaga tgacccagag ccccagcagc ctgagcgcca gcgtgggcga cagagtgacc      60
   atcacctgtc gggccagcca gggcatcagc aactggctgg cctggtatca gcagaagccc     120
   ggcaaggccc ccaagctgct gatctacggc gccagctccc tgcagagcgg cgtgccaagc     180
   agattcagcg gcagcggctc cggcaccgac ttcaccctga ccatcagcag cctgcagccc     240
   gaggacttcg ccacctacta ctgccagcag tacagcagct tccccaccac cttcggccag     300
   ggcaccaagg tggaaatcaa g                                               321
<210> 125
   <211> 351
   <212> DNA
   <213> Homo sapiens
<400> 125
   gaggtgcaat tgctggaaag cggcggaggc ctggtgcagc ctggcggcag cctgagactg        60
   tcttgcgccg ccagcggctt caccttcagc agctacgcca tgagctgggt ccgccaggcc       120
   cctggcaagg gactggaatg ggtgtccgtg atcagcgcct ggggccacgt gaagtactac       180
   gccgacagcg tgaagggccg gttcaccatc agccgggaca acagcaagaa caccctgtac       240
   ctgcagatga acagcctgcg ggccgaggac accgccgtgt actactgtgc cagatggggc       300
   gacgagggct tcgacatctg gggccagggc accctggtca ccgtcagctc a                351
<210> 126
   <211> 214
   <212> PRT
   <213> Homo sapiens
<400> 126
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 127
   <211> 447
   <212> PRT
   <213> Homo sapiens
<400> 127
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Ile Ser Ala Trp Gly His Val Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
   210 215 220
   Thr Cys Pro Pro Cys Pro Ala Pro Glu Leu Leu Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 128
   <211> 5
   <212> PRT
   <213> Homo sapiens
<400> 128
   Ser Tyr Ala Met Ser
   1               5
<210> 129
   <211> 17
   <212> PRT
   <213> Homo sapiens
<400> 129
   Ala Ile Asn Ser Gln Gly Lys Ser Thr Tyr Tyr Ala Asp Ser Val Lys
   1               5                   10                  15
   Gly
<210> 130
   <211> 8
   <212> PRT
   <213> Homo sapiens
<400> 130
   Trp Gly Asp Glu Gly Phe Asp Ile
   1               5
<210> 131
   <211> 11
   <212> PRT
   <213> Homo sapiens
<400> 131
   Arg Ala Ser Gln Gly Ile Ser Asn Trp Leu Ala
   1               5                   10
<210> 132
   <211> 7
   <212> PRT
   <213> Homo sapiens
<400> 132
   Gly Ala Ser Ser Leu Gln Ser
   1               5
<210> 133
   <211> 9
   <212> PRT
   <213> Homo sapiens
<400> 133
   Gln Gln Tyr Ser Ser Phe Pro Thr Thr
   1 5
<210> 134
   <211> 7
   <212> PRT
   <213> Homo sapiens
<400> 134
   Gly Phe Thr Phe Ser Ser Tyr
   1 5
<210> 135
   <211> 6
   <212> PRT
   <213> Homo sapiens
<400> 135
   Asn Ser Gln Gly Lys Ser
   1 5
<210> 136
   <211> 8
   <212> PRT
   <213> Homo sapiens
<400> 136
   Trp Gly Asp Glu Gly Phe Asp Ile
   1 5
<210> 137
   <211> 7
   <212> PRT
   <213> Homo sapiens
<400> 137
   Ser Gln Gly Ile Ser Asn Trp
   1 5
<210> 138
   <211> 3
   <212> PRT
   <213> Homo sapiens
<400> 138
   Gly Ala Ser
   1
<210> 139
   <211> 6
   <212> PRT
   <213> Homo sapiens
<400> 139
   Tyr Ser Ser Phe Pro Thr
   1               5
<210> 140
   <211> 107
   <212> PRT
   <213> Homo sapiens
<400> 140
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 141
   <211> 117
   <212> PRT
   <213> Homo sapiens
<400> 141
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Asn Ser Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 142
   <211> 321
   <212> DNA
   <213> Homo sapiens
<400> 142
   gatatccaga tgacccagag ccccagcagc ctgagcgcca gcgtgggcga cagagtgacc      60
   atcacctgtc gggccagcca gggcatcagc aactggctgg cctggtatca gcagaagccc     120
   ggcaaggccc ccaagctgct gatctacggc gccagctccc tgcagagcgg cgtgccaagc     180
   agattcagcg gcagcggctc cggcaccgac ttcaccctga ccatcagcag cctgcagccc     240
   gaggacttcg ccacctacta ctgccagcag tacagcagct tccccaccac cttcggccag     300
   ggcaccaagg tggaaatcaa g                                                321
<210> 143
   <211> 351
   <212> DNA
   <213> Homo sapiens
<400> 143
   gaggtgcaat tgctggaaag cggcggaggc ctggtgcagc ctggcggcag cctgagactg 60
   tcttgcgccg ccagcggctt caccttcagc agctacgcca tgagctgggt ccgccaggcc 120
   cctggcaagg gactggaatg ggtgtccgcc atcaacagcc agggcaagag cacctactac 180
   gccgacagcg tgaagggccg gttcaccatc agccgggaca acagcaagaa caccctgtac 240
   ctgcagatga acagcctgcg ggccgaggac accgccgtgt actactgtgc cagatggggc 300
   gacgagggct tcgacatctg gggccagggc accctggtca ccgtcagctc a 351
<210> 144
   <211> 214
   <212> PRT
   <213> Homo sapiens
<400> 144
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Ser Phe Pro Thr
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 145
   <211> 447
   <212> PRT
   <213> Homo sapiens
<400> 145
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Asn Ser Gln Gly Lys Ser Thr Tyr Tyr Ala Asp Ser Val
   50 55 60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
           435                 440                 445
<210> 146
   <211> 5
   <212> PRT
   <213> Homo sapiens
<400> 146
   Ser Tyr Ala Met Ser
   1               5
<210> 147
   <211> 17
   <212> PRT
   <213> Homo sapiens
<400> 147
   Ala Ile Ser Ser Gln Gly Lys Ser Thr Tyr Tyr Ala Asp Ser Val Lys
   1               5                   10                  15
   Gly
<210> 148
   <211> 8
   <212> PRT
   <213> Homo sapiens
<400> 148
   Trp Gly Asp Glu Gly Phe Asp Ile
   1 5
<210> 149
   <211> 11
   <212> PRT
   <213> Homo sapiens
<400> 149
   Arg Ala Ser Gln Gly Ile Ser Asn Trp Leu Ala
   1 5 10
<210> 150
   <211> 7
   <212> PRT
   <213> Homo sapiens
<400> 150
   Gly Ala Ser Ser Leu Gln Ser
   1 5
<210> 151
   <211> 9
   <212> PRT
   <213> Homo sapiens
<400> 151
   Gln Gln Tyr Ser Ser Phe Pro Thr Thr
   1 5
<210> 152
   <211> 7
   <212> PRT
   <213> Homo sapiens
<400> 152
   Gly Phe Thr Phe Ser Ser Tyr
   1 5
<210> 153
   <211> 6
   <212> PRT
   <213> Homo sapiens
<400> 153
   Ser Ser Gln Gly Lys Ser
   1 5
<210> 154
   <211> 8
   <212> PRT
   <213> Homo sapiens
<400> 154
   Trp Gly Asp Glu Gly Phe Asp Ile
   1               5
<210> 155
   <211> 7
   <212> PRT
   <213> Homo sapiens
<400> 155
   Ser Gln Gly Ile Ser Asn Trp
   1               5
<210> 156
   <211> 3
   <212> PRT
   <213> Homo sapiens
<400> 156
   Gly Ala Ser
   1
<210> 157
   <211> 6
   <212> PRT
   <213> Homo sapiens
<400> 157
   Tyr Ser Ser Phe Pro Thr
   1               5
<210> 158
   <211> 107
   <212> PRT
   <213> Homo sapiens
<400> 158
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys
                       100                 105
<210> 159
   <211> 117
   <212> PRT
   <213> Homo sapiens
<400> 159
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Ser Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 160
   <211> 321
   <212> DNA
   <213> Homo sapiens
<400> 160
   gatatccaga tgacccagag ccccagcagc ctgagcgcca gcgtgggcga cagagtgacc       60
   atcacctgtc gggccagcca gggcatcagc aactggctgg cctggtatca gcagaagccc      120
   ggcaaggccc ccaagctgct gatctacggc gccagctccc tgcagagcgg cgtgccaagc      180
   agattcagcg gcagcggctc cggcaccgac ttcaccctga ccatcagcag cctgcagccc      240
   gaggacttcg ccacctacta ctgccagcag tacagcagct tccccaccac cttcggccag      300
   ggcaccaagg tggaaatcaa g                                                321
<210> 161
   <211> 351
   <212> DNA
   <213> Homo sapiens
<400> 161
   gaggtgcaat tgctggaaag cggcggaggc ctggtgcagc ctggcggcag cctgagactg        60
   tcttgcgccg ccagcggctt caccttcagc agctacgcca tgagctgggt ccgccaggcc       120
   cctggcaagg gactggaatg ggtgtccgcc atcagcagcc agggcaagag cacctactac       180
   gccgacagcg tgaagggccg gttcaccatc agccgggaca acagcaagaa caccctgtac       240
   ctgcagatga acagcctgcg ggccgaggac accgccgtgt actactgtgc cagatggggc       300
   gacgagggct tcgacatctg gggccagggc accctggtca ccgtcagctc a                351
<210> 162
   <211> 214
   <212> PRT
   <213> Homo sapiens
<400> 162
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 163
   <211> 447
   <212> PRT
   <213> Homo sapiens
<400> 163
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Ser Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 164
   <211> 5
   <212> PRT
   <213> Homo sapiens
<400> 164
   Ser Tyr Ala Met Ser
   1 5
<210> 165
   <211> 17
   <212> PRT
   <213> Homo sapiens
<400> 165
   Ala Ile Gly Ser Gln Gly Lys Ser Thr Tyr Tyr Ala Asp Ser Val Lys
   1 5 10 15
   Gly
<210> 166
   <211> 8
   <212> PRT
   <213> Homo sapiens
<400> 166
   Trp Gly Asp Glu Gly Phe Asp Ile
   1 5
<210> 167
   <211> 11
   <212> PRT
   <213> Homo sapiens
<400> 167
   Arg Ala Ser Gln Gly Ile Ser Asn Trp Leu Ala
   1 5 10
<210> 168
   <211> 7
   <212> PRT
   <213> Homo sapiens
<400> 168
   Gly Ala Ser Ser Leu Gln Ser
   1 5
<210> 169
   <211> 9
   <212> PRT
   <213> Homo sapiens
<400> 169
   Gln Gln Tyr Ser Ser Phe Pro Thr Thr
   1 5
<210> 170
   <211> 7
   <212> PRT
   <213> Homo sapiens
<400> 170
   Gly Phe Thr Phe Ser Ser Tyr
   1 5
<210> 171
   <211> 6
   <212> PRT
   <213> Homo sapiens
<400> 171
   Gly Ser Gln Gly Lys Ser
   1 5
<210> 172
   <211> 8
   <212> PRT
   <213> Homo sapiens
<400> 172
   Trp Gly Asp Glu Gly Phe Asp Ile
   1 5
<210> 173
   <211> 7
   <212> PRT
   <213> Homo sapiens
<400> 173
   Ser Gln Gly Ile Ser Asn Trp
   1 5
<210> 174
   <211> 3
   <212> PRT
   <213> Homo sapiens
<400> 174
   Gly Ala Ser
   1
<210> 175
   <211> 6
   <212> PRT
   <213> Homo sapiens
<400> 175
   Tyr Ser Ser Phe Pro Thr
   1               5
<210> 176
   <211> 107
   <212> PRT
   <213> Homo sapiens
<400> 176
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 177
   <211> 117
   <212> PRT
   <213> Homo sapiens
<400> 177
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Gly Ser Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 178
   <211> 321
   <212> DNA
   <213> Homo sapiens
<400> 178
   gatatccaga tgacccagag ccccagcagc ctgagcgcca gcgtgggcga cagagtgacc      60
   atcacctgtc gggccagcca gggcatcagc aactggctgg cctggtatca gcagaagccc     120
   ggcaaggccc ccaagctgct gatctacggc gccagctccc tgcagagcgg cgtgccaagc     180
   agattcagcg gcagcggctc cggcaccgac ttcaccctga ccatcagcag cctgcagccc     240
   gaggacttcg ccacctacta ctgccagcag tacagcagct tccccaccac cttcggccag     300
   ggcaccaagg tggaaatcaa g                                               321
<210> 179
   <211> 351
   <212> DNA
   <213> Homo sapiens
<400> 179
   gaggtgcaat tgctggaaag cggcggaggc ctggtgcagc ctggcggcag cctgagactg      60
   tcttgcgccg ccagcggctt caccttcagc agctacgcca tgagctgggt ccgccaggcc     120
   cctggcaagg gactggaatg ggtgtccgcc atcggcagcc agggcaagag cacctactac     180
   gccgacagcg tgaagggccg gttcaccatc agccgggaca acagcaagaa caccctgtac     240
   ctgcagatga acagcctgcg ggccgaggac accgccgtgt actactgtgc cagatggggc     300
   gacgagggct tcgacatctg gggccagggc accctggtca ccgtcagctc a              351
<210> 180
   <211> 214
   <212> PRT
   <213> Homo sapiens
<400> 180
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
   20 25 30
   Leu Ala Trp Tyr Gln Gln Lys Pro Gly Lys Ala Pro Lys Leu Leu Ile
   35 40 45
   Tyr Gly Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Ser Phe Pro Thr
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 181
   <211> 447
   <212> PRT
   <213> Homo sapiens
<400> 181
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Gly Ser Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
               275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
           435                 440                 445
<210> 182
   <211> 5
   <212> PRT
   <213> Homo sapiens
<400> 182
   Ser Tyr Ala Met Ser
   1               5
<210> 183
   <211> 17
   <212> PRT
   <213> Homo sapiens
<400> 183
   Ala Ile Ser Asn Gln Gly Lys Ser Thr Tyr Tyr Ala Asp Ser Val Lys
   1               5                   10                  15
   Gly
<210> 184
   <211> 8
   <212> PRT
   <213> Homo sapiens
<400> 184
   Trp Gly Asp Glu Gly Phe Asp Ile
   1               5
<210> 185
   <211> 11
   <212> PRT
   <213> Homo sapiens
<400> 185
   Arg Ala Ser Gln Gly Ile Ser Asn Trp Leu Ala
   1               5                   10
<210> 186
   <211> 7
   <212> PRT
   <213> Homo sapiens
<400> 186
   Gly Ala Ser Ser Leu Gln Ser
   1               5
<210> 187
   <211> 9
   <212> PRT
   <213> Homo sapiens
<400> 187
   Gln Gln Tyr Ser Ser Phe Pro Thr Thr
   1               5
<210> 188
   <211> 7
   <212> PRT
   <213> Homo sapiens
<400> 188
   Gly Phe Thr Phe Ser Ser Tyr
   1               5
<210> 189
   <211> 6
   <212> PRT
   <213> Homo sapiens
<400> 189
   Ser Asn Gln Gly Lys Ser
   1 5
<210> 190
   <211> 8
   <212> PRT
   <213> Homo sapiens
<400> 190
   Trp Gly Asp Glu Gly Phe Asp Ile
   1 5
<210> 191
   <211> 7
   <212> PRT
   <213> Homo sapiens
<400> 191
   Ser Gln Gly Ile Ser Asn Trp
   1 5
<210> 192
   <211> 3
   <212> PRT
   <213> Homo sapiens
<400> 192
   Gly Ala Ser
   1
<210> 193
   <211> 6
   <212> PRT
   <213> Homo sapiens
<400> 193
   Tyr Ser Ser Phe Pro Thr
   1 5
<210> 194
   <211> 107
   <212> PRT
   <213> Homo sapiens
<400> 194
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Ser Asn Trp
   20 25 30
   Leu Ala Trp Tyr Gln Gln Lys Pro Gly Lys Ala Pro Lys Leu Leu Ile
   35 40 45
   Tyr Gly Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Ser Phe Pro Thr
   85 90 95
   Thr Phe Gly Gln Gly Thr Lys Val Glu Ile Lys
   100 105
<210> 195
   <211> 117
   <212> PRT
   <213> Homo sapiens
<400> 195
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Asn Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 196
   <211> 321
   <212> DNA
   <213> Homo sapiens
<400> 196
   gatatccaga tgacccagag ccccagcagc ctgagcgcca gcgtgggcga cagagtgacc      60
   atcacctgtc gggccagcca gggcatcagc aactggctgg cctggtatca gcagaagccc     120
   ggcaaggccc ccaagctgct gatctacggc gccagctccc tgcagagcgg cgtgccaagc     180
   agattcagcg gcagcggctc cggcaccgac ttcaccctga ccatcagcag cctgcagccc     240
   gaggacttcg ccacctacta ctgccagcag tacagcagct tccccaccac cttcggccag     300
   ggcaccaagg tggaaatcaa g                                               321
<210> 197
   <211> 351
   <212> DNA
   <213> Homo sapiens
<400> 197
   gaggtgcaat tgctggaaag cggcggaggc ctggtgcagc ctggcggcag cctgagactg       60
   tcttgcgccg ccagcggctt caccttcagc agctacgcca tgagctgggt ccgccaggcc      120
   cctggcaagg gactggaatg ggtgtccgcc atcagcaacc agggcaagag cacctactac      180
   gccgacagcg tgaagggccg gttcaccatc agccgggaca acagcaagaa caccctgtac      240
   ctgcagatga acagcctgcg ggccgaggac accgccgtgt actactgtgc cagatggggc      300
   gacgagggct tcgacatctg gggccagggc accctggtca ccgtcagctc a               351
<210> 198
   <211> 214
   <212> PRT
   <213> Homo sapiens
<400> 198
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 199
   <211> 447
   <212> PRT
   <213> Homo sapiens
<400> 199
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Asn Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145 150 155 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Arg Val Glu Pro Lys Ser Cys Asp Lys Thr His
   210 215 220
   Thr Cys Pro Pro Cys Pro Ala Pro Glu Leu Leu Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 200
   <211> 5
   <212> PRT
   <213> Homo sapiens
<400> 200
   Ser Tyr Ala Met Ser
   1 5
<210> 201
   <211> 17
   <212> PRT
   <213> Homo sapiens
<400> 201
   Val Ile Ser Ser Gln Gly Lys Ser Thr Tyr Tyr Ala Asp Ser Val Lys
   1 5 10 15
   Gly
<210> 202
   <211> 8
   <212> PRT
   <213> Homo sapiens
<400> 202
   Trp Gly Asp Glu Gly Phe Asp Ile
   1 5
<210> 203
   <211> 11
   <212> PRT
   <213> Homo sapiens
<400> 203
   Arg Ala Ser Gln Gly Ile Ser Asn Trp Leu Ala
   1 5 10
<210> 204
   <211> 7
   <212> PRT
   <213> Homo sapiens
<400> 204
   Gly Ala Ser Ser Leu Gln Ser
   1 5
<210> 205
   <211> 9
   <212> PRT
   <213> Homo sapiens
<400> 205
   Gln Gln Tyr Ser Ser Phe Pro Thr Thr
   1 5
<210> 206
   <211> 7
   <212> PRT
   <213> Homo sapiens
<400> 206
   Gly Phe Thr Phe Ser Ser Tyr
   1 5
<210> 207
   <211> 6
   <212> PRT
   <213> Homo sapiens
<400> 207
   Ser Ser Gln Gly Lys Ser
   1 5
<210> 208
   <211> 8
   <212> PRT
   <213> Homo sapiens
<400> 208
   Trp Gly Asp Glu Gly Phe Asp Ile
   1 5
<210> 209
   <211> 7
   <212> PRT
   <213> Homo sapiens
<400> 209
   Ser Gln Gly Ile Ser Asn Trp
   1 5
<210> 210
   <211> 3
   <212> PRT
   <213> Homo sapiens
<400> 210
   Gly Ala Ser
   1
<210> 211
   <211> 6
   <212> PRT
   <213> Homo sapiens
<400> 211
   Tyr Ser Ser Phe Pro Thr
   1 5
<210> 212
   <211> 107
   <212> PRT
   <213> Homo sapiens
<400> 212
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Ser Asn Trp
   20 25 30
   Leu Ala Trp Tyr Gln Gln Lys Pro Gly Lys Ala Pro Lys Leu Leu Ile
   35 40 45
   Tyr Gly Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Ser Phe Pro Thr
   85 90 95
   Thr Phe Gly Gln Gly Thr Lys Val Glu Ile Lys
   100 105
<210> 213
   <211> 117
   <212> PRT
   <213> Homo sapiens
<400> 213
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Ile Ser Ser Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 214
   <211> 321
   <212> DNA
   <213> Homo sapiens
<400> 214
   gatatccaga tgacccagag ccccagcagc ctgagcgcca gcgtgggcga cagagtgacc      60
   atcacctgtc gggccagcca gggcatcagc aactggctgg cctggtatca gcagaagccc     120
   ggcaaggccc ccaagctgct gatctacggc gccagctccc tgcagagcgg cgtgccaagc     180
   agattcagcg gcagcggctc cggcaccgac ttcaccctga ccatcagcag cctgcagccc     240
   gaggacttcg ccacctacta ctgccagcag tacagcagct tccccaccac cttcggccag     300
   ggcaccaagg tggaaatcaa g                                               321
<210> 215
   <211> 351
   <212> DNA
   <213> Homo sapiens
<400> 215
   gaggtgcaat tgctggaaag cggcggaggc ctggtgcagc ctggcggcag cctgagactg        60
   tcttgcgccg ccagcggctt caccttcagc agctacgcca tgagctgggt ccgccaggcc       120
   cctggcaagg gactggaatg ggtgtccgtc atcagcagcc agggcaagag cacctactac       180
   gccgacagcg tgaagggccg gttcaccatc agccgggaca acagcaagaa caccctgtac       240
   ctgcagatga acagcctgcg ggccgaggac accgccgtgt actactgtgc cagatggggc       300
   gacgagggct tcgacatctg gggccagggc accctggtca ccgtcagctc a                351
<210> 216
   <211> 214
   <212> PRT
   <213> Homo sapiens
<400> 216
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 217
   <211> 447
   <212> PRT
   <213> Homo sapiens
<400> 217
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Ile Ser Ser Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 218
   <211> 5
   <212> PRT
   <213> Homo sapiens
<400> 218
   Ser Tyr Ala Met Ser
   1 5
<210> 219
   <211> 17
   <212> PRT
   <213> Homo sapiens
<400> 219
   Val Ile Gly Ser Gln Gly Lys Ser Thr Tyr Tyr Ala Asp Ser Val Lys
   1               5                   10                  15
   Gly
<210> 220
   <211> 8
   <212> PRT
   <213> Homo sapiens
<400> 220
   Trp Gly Asp Glu Gly Phe Asp Ile
   1               5
<210> 221
   <211> 11
   <212> PRT
   <213> Homo sapiens
<400> 221
   Arg Ala Ser Gln Gly Ile Ser Asn Trp Leu Ala
   1               5                   10
<210> 222
   <211> 7
   <212> PRT
   <213> Homo sapiens
<400> 222
   Gly Ala Ser Ser Leu Gln Ser
   1               5
<210> 223
   <211> 9
   <212> PRT
   <213> Homo sapiens
<400> 223
   Gln Gln Tyr Ser Ser Phe Pro Thr Thr
   1               5
<210> 224
   <211> 7
   <212> PRT
   <213> Homo sapiens
<400> 224
   Gly Phe Thr Phe Ser Ser Tyr
   1 5
<210> 225
   <211> 6
   <212> PRT
   <213> Homo sapiens
<400> 225
   Gly Ser Gln Gly Lys Ser
   1 5
<210> 226
   <211> 8
   <212> PRT
   <213> Homo sapiens
<400> 226
   Trp Gly Asp Glu Gly Phe Asp Ile
   1 5
<210> 227
   <211> 7
   <212> PRT
   <213> Homo sapiens
<400> 227
   Ser Gln Gly Ile Ser Asn Trp
   1 5
<210> 228
   <211> 3
   <212> PRT
   <213> Homo sapiens
<400> 228
   Gly Ala Ser
   1
<210> 229
   <211> 6
   <212> PRT
   <213> Homo sapiens
<400> 229
   Tyr Ser Ser Phe Pro Thr
   1 5
<210> 230
   <211> 107
   <212> PRT
   <213> Homo sapiens
<400> 230
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 231
   <211> 117
   <212> PRT
   <213> Homo sapiens
<400> 231
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Ile Gly Ser Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 232
   <211> 321
   <212> DNA
   <213> Homo sapiens
<400> 232
   gatatccaga tgacccagag ccccagcagc ctgagcgcca gcgtgggcga cagagtgacc 60
   atcacctgtc gggccagcca gggcatcagc aactggctgg cctggtatca gcagaagccc 120
   ggcaaggccc ccaagctgct gatctacggc gccagctccc tgcagagcgg cgtgccaagc 180
   agattcagcg gcagcggctc cggcaccgac ttcaccctga ccatcagcag cctgcagccc 240
   gaggacttcg ccacctacta ctgccagcag tacagcagct tccccaccac cttcggccag 300
   ggcaccaagg tggaaatcaa g 321
<210> 233
   <211> 351
   <212> DNA
   <213> Homo sapiens
<400> 233
   gaggtgcaat tgctggaaag cggcggaggc ctggtgcagc ctggcggcag cctgagactg        60
   tcttgcgccg ccagcggctt caccttcagc agctacgcca tgagctgggt ccgccaggcc       120
   cctggcaagg gactggaatg ggtgtccgtc atcggcagcc agggcaagag cacctactac       180
   gccgacagcg tgaagggccg gttcaccatc agccgggaca acagcaagaa caccctgtac       240
   ctgcagatga acagcctgcg ggccgaggac accgccgtgt actactgtgc cagatggggc       300
   gacgagggct tcgacatctg gggccagggc accctggtca ccgtcagctc a                351
<210> 234
   <211> 214
   <212> PRT
   <213> Homo sapiens
<400> 234
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 235
   <211> 447
   <212> PRT
   <213> Homo sapiens
<400> 235
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Val Ile Gly Ser Gln Gly Lys Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 236
   <211> 5
   <212> PRT
   <213> Homo sapiens
<400> 236
   Ser Tyr Ala Met Ser
   1 5
<210> 237
   <211> 17
   <212> PRT
   <213> Homo sapiens
<400> 237
   Ala Ile Asn Ala Gln Gly Lys Ser Thr Tyr Tyr Ala Asp Ser Val Lys
   1 5 10 15
   Gly
<210> 238
   <211> 8
   <212> PRT
   <213> Homo sapiens
<400> 238
   Trp Gly Asp Glu Gly Phe Asp Ile
   1 5
<210> 239
   <211> 11
   <212> PRT
   <213> Homo sapiens
<400> 239
   Arg Ala Ser Gln Gly Ile Ser Asn Trp Leu Ala
   1 5 10
<210> 240
   <211> 7
   <212> PRT
   <213> Homo sapiens
<400> 240
   Gly Ala Ser Ser Leu Gln Ser
   1 5
<210> 241
   <211> 9
   <212> PRT
   <213> Homo sapiens
<400> 241
   Gln Gln Tyr Ser Ser Phe Pro Thr Thr
   1 5
<210> 242
   <211> 7
   <212> PRT
   <213> Homo sapiens
<400> 242
   Gly Phe Thr Phe Ser Ser Tyr
   1 5
<210> 243
   <211> 6
   <212> PRT
   <213> Homo sapiens
<400> 243
   Asn Ala Gln Gly Lys Ser
   1 5
<210> 244
   <211> 8
   <212> PRT
   <213> Homo sapiens
<400> 244
   Trp Gly Asp Glu Gly Phe Asp Ile
   1 5
<210> 245
   <211> 7
   <212> PRT
   <213> Homo sapiens
<400> 245
   Ser Gln Gly Ile Ser Asn Trp
   1 5
<210> 246
   <211> 3
   <212> PRT
   <213> Homo sapiens
<400> 246
   Gly Ala Ser
   1
<210> 247
   <211> 6
   <212> PRT
   <213> Homo sapiens
<400> 247
   Tyr Ser Ser Phe Pro Thr
   1 5
<210> 248
   <211> 107
   <212> PRT
   <213> Homo sapiens
<400> 248
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 249
   <211> 117
   <212> PRT
   <213> Homo sapiens
<400> 249
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Asn Ala Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 250
   <211> 321
   <212> DNA
   <213> Homo sapiens
<400> 250
   gatatccaga tgacccagag ccccagcagc ctgagcgcca gcgtgggcga cagagtgacc        60
   atcacctgtc gggccagcca gggcatcagc aactggctgg cctggtatca gcagaagccc       120
   ggcaaggccc ccaagctgct gatctacggc gccagctccc tgcagagcgg cgtgccaagc       180
   agattcagcg gcagcggctc cggcaccgac ttcaccctga ccatcagcag cctgcagccc       240
   gaggacttcg ccacctacta ctgccagcag tacagcagct tccccaccac cttcggccag       300
   ggcaccaagg tggaaatcaa g                                                 321
<210> 251
   <211> 351
   <212> DNA
   <213> Homo sapiens
<400> 251
   gaggtgcaat tgctggaaag cggcggaggc ctggtgcagc ctggcggcag cctgagactg        60
   tcttgcgccg ccagcggctt caccttcagc agctacgcca tgagctgggt ccgccaggcc       120
   cctggcaagg gactggaatg ggtgtccgcc atcaacgccc agggcaagag cacctactac       180
   gccgacagcg tgaagggccg gttcaccatc agccgggaca acagcaagaa caccctgtac       240
   ctgcagatga acagcctgcg ggccgaggac accgccgtgt actactgtgc cagatggggc       300
   gacgagggct tcgacatctg gggccagggc accctggtca ccgtcagctc a                351
<210> 252
   <211> 214
   <212> PRT
   <213> Homo sapiens
<400> 252
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 253
   <211> 447
   <212> PRT
   <213> Homo sapiens
<400> 253
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Asn Ala Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
   210 215 220
   Thr Cys Pro Pro Cys Pro Ala Pro Glu Leu Leu Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 254
   <211> 5
   <212> PRT
   <213> Homo sapiens
<400> 254
   Ser Tyr Ala Met Ser
   1 5
<210> 255
   <211> 17
   <212> PRT
   <213> Homo sapiens
<400> 255
   Ala Ile Asn Thr Gln Gly Lys Ser Thr Tyr Tyr Ala Asp Ser Val Lys
   1 5 10 15
   Gly
<210> 256
   <211> 8
   <212> PRT
   <213> Homo sapiens
<400> 256
   Trp Gly Asp Glu Gly Phe Asp Ile
   1 5
<210> 257
   <211> 11
   <212> PRT
   <213> Homo sapiens
<400> 257
   Arg Ala Ser Gln Gly Ile Ser Asn Trp Leu Ala
   1 5 10
<210> 258
   <211> 7
   <212> PRT
   <213> Homo sapiens
<400> 258
   Gly Ala Ser Ser Leu Gln Ser
   1 5
<210> 259
   <211> 9
   <212> PRT
   <213> Homo sapiens
<400> 259
   Gln Gln Tyr Ser Ser Phe Pro Thr Thr
   1               5
<210> 260
   <211> 7
   <212> PRT
   <213> Homo sapiens
<400> 260
   Gly Phe Thr Phe Ser Ser Tyr
   1               5
<210> 261
   <211> 6
   <212> PRT
   <213> Homo sapiens
<400> 261
   Asn Thr Gln Gly Lys Ser
   1               5
<210> 262
   <211> 8
   <212> PRT
   <213> Homo sapiens
<400> 262
   Trp Gly Asp Glu Gly Phe Asp Ile
   1               5
<210> 263
   <211> 7
   <212> PRT
   <213> Homo sapiens
<400> 263
   Ser Gln Gly Ile Ser Asn Trp
   1               5
<210> 264
   <211> 3
   <212> PRT
   <213> Homo sapiens
<400> 264
   Gly Ala Ser
   1
<210> 265
   <211> 6
   <212> PRT
   <213> Homo sapiens
<400> 265
   Tyr Ser Ser Phe Pro Thr
   1               5
<210> 266
   <211> 107
   <212> PRT
   <213> Homo sapiens
<400> 266
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 267
   <211> 117
   <212> PRT
   <213> Homo sapiens
<400> 267
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Asn Thr Gln Gly Lys Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 268
   <211> 321
   <212> DNA
   <213> Homo sapiens
<400> 268
   gatatccaga tgacccagag ccccagcagc ctgagcgcca gcgtgggcga cagagtgacc     60
   atcacctgtc gggccagcca gggcatcagc aactggctgg cctggtatca gcagaagccc    120
   ggcaaggccc ccaagctgct gatctacggc gccagctccc tgcagagcgg cgtgccaagc    180
   agattcagcg gcagcggctc cggcaccgac ttcaccctga ccatcagcag cctgcagccc    240
   gaggacttcg ccacctacta ctgccagcag tacagcagct tccccaccac cttcggccag    300
   ggcaccaagg tggaaatcaa g                                              321
<210> 269
   <211> 351
   <212> DNA
   <213> Homo sapiens
<400> 269
   gaggtgcaat tgctggaaag cggcggaggc ctggtgcagc ctggcggcag cctgagactg        60
   tcttgcgccg ccagcggctt caccttcagc agctacgcca tgagctgggt ccgccaggcc       120
   cctggcaagg gactggaatg ggtgtccgcc atcaacaccc agggcaagag cacctactac       180
   gccgacagcg tgaagggccg gttcaccatc agccgggaca acagcaagaa caccctgtac       240
   ctgcagatga acagcctgcg ggccgaggac accgccgtgt actactgtgc cagatggggc       300
   gacgagggct tcgacatctg gggccagggc accctggtca ccgtcagctc a                351
<210> 270
   <211> 214
   <212> PRT
   <213> Homo sapiens
<400> 270
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Ser Phe Pro Thr
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 271
   <211> 447
   <212> PRT
   <213> Homo sapiens
<400> 271
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Asn Thr Gln Gly Lys Ser Thr Tyr Tyr Ala Asp Ser Val
   50 55 60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 272
   <211> 5
   <212> PRT
   <213> Homo sapiens
<400> 272
   Ser Tyr Ala Met Ser
   1 5
<210> 273
   <211> 17
   <212> PRT
   <213> Homo sapiens
<400> 273
   Val Thr Gly Ala Val Gly Ser Ser Thr Tyr Tyr Pro Asp Ser Val Lys
   1 5 10 15
   Gly
<210> 274
   <211> 8
   <212> PRT
   <213> Homo sapiens
<400> 274
   Trp Gly Asp Glu Gly Phe Asp Ile
   1               5
<210> 275
   <211> 11
   <212> PRT
   <213> Homo sapiens
<400> 275
   Arg Ala Ser Gln Gly Ile Ser Asn Trp Leu Ala
   1               5                   10
<210> 276
   <211> 7
   <212> PRT
   <213> Homo sapiens
<400> 276
   Gly Ala Ser Ser Leu Gln Ser
   1               5
<210> 277
   <211> 9
   <212> PRT
   <213> Homo sapiens
<400> 277
   Gln Gln Tyr Ser Ser Phe Pro Thr Thr
   1               5
<210> 278
   <211> 7
   <212> PRT
   <213> Homo sapiens
<400> 278
   Gly Phe Thr Phe Ser Ser Tyr
   1               5
<210> 279
   <211> 6
   <212> PRT
   <213> Homo sapiens
<400> 279
   Gly Ala Val Gly Ser Ser
   1               5
<210> 280
   <211> 8
   <212> PRT
   <213> Homo sapiens
<400> 280
   Trp Gly Asp Glu Gly Phe Asp Ile
   1 5
<210> 281
   <211> 7
   <212> PRT
   <213> Homo sapiens
<400> 281
   Ser Gln Gly Ile Ser Asn Trp
   1 5
<210> 282
   <211> 3
   <212> PRT
   <213> Homo sapiens
<400> 282
   Gly Ala Ser
   1
<210> 283
   <211> 6
   <212> PRT
   <213> Homo sapiens
<400> 283
   Tyr Ser Ser Phe Pro Thr
   1 5
<210> 284
   <211> 107
   <212> PRT
   <213> Homo sapiens
<400> 284
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys
                       100                 105
<210> 285
   <211> 117
   <212> PRT
   <213> Homo sapiens
<400> 285
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Thr Gly Ala Val Gly Ser Ser Thr Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 286
   <211> 321
   <212> DNA
   <213> Homo sapiens
<400> 286
   gatatccaga tgacccagag ccccagcagc ctgagcgcca gcgtgggcga cagagtgacc 60
   atcacctgtc gggccagcca gggcatcagc aactggctgg cctggtatca gcagaagccc 120
   ggcaaggccc ccaagctgct gatctacggc gccagctccc tgcagagcgg cgtgccaagc 180
   agattcagcg gcagcggctc cggcaccgac ttcaccctga ccatcagcag cctgcagccc 240
   gaggacttcg ccacctacta ctgccagcag tacagcagct tccccaccac cttcggccag 300
   ggcaccaagg tggaaatcaa g 321
<210> 287
   <211> 351
   <212> DNA
   <213> Homo sapiens
<400> 287
   gaggtgcaat tgctggaaag cggcggaggc ctggtgcagc ctggcggcag cctgagactg      60
   tcttgcgccg ccagcggctt caccttcagc agctacgcca tgagctgggt ccgccaggcc     120
   cctggcaagg gactggaatg ggtgtccgtg acaggcgccg tgggcagcag cacctactac     180
   cccgacagcg tgaagggccg gttcaccatc agccgggaca acagcaagaa caccctgtac     240
   ctgcagatga acagcctgcg ggccgaggac accgccgtgt actactgtgc cagatggggc     300
   gacgagggct tcgacatctg gggccagggc accctggtca ccgtcagctc a               351
<210> 288
   <211> 214
   <212> PRT
   <213> Homo sapiens
<400> 288
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                       165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 289
   <211> 447
   <212> PRT
   <213> Homo sapiens
<400> 289
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Thr Gly Ala Val Gly Ser Ser Thr Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 290
   <211> 5
   <212> PRT
   <213> Homo sapiens
<400> 290
   Ser Tyr Ala Met Ser
   1 5
<210> 291
   <211> 17
   <212> PRT
   <213> Homo sapiens
<400> 291
   Val Thr Gly Ala Val Gly Gly Ser Thr Tyr Tyr Pro Asp Ser Val Lys
   1 5 10 15
   Gly
<210> 292
   <211> 8
   <212> PRT
   <213> Homo sapiens
<400> 292
   Trp Gly Asp Glu Gly Phe Asp Ile
   1 5
<210> 293
   <211> 11
   <212> PRT
   <213> Homo sapiens
<400> 293
   Arg Ala Ser Gln Gly Ile Ser Asn Trp Leu Ala
   1 5 10
<210> 294
   <211> 7
   <212> PRT
   <213> Homo sapiens
<400> 294
   Gly Ala Ser Ser Leu Gln Ser
   1 5
<210> 295
   <211> 9
   <212> PRT
   <213> Homo sapiens
<400> 295
   Gln Gln Tyr Ser Ser Phe Pro Thr Thr
   1 5
<210> 296
   <211> 7
   <212> PRT
   <213> Homo sapiens
<400> 296
   Gly Phe Thr Phe Ser Ser Tyr
   1 5
<210> 297
   <211> 6
   <212> PRT
   <213> Homo sapiens
<400> 297
   Gly Ala Val Gly Gly Ser
   1 5
<210> 298
   <211> 8
   <212> PRT
   <213> Homo sapiens
<400> 298
   Trp Gly Asp Glu Gly Phe Asp Ile
   1 5
<210> 299
   <211> 7
   <212> PRT
   <213> Homo sapiens
<400> 299
   Ser Gln Gly Ile Ser Asn Trp
   1 5
<210> 300
   <211> 3
   <212> PRT
   <213> Homo sapiens
<400> 300
   Gly Ala Ser
   1
<210> 301
   <211> 6
   <212> PRT
   <213> Homo sapiens
<400> 301
   Tyr Ser Ser Phe Pro Thr
   1 5
<210> 302
   <211> 107
   <212> PRT
   <213> Homo sapiens
<400> 302
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Ser Asn Trp
   20 25 30
   Leu Ala Trp Tyr Gln Gln Lys Pro Gly Lys Ala Pro Lys Leu Leu Ile
   35 40 45
   Tyr Gly Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Ser Phe Pro Thr
   85 90 95
   Thr Phe Gly Gln Gly Thr Lys Val Glu Ile Lys
   100 105
<210> 303
   <211> 117
   <212> PRT
   <213> Homo sapiens
<400> 303
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Thr Gly Ala Val Gly Gly Ser Thr Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 304
   <211> 321
   <212> DNA
   <213> Homo sapiens
<400> 304
   gatatccaga tgacccagag ccccagcagc ctgagcgcca gcgtgggcga cagagtgacc      60
   atcacctgtc gggccagcca gggcatcagc aactggctgg cctggtatca gcagaagccc     120
   ggcaaggccc ccaagctgct gatctacggc gccagctccc tgcagagcgg cgtgccaagc     180
   agattcagcg gcagcggctc cggcaccgac ttcaccctga ccatcagcag cctgcagccc     240
   gaggacttcg ccacctacta ctgccagcag tacagcagct tccccaccac cttcggccag     300
   ggcaccaagg tggaaatcaa g                                               321
<210> 305
   <211> 351
   <212> DNA
   <213> Homo sapiens
<400> 305
   gaggtgcaat tgctggaaag cggcggaggc ctggtgcagc ctggcggcag cctgagactg      60
   tcttgcgccg ccagcggctt caccttcagc agctacgcca tgagctgggt ccgccaggcc     120
   cctggcaagg gactggaatg ggtgtccgtg acaggcgccg tgggcggaag cacctactac     180
   cccgacagcg tgaagggccg gttcaccatc agccgggaca acagcaagaa caccctgtac     240
   ctgcagatga acagcctgcg ggccgaggac accgccgtgt actactgtgc cagatggggc     300
   gacgagggct tcgacatctg gggccagggc accctggtca ccgtcagctc a              351
<210> 306
   <211> 214
   <212> PRT
   <213> Homo sapiens
<400> 306
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
   20 25 30
   Leu Ala Trp Tyr Gln Gln Lys Pro Gly Lys Ala Pro Lys Leu Leu Ile
   35 40 45
   Tyr Gly Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Ser Phe Pro Thr
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 307
   <211> 447
   <212> PRT
   <213> Homo sapiens
<400> 307
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
   35 40 45
   Ser Val Thr Gly Ala Val Gly Gly Ser Thr Tyr Tyr Pro Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Trp Gly Asp Glu Gly Phe Asp Ile Trp Gly Gln Gly Thr Leu
   100 105 110
   Val Thr Val Ser Ser Ala Ser Thr Lys Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145 150 155 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Arg Val Glu Pro Lys Ser Cys Asp Lys Thr His
   210 215 220
   Thr Cys Pro Pro Cys Pro Ala Pro Glu Leu Leu Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 308
   <211> 5
   <212> PRT
   <213> Homo sapiens
<400> 308
   Ser Tyr Ala Met Ser
   1 5
<210> 309
   <211> 17
   <212> PRT
   <213> Homo sapiens
<400> 309
   Val Thr Gly Ala Val Gly Lys Ser Thr Tyr Tyr Pro Asp Ser Val Lys
   1 5 10 15
   Gly
<210> 310
   <211> 8
   <212> PRT
   <213> Homo sapiens
<400> 310
   Trp Gly Asp Glu Gly Phe Asp Ile
   1 5
<210> 311
   <211> 11
   <212> PRT
   <213> Homo sapiens
<400> 311
   Arg Ala Ser Gln Gly Ile Ser Asn Trp Leu Ala
   1 5 10
<210> 312
   <211> 7
   <212> PRT
   <213> Homo sapiens
<400> 312
   Gly Ala Ser Ser Leu Gln Ser
   1 5
<210> 313
   <211> 9
   <212> PRT
   <213> Homo sapiens
<400> 313
   Gln Gln Tyr Ser Ser Phe Pro Thr Thr
   1 5
<210> 314
   <211> 7
   <212> PRT
   <213> Homo sapiens
<400> 314
   Gly Phe Thr Phe Ser Ser Tyr
   1 5
<210> 315
   <211> 6
   <212> PRT
   <213> Homo sapiens
<400> 315
   Gly Ala Val Gly Lys Ser
   1 5
<210> 316
   <211> 8
   <212> PRT
   <213> Homo sapiens
<400> 1
<210> 317
   <211> 7
   <212> PRT
   <213> Homo sapiens
<400> 317
   Ser Gln Gly Ile Ser Asn Trp
   1 5
<210> 318
   <211> 3
   <212> PRT
   <213> Homo sapiens
<400> 318
   Gly Ala Ser
   1
<210> 319
   <211> 6
   <212> PRT
   <213> Homo sapiens
<400> 319
   Tyr Ser Ser Phe Pro Thr
   1 5
<210> 320
   <211> 107
   <212> PRT
   <213> Homo sapiens
<400> 320
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 321
   <211> 117
   <212> PRT
   <213> Homo sapiens
<400> 321
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Thr Gly Ala Val Gly Lys Ser Thr Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 322
   <211> 321
   <212> DNA
   <213> Homo sapiens
<400> 322
   gatatccaga tgacccagag ccccagcagc ctgagcgcca gcgtgggcga cagagtgacc      60
   atcacctgtc gggccagcca gggcatcagc aactggctgg cctggtatca gcagaagccc     120
   ggcaaggccc ccaagctgct gatctacggc gccagctccc tgcagagcgg cgtgccaagc     180
   agattcagcg gcagcggctc cggcaccgac ttcaccctga ccatcagcag cctgcagccc     240
   gaggacttcg ccacctacta ctgccagcag tacagcagct tccccaccac cttcggccag     300
   ggcaccaagg tggaaatcaa g                                               321
<210> 323
   <211> 351
   <212> DNA
   <213> Homo sapiens
<400> 323
   gaggtgcaat tgctggaaag cggcggaggc ctggtgcagc ctggcggcag cctgagactg      60
   tcttgcgccg ccagcggctt caccttcagc agctacgcca tgagctgggt ccgccaggcc     120
   cctggcaagg gactggaatg ggtgtccgtg acaggcgccg tgggcaaaag cacctactac     180
   cccgacagcg tgaagggccg gttcaccatc agccgggaca acagcaagaa caccctgtac     240
   ctgcagatga acagcctgcg ggccgaggac accgccgtgt actactgtgc cagatggggc     300
   gacgagggct tcgacatctg gggccagggc accctggtca ccgtcagctc a             351
<210> 324
   <211> 214
   <212> PRT
   <213> Homo sapiens
<400> 324
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 325
   <211> 447
   <212> PRT
   <213> Homo sapiens
<400> 325
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Thr Gly Ala Val Gly Lys Ser Thr Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
           435                 440                 445
<210> 326
   <211> 5
   <212> PRT
   <213> Homo sapiens
<400> 326
   Ser Tyr Ala Met Ser
   1               5
<210> 327
   <211> 16
   <212> PRT
   <213> Homo sapiens
<400> 327
   Val Thr Gly Ala Val Gly Arg Thr Tyr Tyr Pro Asp Ser Val Lys Gly
   1               5                   10                  15
<210> 328
   <211> 8
   <212> PRT
   <213> Homo sapiens
<400> 328
   Trp Gly Asp Glu Gly Phe Asp Ile
   1               5
<210> 329
   <211> 11
   <212> PRT
   <213> Homo sapiens
<400> 329
   Arg Ala Ser Gln Gly Ile Ser Asn Trp Leu Ala
   1               5                   10
<210> 330
   <211> 7
   <212> PRT
   <213> Homo sapiens
<400> 330
   Gly Ala Ser Ser Leu Gln Ser
   1 5
<210> 331
   <211> 9
   <212> PRT
   <213> Homo sapiens
<400> 331
   Gln Gln Tyr Ser Ser Phe Pro Thr Thr
   1 5
<210> 332
   <211> 7
   <212> PRT
   <213> Homo sapiens
<400> 332
   Gly Phe Thr Phe Ser Ser Tyr
   1 5
<210> 333
   <211> 6
   <212> PRT
   <213> Homo sapiens
<400> 333
   Gly Ala Val Gly Arg Thr
   1 5
<210> 334
   <211> 8
   <212> PRT
   <213> Homo sapiens
<400> 334
   Trp Gly Asp Glu Gly Phe Asp Ile
   1 5
<210> 335
   <211> 7
   <212> PRT
   <213> Homo sapiens
<400> 335
   Ser Gln Gly Ile Ser Asn Trp
   1 5
<210> 336
   <211> 3
   <212> PRT
   <213> Homo sapiens
<400> 336
   Gly Ala Ser
   1
<210> 337
   <211> 6
   <212> PRT
   <213> Homo sapiens
<400> 337
   Tyr Ser Ser Phe Pro Thr
   1 5
<210> 338
   <211> 107
   <212> PRT
   <213> Homo sapiens
<400> 338
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Ser Asn Trp
   20 25 30
   Leu Ala Trp Tyr Gln Gln Lys Pro Gly Lys Ala Pro Lys Leu Leu Ile
   35 40 45
   Tyr Gly Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Ser Phe Pro Thr
   85 90 95
   Thr Phe Gly Gln Gly Thr Lys Val Glu Ile Lys
   100 105
<210> 339
   <211> 116
   <212> PRT
   <213> Homo sapiens
<400> 339
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Thr Gly Ala Val Gly Arg Thr Tyr Tyr Pro Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Trp Gly Asp Glu Gly Phe Asp Ile Trp Gly Gln Gly Thr Leu Val
               100                 105                 110
   Thr Val Ser Ser
           115
<210> 340
   <211> 321
   <212> DNA
   <213> Homo sapiens
<400> 340
   gatatccaga tgacccagag ccccagcagc ctgagcgcca gcgtgggcga cagagtgacc      60
   atcacctgtc gggccagcca gggcatcagc aactggctgg cctggtatca gcagaagccc     120
   ggcaaggccc ccaagctgct gatctacggc gccagctccc tgcagagcgg cgtgccaagc     180
   agattcagcg gcagcggctc cggcaccgac ttcaccctga ccatcagcag cctgcagccc     240
   gaggacttcg ccacctacta ctgccagcag tacagcagct tccccaccac cttcggccag     300
   ggcaccaagg tggaaatcaa g                                               321
<210> 341
   <211> 348
   <212> DNA
   <213> Homo sapiens
<400> 341
   gaggtgcaat tgctggaaag cggcggaggc ctggtgcagc ctggcggcag cctgagactg      60
   tcttgcgccg ccagcggctt caccttcagc agctacgcca tgagctgggt ccgccaggcc     120
   cctggcaagg gactggaatg ggtgtccgtg acaggcgccg tgggcagaac ctactacccc     180
   gacagcgtga agggccggtt caccatcagc cgggacaaca gcaagaacac cctgtacctg     240
   cagatgaaca gcctgcgggc cgaggacacc gccgtgtact actgtgccag atggggcgac     300
   gagggcttcg acatctgggg ccagggcacc ctggtcaccg tcagctca                 348
<210> 342
   <211> 214
   <212> PRT
   <213> Homo sapiens
<400> 342
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 343
   <211> 446
   <212> PRT
   <213> Homo sapiens
<400> 343
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Val Thr Gly Ala Val Gly Arg Thr Tyr Tyr Pro Asp Ser Val Lys
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Trp Gly Asp Glu Gly Phe Asp Ile Trp Gly Gln Gly Thr Leu Val
   100 105 110
   Thr Val Ser Ser Ala Ser Thr Lys Gly Pro Ser Val Phe Pro Leu Ala
   115 120 125
   Pro Ser Ser Lys Ser Thr Ser Gly Gly Thr Ala Ala Leu Gly Cys Leu
   130 135 140
   Val Lys Asp Tyr Phe Pro Glu Pro Val Thr Val Ser Trp Asn Ser Gly
   145 150 155 160
   Ala Leu Thr Ser Gly Val His Thr Phe Pro Ala Val Leu Gln Ser Ser
   165 170 175
   Gly Leu Tyr Ser Leu Ser Ser Val Val Thr Val Pro Ser Ser Ser Leu
   180 185 190
   Gly Thr Gln Thr Tyr Ile Cys Asn Val Asn His Lys Pro Ser Asn Thr
   195 200 205
   Lys Val Asp Lys Arg Val Glu Pro Lys Ser Cys Asp Lys Thr His Thr
   210 215 220
   Cys Pro Pro Cys Pro Ala Pro Glu Leu Leu Gly Gly Pro Ser Val Phe
   225 230 235 240
   Leu Phe Pro Pro Lys Pro Lys Asp Thr Leu Met Ile Ser Arg Thr Pro
   245 250 255
   Glu Val Thr Cys Val Val Val Asp Val Ser His Glu Asp Pro Glu Val
   260 265 270
   Lys Phe Asn Trp Tyr Val Asp Gly Val Glu Val His Asn Ala Lys Thr
   275 280 285
   Lys Pro Arg Glu Glu Gln Tyr Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Ala Leu Pro Ala Pro Ile Glu Lys Thr Ile Ser
   325 330 335
   Lys Ala Lys Gly Gln Pro Arg Glu Pro Gln Val Tyr Thr Leu Pro Pro
   340 345 350
   Ser Arg Glu Glu Met Thr Lys Asn Gln Val Ser Leu Thr Cys Leu Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Tyr Ser Lys Leu Thr Val Asp Lys Ser Arg Trp
   405 410 415
   Gln Gln Gly Asn Val Phe Ser Cys Ser Val Met His Glu Ala Leu His
   420 425 430
   Asn His Tyr Thr Gln Lys Ser Leu Ser Leu Ser Pro Gly Lys
   435 440 445
<210> 344
   <211> 5
   <212> PRT
   <213> Homo sapiens
<400> 344
   Ser Tyr Ala Met Ser
   1 5
<210> 345
   <211> 17
   <212> PRT
   <213> Homo sapiens
<400> 345
   Val Ile Asn Gly Leu Gly Tyr Thr Thr Phe Tyr Ala Asp Ser Val Lys
   1 5 10 15
   Gly
<210> 346
   <211> 8
   <212> PRT
   <213> Homo sapiens
<400> 346
   Trp Gly Asp Glu Gly Phe Asp Ile
   1 5
<210> 347
   <211> 11
   <212> PRT
   <213> Homo sapiens
<400> 347
   Arg Ala Ser Gln Gly Ile Ser Asn Trp Leu Ala
   1 5 10
<210> 348
   <211> 7
   <212> PRT
   <213> Homo sapiens
<400> 348
   Gly Ala Ser Ser Leu Gln Ser
   1 5
<210> 349
   <211> 9
   <212> PRT
   <213> Homo sapiens
<400> 349
   Gln Gln Tyr Ser Ser Phe Pro Thr Thr
   1 5
<210> 350
   <211> 7
   <212> PRT
   <213> Homo sapiens
<400> 350
   Gly Phe Thr Phe Ser Ser Tyr
   1 5
<210> 351
   <211> 6
   <212> PRT
   <213> Homo sapiens
<400> 351
   Asn Gly Leu Gly Tyr Thr
   1 5
<210> 352
   <211> 8
   <212> PRT
   <213> Homo sapiens
<400> 352
   Trp Gly Asp Glu Gly Phe Asp Ile
   1 5
<210> 353
   <211> 7
   <212> PRT
   <213> Homo sapiens
<400> 353
   Ser Gln Gly Ile Ser Asn Trp
   1 5
<210> 354
   <211> 3
   <212> PRT
   <213> Homo sapiens
<400> 354
   Gly Ala Ser
   1
<210> 355
   <211> 6
   <212> PRT
   <213> Homo sapiens
<400> 355
   Tyr Ser Ser Phe Pro Thr
   1 5
<210> 356
   <211> 107
   <212> PRT
   <213> Homo sapiens
<400> 356
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 357
   <211> 117
   <212> PRT
   <213> Homo sapiens
<400> 357
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Ile Asn Gly Leu Gly Tyr Thr Thr Phe Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser
           115
<210> 358
   <211> 321
   <212> DNA
   <213> Homo sapiens
<400> 358
   gatatccaga tgacccagag cccgtctagc ctgagcgcga gcgtgggtga tcgtgtgacc       60
   attacctgca gagcgagcca gggtatttct aattggctgg cttggtacca gcagaaacca      120
   ggtaaagcac cgaaactatt aatttatggt gcttcttctt tgcaaagcgg ggtcccgtcc      180
   cgttttagcg gctctggatc cggcactgat tttaccctga ccattagcag cctgcaacct      240
   gaagactttg cggtttatta ttgccagcag tattcttctt ttcctactac ctttggccag      300
   ggtacgaaag ttgaaattaa a                                                321
<210> 359
   <211> 351
   <212> DNA
   <213> Homo sapiens
<400> 359
   caggtgcaat tggtggaaag cggcggcggc ctggtgcaac cgggcggcag cctgcgtctg        60
   agctgcgcgg cctccggatt tacctttagc agctatgcga tgagctgggt gcgccaagcc       120
   cctgggaagg gtctcgagtg ggtgagcgtt attaatggtc ttggttatac tactttttat       180
   gctgattctg ttaagggtcg ttttaccatt tcacgtgata attcgaaaaa caccctgtat       240
   ctgcaaatga acagcctgcg tgcggaagat acggccgtgt attattgcgc gcgttggggt       300
   gatgagggtt ttgatatttg gggccaaggc accctggtga cggttagctc a                351
<210> 360
   <211> 214
   <212> PRT
   <213> Homo sapiens
<400> 360
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 361
   <211> 447
   <212> PRT
   <213> Homo sapiens
<400> 361
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Ile Asn Gly Leu Gly Tyr Thr Thr Phe Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Asp Glu Gly Phe Asp Ile Trp Gly Gln Gly Thr Leu
               100                 105                 110
   Val Thr Val Ser Ser Ala Ser Thr Lys Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145 150 155 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Arg Val Glu Pro Lys Ser Cys Asp Lys Thr His
   210 215 220
   Thr Cys Pro Pro Cys Pro Ala Pro Glu Leu Leu Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 362
   <211> 5
   <212> PRT
   <213> Homo sapiens
<400> 362
   Ser Tyr Ala Met Ser
   1 5
<210> 363
   <211> 16
   <212> PRT
   <213> Homo sapiens
<400> 363
   Gly Thr Gly Pro Tyr Gly Gly Thr Tyr Tyr Pro Asp Ser Val Lys Gly
   1 5 10 15
<210> 364
   <211> 8
   <212> PRT
   <213> Homo sapiens
<400> 364
   Trp Gly Asp Glu Gly Phe Asp Ile
   1 5
<210> 365
   <211> 11
   <212> PRT
   <213> Homo sapiens
<400> 365
   Arg Ala Ser Gln Gly Ile Ser Asn Trp Leu Ala
   1 5 10
<210> 366
   <211> 7
   <212> PRT
   <213> Homo sapiens
<400> 366
   Gly Ala Ser Ser Leu Gln Ser
   1 5
<210> 367
   <211> 9
   <212> PRT
   <213> Homo sapiens
<400> 367
   Gln Gln Tyr Ser Ser Phe Pro Thr Thr
   1 5
<210> 368
   <211> 7
   <212> PRT
   <213> Homo sapiens
<400> 368
   Gly Phe Thr Phe Ser Ser Tyr
   1 5
<210> 369
   <211> 5
   <212> PRT
   <213> Homo sapiens
<400> 369
   Gly Pro Tyr Gly Gly
   1 5
<210> 370
   <211> 8
   <212> PRT
   <213> Homo sapiens
<400> 370
   Trp Gly Asp Glu Gly Phe Asp Ile
   1 5
<210> 371
   <211> 7
   <212> PRT
   <213> Homo sapiens
<400> 371
   Ser Gln Gly Ile Ser Asn Trp
   1               5
<210> 372
   <211> 3
   <212> PRT
   <213> Homo sapiens
<400> 372
   Gly Ala Ser
   1
<210> 373
   <211> 6
   <212> PRT
   <213> Homo sapiens
<400> 373
   Tyr Ser Ser Phe Pro Thr
   1               5
<210> 374
   <211> 107
   <212> **PRT**
   <213> Homo sapiens
<400> 374
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Ser Ser Phe Pro Thr
                   85                  90                  95
   Thr Phe Gly Gln Gly Thr Lys Val Glu Ile Lys
               100                 105
<210> 375
   <211> 116
   <212> PRT
   <213> Homo sapiens
<400> 375
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Gly Thr Gly Pro Tyr Gly Gly Thr Tyr Tyr Pro Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Trp Gly Asp Glu Gly Phe Asp Ile Trp Gly Gln Gly Thr Leu Val
               100                 105                 110
   Thr Val Ser Ser
           115
<210> 376
   <211> 321
   <212> DNA
   <213> Homo sapiens
<400> 376
   gatatccaga tgacccagag cccgtctagc ctgagcgcga gcgtgggtga tcgtgtgacc       60
   attacctgca gagcgagcca gggtatttct aattggctgg cttggtacca gcagaaacca      120
   ggtaaagcac cgaaactatt aatttatggt gcttcttctt tgcaaagcgg ggtcccgtcc      180
   cgttttagcg gctctggatc cggcactgat tttaccctga ccattagcag cctgcaacct      240
   gaagactttg cggtttatta ttgccagcag tattcttctt ttcctactac ctttggccag      300
   ggtacgaaag ttgaaattaa a                                                321
<210> 377
   <211> 348
   <212> DNA
   <213> Homo sapiens
<400> 377
   caggtgcaat tggtggaaag cggcggcggc ctggtgcaac cgggcggcag cctgcgtctg      60
   agctgcgcgg cctccggatt tacctttagc agctatgcga tgagctgggt gcgccaagcc     120
   cctgggaagg gtctcgagtg ggtgagcggt actggtcctt atggtggtac ttattatcct     180
   gattctgtta agggtcgttt taccatttca cgtgataatt cgaaaaacac cctgtatctg     240
   caaatgaaca gcctgcgtgc ggaagatacg gccgtgtatt attgcgcgcg ttggggtgat     300
   gagggttttg atatttgggg ccaaggcacc ctggtgacgg ttagctca                  348
<210> 378
   <211> 214
   <212> **PRT**
   <213> Homo sapiens
<400> 378
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Ser Ser Phe Pro Thr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 379
   <211> 446
   <212> PRT
   <213> Homo sapiens
<400> 379
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Gly Thr Gly Pro Tyr Gly Gly Thr Tyr Tyr Pro Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Trp Gly Asp Glu Gly Phe Asp Ile Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Arg Val Glu Pro Lys Ser Cys Asp Lys Thr His Thr
       210                 215                 220
   Cys Pro Pro Cys Pro Ala Pro Glu Leu Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser His Glu Asp Pro Glu Val
               260                 265                 270
   Lys Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Ala Leu Pro Ala Pro Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Arg Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Gln Gly Asn Val Phe Ser Cys Ser Val Met His Glu Ala Leu His
               420                 425                 430
   Asn His Tyr Thr Gln Lys Ser Leu Ser Leu Ser Pro Gly Lys
           435                 440                 445